# EUROPÄISCHE PATENTANMELDUNG

(11) **EP 3 056 561 A1**
(43) Veröffentlichungstag der Anmeldung: **17.08.2016**
(21) Anmeldenummer: 15155196.7
(22) Anmeldetag: 16.02.2015
(51) Int. Cl.: C12N 1/20, C12N 9/02, C12P 7/24

(54) **Mikroorganismen mit reduzierter Enzymaktivität**

(71) Anmelder: Evonik Degussa GmbH, 45128 Essen (DE)
(72) Erfinder: Engel, Philip, 45147 Essen (DE); Andrea, Heiko, 45768 Marl (DE); Schafer, Steffen, 45699 Herten (DE); Scholz, Judith, 44139 Dortmund (DE)

(57) **Zusammenfassung**

Gegenstand der Erfindung sind Mikroorganismen mit einer reduzierten Enzymaktivität sowie deren Einsatz zur Herstellung von Aldehyden.

## Beschreibung

### Gebiet der Erfindung

Gegenstand der Erfindung sind Mikroorganismen mit einer reduzierten Enzymaktivität sowie deren Einsatz zur Herstellung von Aldehyden.

### Stand der Technik

Aldehyde sind wichtige Produkte und Intermediate in der Chemie. Ein wichtiger Einsatzbereich für kurzkettige Aldehyde ist z.B. die Herstellung von Weichmacheralkoholen, wie Butanol oder 2-Ethylhexanol aus Butyraldehyd. Aldehyde werden heutzutage primär aus petrochemischen Rohstoffen chemisch hergestellt. Butyraldehyd wird z.B. aus Propylen und Synthesegas gewonnen.

Die Aufgabe dieser Erfindung bestand darin ein neues biotechnologisches Verfahren zur Herstellung von kurzkettigen Aldehyden zu entwickeln. Dabei wurde überraschend festgestellt, dass Organismen, die in der Lage sind die Alkane terminal zu oxidieren, vermehrt die entsprechenden Aldehyde bilden, wenn die Aktivität von einzelnen oder mehreren ausgewählten Aldehyd-Dehydrogenasen gezielt reduziert wurde.

Alkangase sind dabei eine alternative attraktive Rohstoffquelle. Chemisch sind diese nur sehr schwer nutzbar zu machen, daher steht heutzutage lediglich die energetische Nutzung im Vordergrund. Biologisch gibt es jedoch eine Vielzahl von Enzymen und Organismen, die auch kurzkettige Alkane C2-C9 selektiv aktivieren können. In der Literatur beschriebene Enzymsysteme bzw. Organismen sind z.B. alkB Monooxygenase aus *P. putida,* P450 Monooxygenasen oder Butane Monooxygenasen aus *Thauera butanivorans* (Shennen, 2006, J Chem, Technol. Biotechnol., 81, 237-256; Beilen, Funhoff, Curr. Op. Biotech. 2005, 16,308-314).

Die biotechnologische Herstellung von Butyraldehyd wurde bisher in zwei Patentanmeldungen beschrieben (WO2013/067325 - *Microbial production of n-butyraldehyde;* WO2012/112416-*Method for producing butyraldehyde).* In WO2013/067325 wurde die die Bildung von Butyraldehyd durch die heterologe Expression geeigneter Enzyme (atoB, crt, hdb, bldh) in Kombination mit knock-out mindestens eines natürlichen Genes (IdhA, adhE, frdBC, pta and yqhD) erzielt. WO2012/112416 beschriebt die Bildung von Butyraldehyd durch Reduktion oder knock-out von Enzymen des Butyraldehyd-Abbaus (z.B. Butanol (oder Alkohol) Dehydrogenase, Butyraldehyde Dehydrogenase, Lactate Dehydrogenase, hydrogen-evolving Hydrogenase oder Format Dehydrogenase und der möglichen Erhöhung einer Butyraldehyd dehydrogenase Aktivität. Der Einsatz bzw. die Kombination mit Alkan-oxidierenden Organismen ist hier jedoch nicht aufgeführt.

In dieser Erfindung wurde die Bildung von Aldehyden (C2-C9) aus Alkanen (C2-C9) durch eine reduzierte Aktivität oder *knock-out* einer oder mehrerer bisher unbekannter Aldehyd Dehydrogenasen des Organismus *Thauera butanivorans* ermöglicht:

Für den Organismus *Thauera butanivorans* sind zwei Alkohol Dehydrogenasen des Butan Metabolismus untersucht (Vangnai and Arp, 2001, 147, 745-756; Vangnai et al., 2002, J. Bacteriol. 184, 1916-1924), BOH (Accession Nummer AF326086) und BDH (Accession Nummer 355798). Wenn eine der beiden Gene ausgeschaltet wurde, konnte auf Butan ein verringertes Wachstum beobachtet werden. Wenn beide Gene ausgeschaltet wurden, war kein Wachstum mehr auf Butan festzustellen. Bei Zellen der Doppelmutante, die zunächst auf Citrat angezogen und dann mit Butan inkubiert wurden, könnten Spuren von Butanol gefunden werden (<< 1 ppm). Eine Butyraldehyd Bildung wurde nicht beschrieben. *In-vitro* wurde für beide Enzyme neben der primären Butanol Dehydrogenase Aktivität auch eine verringerte Butyraldehyd Dehydrogenase Aktivität gemessen.

Aufgabe der Erfindung war es, Organismen bereitzustellen, die hervorragende Biokatalysatoren zur Herstellung von kurzkettigen Aldehyden darstellen.

### Beschreibung der Erfindung

Überraschenderweise wurde gefunden, dass die im Folgenden beschriebenen Mikroorganismen einen Beitrag zur Lösung der gestellten Aufgabe leisten können.

Gegenstand der vorliegenden Erfindung sind daher Mikroorganismen mit einer ersten genetischen Modifikation, die zur Reduktion einer Butyraldehyd-Dehydrogenase-Aktivität einer oder mehrerer bestimmter Proteinsequenzen führt.

Ein weiterer Gegenstand der Erfindung ist ein Verfahren zur Herstellung von kurzkettigen Aldehyden unter Einsatz vorgenannter Organismen.

Ein Vorteil der vorliegenden Erfindung ist es, dass die Raum-Zeit-Ausbeute und Kohlenstoffausbeute des Verfahrens erhöht ist, verglichen zu Mikroorganismen, die keine erste genetischen Modifikation enthalten, welche zur Reduktion einer Butyraldehyd-Dehydrogenase-Aktivität einer oder mehrerer bestimmter Proteinsequenzen führt.

Ein weiterer Vorteil der vorliegenden Erfindung ist, dass die Produktkonzentration im Kulturüberstand erhöht wird, so dass eine effiziente Aufarbeitung erleichtert wird.

Gegenstand der vorliegenden Erfindung ist somit ein Mikroorganismus aufweisend eine erste genetische Modifikation, so dass er verglichen zu seinem Wildtyp eine erniedrigte Aktivität eines Enzymes E₁ aufweist, ausgewählt aus mindestens einem der Gruppe der Proteine aufweisend die, bevorzugt bestehend aus den, Polypeptidsequenzen Seq ID Nr. 2, Seq ID Nr. 4, Seq ID Nr. 6, Seq ID Nr. 8, Seq ID Nr. 10, Seq ID Nr. 12, Seq ID Nr. 14, Seq ID Nr. 16, Seq ID Nr. 18, Seq ID Nr. 20, Seq ID Nr. 22, Seq ID Nr. 24, Seq ID Nr. 26 und Seq ID Nr. 28,
sowie Polypeptidsequenzen bei denen bis zu 5%, bevorzugt bis zu 4%, insbesondere bis zu 3%, besonders bevorzugt bis zu 2%, insbesondere besonders bevorzugt bis zu 1%, der Aminosäurereste gegenüber Seq ID Nr. 2, Seq ID Nr. 6, Seq ID Nr. 8, Seq ID Nr. 18, Seq ID Nr. 22, Seq ID Nr. 24, und Seq ID Nr. 28,
sowie bei denen bis zu 2%, bevorzugt bis zu 1%, der Aminosäurereste gegenüber Seq ID Nr. 4, Seq ID Nr. 10, Seq ID Nr. 12, Seq ID Nr. 14, Seq ID Nr. 16 und Seq ID Nr. 26 durch Deletion, Insertion, Substitution oder eine Kombination davon verändert sind
und die noch mindestens 50%, bevorzugt noch mindestens 65%, besonders bevorzugt noch mindestens 80%, insbesondere noch mindestens 90%, der enzymatischen Aktivität des Enzyms mit der jeweiligen Bezugssequenz Seq ID Nr. 2, Seq ID Nr. 4, Seq ID Nr. 6, Seq ID Nr. 8, Seq ID Nr. 10, Seq ID Nr. 12, Seq ID Nr. 14, Seq ID Nr. 16, Seq ID Nr. 18, Seq ID Nr. 20, Seq ID Nr. 22, Seq ID Nr. 24, Seq ID Nr. 26 und Seq ID Nr. 28, besitzen,
wobei unter enzymatische Aktivität für ein Enzym E₁ die Fähigkeit verstanden wird, Butanal, Wasser und NAD+, NADP+, FAD oder oxidiertes PQQ zu n-Butansäure und NADH, NADPH, FADH oder reduziertem PQQ umzusetzen.

Unter einem "Wildtyp" einer Zelle wird im Zusammenhang mit der vorliegenden Erfindung bevorzugt der Ausgangsstamm verstanden, aus dem die erfindungsgemäße Zelle durch gentechnische Manipulation an den genetischen Elementen, die für die Aktivitäten der Enzyme der genannten Seq ID Nr. verantwortlich sind, hervorgegangen ist.

Der Begriff "erniedrigte Aktivität eines Enzyms" ist vorzugsweise als erniedrigte intrazelluläre Aktivität zu verstehen.

Es ist trivial und nur der Vollständigkeit halber erwähnt, dass, um eine verglichen zum Wildtyp einer Zelle gesteigerte oder erniedrigte Aktivität zu bestimmen, eine Wildtyp-Referenz-Kultur eingesetzt wird, die denselben Bedingungen ausgesetzt wurde, wie die der zu bestimmende Probe.

Änderungen von Aminosäureresten einer gegebenen Polypeptidsequenz, die zu keinen wesentlichen Änderungen der Eigenschaften und Funktion des gegebenen Polypeptides führen, sind dem Fachmann bekannt. So können beispielsweise sogenannte konservierte Aminosäuren gegeneinander ausgetauscht werden; Beispiele für solche geeigneten Aminosäuresubstitutionen sind: Ala gegen Ser; Arg gegen Lys; Asn gegen Gin oder His; Asp gegen Glu; Cys gegen Ser; Gin gegen Asn; Glu gegen Asp; Gly gegen Pro; His gegen Asn oder Gin; Ile gegen Leu oder Val; Leu gegen Met oder Val; Lys gegen Arg oder Gin oder Glu; Met gegen Leu oder Ile; Phe gegen Met oder Leu oder Tyr; Ser gegen Thr; Thr gegen Ser; Trp gegen Tyr; Tyr gegen Trp oder Phe; Val gegen Ile oder Leu. Ebenso ist bekannt, dass Änderungen besonders am N- oder C-Terminus eines Polypeptides in Form von beispielsweise Aminosäureinsertionen oder -deletionen oft keinen wesentlichen Einfluss auf die Funktion des Polypeptides ausüben.

Die "Aminosäure-Identität" im Zusammenhang mit den im Rahmen der Erfindung genutzten Enzyme wird mit Hilfe bekannter Verfahren bestimmt. Generell werden besondere Computerprogramme mit Algorithmen unter Berücksichtigung spezieller Erfordernisse verwendet. Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG- Programmpaket, einschließlich

GAP (Deveroy, J. et al., Nucleic Acid Research 12 (1984), Seite 387, Genetics Computer Group University of Wisconsin, Medicine (Wi), und BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).

Der bekannte Smith-Waterman Algorithmus kann ebenso zur Bestimmung der von Identitäten verwendet werden.

Bevorzugte Parameter für die Bestimmung der "Aminosäure-Identität" sind bei Verwendung des BLASTP-Programms (Altschul, S. et al., Journal of Molecular Biology 215 (1990), Seiten 403-410:

| | |
|---|---|
| Expect Threshold: | 10 |
| Word size: | 3 |
| Matrix: | BLOSUM62 |
| Gap costs: | Existence: 11; Extension: 1 |
| Compositional adjustments: | Conditional compositional score matrix adjustment |

Die vorstehenden Parameter sind die default-Parameter im Aminosäuresequenzvergleich. Das GAP-Programm ist ebenso zur Verwendung mit den vorstehenden Parametern geeignet. Eine Identität von 60% gemäß dem vorstehenden Algorithmus bedeutet im Zusammenhang mit der vorliegenden Erfindung 60% Identität. Das gleiche gilt für höhere Identitäten.

Unter der verwendeten Formulierung "verringerte Aktivität eines Enzyms Eₓ" im Zusammenhang mit der vorliegenden Erfindung wird dementsprechend vorzugsweise eine um einen Faktor von mindestens 0,5, besonders bevorzugt von mindestens 0,1, darüber hinaus bevorzugt von mindestens 0,01, darüber hinaus noch mehr bevorzugt von mindestens 0,001 und am meisten bevorzugt von mindestens 0,0001 verringerte Aktivität verstanden. Die Formulierung "verringerte Aktivität" beinhaltet auch keine detektierbare Aktivität ("Aktivität von null"). Die Verminderung der Aktivität eines bestimmten Enzyms kann beispielsweise durch gezielte Mutation oder durch andere, dem Fachmann bekannte Maßnahmen zur Verminderung der Aktivität eines bestimmten Enzyms erfolgen.

Verfahren zur Verringerung von enzymatischen Aktivitäten in Mikroorganismen sind dem Fachmann bekannt. Insbesondere molekularbiologische Techniken bieten sich hier an. Enzymaktivitätsverringerung speziell für *Thauera,* insbesondere zum Unterbrechen spezieller Gene findet der Fachmann beispielsweise in Vangnai and Arp, 2001, 147, 745-756.

Im Zusammenhang mit der vorliegenden Erfindung werden die Begriff n-Butanal, n-Butyraldehyd, Butanal und Butyraldehyd synonym verwendet.

Alle angegebenen Prozent (%) sind, wenn nicht anders angegeben, Massenprozent.

Erfindungsgemäß bevorzugte Mikroorganismen sind dadurch gekennzeichnet, dass die Verringerung der enzymatischen Aktivität erreicht wird durch Modifikation eines Genes umfassend eine der Nukleinsäuresequenzen, die für die Polypeptidsequenzen Seq ID Nr. 2, Seq ID Nr. 4, Seq ID Nr. 6, Seq ID Nr. 8, Seq ID Nr. 10, Seq ID Nr. 12, Seq ID Nr. 14, Seq ID Nr. 16, Seq ID Nr. 18, Seq ID Nr. 20, Seq ID Nr. 22, Seq ID Nr. 24, Seq ID Nr. 26 und Seq ID Nr. 28 sowie die oben genannten Sequenzen mit entsprechenden Sequenzidentitäten kodieren, insbesondere Seq ID Nr. 1, Seq ID Nr. 3, Seq ID Nr. 5, Seq ID Nr. 7, Seq ID Nr. 9, Seq ID Nr. 11, Seq ID Nr. 13, Seq ID Nr. 15, Seq ID Nr. 17, Seq ID Nr. 19, Seq ID Nr. 21, Seq ID Nr. 23, Seq ID Nr. 25 und Seq ID Nr. 27, wobei die Modifikation ausgewählt ist aus der Gruppe umfassend, bevorzugt bestehend aus, Insertion von Fremd-DNA in das Gen, Deletion mindestens von Teilen des Gens, Punktmutationen in der Gensequenz, RNA-Interferenz (siRNA), antisense-RNA oder Modifikation (Insertion, Deletion oder Punktmutationen) von regulatorischen Sequenzen, wie etwa Promotoren und Terminatoren oder von Ribosomenbindestellen, welche das Gen flankieren.

Unter Fremd-DNA ist in diesem Zusammenhang jegliche DNA-Sequenz zu verstehen, die dem Gen (und nicht dem Organismus) "fremd" ist, d.h. auch endogene DNA-Sequenzen können in diesem Zusammenhang als "Fremd-DNA" fungieren.

In diesem Zusammenhang ist es insbesondere bevorzugt, dass das Gen durch Insertion eines Selektionsmarkergens unterbrochen wird, somit die Fremd-DNA ein Selektionsmarkergen ist, wobei bevorzugt die Insertion durch homologe Rekombination in den Genlocus erfolgte.

Unter der enzymatischen Aktivität für ein Enzym E₁ wird insbesondere die Fähigkeit verstanden, Butanal, Wasser und NAD+, NADP+, FAD oder oxidiertes PQQ zu n-Butansäure und NADH, NADPH, FADH oder reduziertem PQQ umzusetzen.

Die Bestimmung der Enzymaktivität des Enzyms E₁ wird basierend auf dem Prinzip des optischenzymatischen Tests nach Warburg und Christian, welche vom Lambert-Beerschen-Gesetz abgeleitet ist, berechnet. Dabei wird 1 Unit (U) als die Menge Enzym definiert, durch die 1 µmol Substrat in einer Minute umgesetzt wird. Ein Ansatz zur Messung der Aktivität eines NAD(P)⁺-abhängigen Enzyms E₁ kann beispielsweise ein Volumen von 1 mL besitzen und enthält 50 mM Kaliumphosphatpuffer pH 8, 1 mM DTT, 2 mM Butyraldehyd und 4 mM NAD⁺ oder NADP⁺. Die Messung der Extinktionszunahme bei 340 nm erfolgt nach Zugabe der Enzymlösung über einen Zeitraum von 60 s bei einer Temperatur von 37 °C. Ein Ansatz zur Messung der Aktivität eines FAD- oder PQQ-abhängigen Enzyms E₁ kann beispielsweise ein Volumen von 1 mL besitzen und enthält 10 µmol Kaliumferricyanid, 0,5 mL Mcllvaine-Puffer, pH 4,5, 1 mM DTT und 2 mM Butyraldehyd. Die Reaktion wird durch Zugabe der Enzymlösung gestartet, 10 min bei 25°C inkubiert und durch Zugabe von 0,5 mL Eisen-Dupanol-Reagenz gestoppt. Dann werden 3,5 mL Wasser zugegeben und für weitere 30 min bei 25°C inkubiert. Anschließend wird die Extinktion bei 660 nm gemessen.

Ein erfindungsgemäß bevorzugter Mikroorganismus ist dadurch gekennzeichnet, dass er eine zweite genetische Modifikation aufweist, so dass er verglichen zu seinem Wildtyp eine erniedrigte Aktivität eines Enzymes E₁ aufweist, ausgewählt aus mindestens einem der Proteine aufweisend die Polypeptidsequenzen Seq ID Nr. 30 und Seq ID Nr. 32,
sowie Polypeptidsequenzen bei denen bis zu 10%, bevorzugt bis zu 5%, insbesondere bis zu 3%, besonders bevorzugt bis zu 2%, insbesondere besonders bevorzugt bis zu 1%, der Aminosäurereste gegenüber Seq ID Nr. 30 und Seq ID Nr. 32 durch Deletion, Insertion, Substitution oder eine Kombination davon verändert sind
und die noch mindestens 50%, bevorzugt noch mindestens 65%, besonders bevorzugt noch mindestens 80%, insbesondere noch mindestens 90 %, der enzymatischen Aktivität des Enzyms mit der jeweiligen Bezugssequenz Seq ID Nr. 30 und Seq ID Nr. 32 besitzen,
Dies hat den technischen Effekt, dass die Ausbeute an Aldehyd gesteigert werden kann. Erfindungsgemäß ist ein Mikroorganismus bevorzugt, der ausgewählt ist aus der Gruppe umfassend, bevorzugt bestehend aus, Hefen und Bakterien, bevorzugt gram negativen Bakterien, insbesondere ausgewählt aus der Gattung *Thauera,* wobei *Thauera butanivorans besonders bevorzugt ist..*

Zur weiteren Ausbeutesteigerung kann es vorteilhaft sein und ist daher bevorzugt, wenn der erfindungsgemäße Mikroorganismus eine dritte genetische Modifikation aufweist, so dass er verglichen zu seinem Wildtyp eine gesteigerte Aktivität mindestens eines Enzymes E₂ aufweist, ausgewählt aus der Gruppe der Alkanmonooxygenasen der EC 1.14.15.3, wobei unter der enzymatischen Aktivität für ein Enzym E₂ insbesondere die Fähigkeit verstanden wird, Butan, Wasser und NAD+, NADP+ oder oxidiertes PQQ zu Butanol und NADH, NADPH oder reduziertem PQQ umzusetzen.

Der Begriff "gesteigerte Aktivität eines Enzyms" im Zusammenhang mit der vorliegenden Erfindung ist vorzugsweise als gesteigerte intrazelluläre Aktivität zu verstehen.

Grundsätzlich lässt sich eine Steigerung der enzymatischen Aktivität dadurch erzielen, dass man die Kopiezahl der Gensequenz bzw. der Gensequenzen erhöht, welche für das Enzym kodieren, einen starken Promotor oder eine verbesserte Ribosomenbindungsstelle verwendet, eine negative Regulation der Genexpression, beispielsweise durch Transkriptionsregulatoren, abschwächt, oder eine positive Regulation der Genexpression, beispielsweise durch Transkriptionsregulatoren, verstärkt, die Kodonnutzung des Gens verändert, auf verschiedene Art und Weise die Halbwertszeit der mRNA oder des Enzyms erhöht, die Regulation der Expression des Gens modifiziert oder ein Gen oder Allel nutzt, das für ein entsprechendes Enzym mit einer gesteigerten Aktivität kodiert und gegebenenfalls diese Maßnahmen kombiniert. Erfindungsgemäß gentechnisch veränderte Mikroorganismen werden beispielsweise durch Transformation, Transduktion, Konjugation oder eine Kombination dieser Methoden mit einem Vektor erzeugt, der das gewünschte Gen, ein Allel dieses Gens oder Teile davon und gegebenenfalls einen die Expression des Gens ermöglichenden Promotor enthält. Die heterologe Expression wird insbesondere durch Integration des Gens oder der Allele in das Chromosom der Zelle oder einen extrachromosomal replizierenden Vektor erzielt.

Einen Überblick über die Möglichkeiten zur Erhöhung der Enzym-Aktivität in Zellen am Beispiel der Pyruvat-Carboxylase gibt DE-A-100 31 999, die hiermit als Referenz eingeführt wird und deren Offenbarungsgehalt hinsichtlich der Möglichkeiten zur Erhöhung der Enzym-Aktivität in Zellen einen Teil der Offenbarung der vorliegenden Erfindung bildet.

Die Expression der vorstehend und aller nachfolgend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei coryneformen Bakterien und zur Identifizierung der Proteine ist die von Hermann et al. (Electrophoresis, 22: 1712.23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschliessende optische Auswertung mit entsprechender Software zur Konzentrationsbestimmung (Lohaus und Meyer (1989) Biospektrum, 5: 32-39; Lottspeich (1999) Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären enzymatischen Aktivitäten können nach verschiedenen beschriebenen Methoden (Donahue et al. (2000) Journal of Bacteriology 182 (19): 5624-5627; Ray et al. (2000) Journal of Bacteriology 182 (8): 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al., Electophoresis, 22: 1712-23 (2001), Lohaus et al., Biospektrum 5 32-39 (1998), Lottspeich, Angewandte Chemie 111: 2630-2647 (1999) und Wilson et al., Journal of Bacteriology 183: 2151-2155 (2001) beschriebenen Methoden. Wird die Erhöhung der Enzymaktivität durch Mutation des endogenen Gens bewerkstelligt, so können derartige Mutationen entweder nach klassischen Methoden ungerichtet erzeugt werden, wie etwa durch UV-Bestrahlung oder durch mutationsauslösende Chemikalien, oder gezielt mittels gentechnologischer Methoden wie Deletion(en), Insertion(en) und/oder Nukleotidaustausch(e). Durch diese Mutationen werden veränderte Zellen erhalten. Besonders bevorzugte Mutanten von Enzymen sind insbesondere auch solche Enzyme, die nicht mehr oder zumindest im Vergleich zum Wildtyp-Enzym vermindert feedback-, produkt- oder substrat-inhibierbar sind.
Wird die Erhöhung der Enzymaktivität durch Erhöhung der Synthese eines Enzyms bewerkstelligt, so erhöht man beispielsweise die Kopiezahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression zu jedem beliebigen Zeitpunkt zu steigern. Des Weiteren können dem Enzym-Gen als regulatorische Sequenzen aber auch sogenannte "Enhancer" zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopiezahl vor oder sind im Chromosom integriert und amplifiziert. Alternativ kann weiterhin eine Überexpression der betreffenden Gene durch Veränderung der Medienzusammensetzung und Kulturführung erreicht werden.
Zur Erhöhung der Expression der jeweiligen Gene werden zum Beispiel episomale Plasmide eingesetzt. Als Plasmide bzw. Vektoren kommen im Prinzip alle dem Fachmann für diesen Zweck zur Verfügung stehenden Ausführungsformen in Frage. Derartige Plasmide und Vektoren können z. B. den Broschüren der Firmen Novagen, Promega, New England Biolabs, Clontech oder Gibco BRL entnommen werden. Weitere bevorzugte Plasmide und Vektoren können gefunden werden in: Glover, D. M. (1985) DNA cloning: a practical approach, Vol. I-III, IRL Press Ltd. , Oxford; Rodriguez, R.L. und Denhardt, D. T (eds) (1988) Vectors : a survey of molecular cloning vectors and their uses, 179-204, Butterworth, Stoneham; Goeddel, D. V. (1990) Systems for heterologous gene expression, Methods Enzymol. 185, 3-7; Sambrook, J.; Fritsch, E. F. und Maniatis, T. (1989), Molecular cloning: a laboratory manual, 2nd ed., Cold Spring Harbor Laboratory Press, New York. Der Plasmidvektor, der das zu amplifizierende Gen enthält, wird anschließend durch Konjugation oder Transformation in den gewünschten Stamm überführt. Die Methode der Konjugation ist beispielsweise bei Schäfer et al., Applied and Environmental Microbiology 60: 756-759 (1994) beschrieben. Methoden zur Transformation sind beispielsweise bei Thierbach et al., Applied Microbiology and Biotechnology 29: 356-362 (1988), Dunican und Shivnan, Bio/Technology 7: 1067-1070 (1989) und Tauch et al., FEMS Microbiology Letters 123: 343-347 (1994) beschrieben. Nach homologer Rekombination mittels eines "cross-over"-Ereignisses enthält der resultierende Stamm mindestens zwei Kopien des betreffenden Gens.
Bevorzugte erfindungsgemäße Mikroorganismen erreichen demnach eine "verglichen zu ihrem Wildtyp gesteigerte Aktivität eines Enzyms Eₓ" durch eine Überexpression des Enzyms Eₓ. Unter der vorstehend und in den nachfolgenden Ausführungen verwendeten Formulierung "eine verglichen zum Wildtyp gesteigerte Aktivität eines Enzyms Eₓ" ist vorzugsweise stets eine um einen Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms Eₓ zu verstehen. Weiterhin umfasst die erfindungsgemäße Zelle, welche "verglichen zu ihrem Wildtyp gesteigerte Aktivität eines Enzyms Eₓ" aufweist, insbesondere auch eine Zelle, deren Wildtyp keine oder zumindest keine nachweisbare Aktivität dieses Enzyms Eₓ aufweist und die erst nach Erhöhung der Enzymaktivität, beispielsweise durch Überexpression, eine nachweisbare Aktivität dieses Enzyms Eₓ zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Synthese des Enzyms Eₓ induziert wird.

Bevorzugte Alkanmonooxygenasen der EC 1.14.15.3, deren Aktivität verglichen zum Wildtyp erfindungsgemäß bevorzugt gesteigert ist, sind ausgewählt aus der Gruppe der Oxidoreduktasen vom AlkB-Typ, die insbesondere in der EP2788493 offenbart werden. Die in der EP2788493 als bevorzugt ausgewiesenen Oxidoreduktasen vom AlkB-Typ werden auch vorliegend erfindungsgemäß bevorzugt eingesetzt.

Zur weiteren Ausbeutesteigerung kann es vorteilhaft sein und ist daher bevorzugt, wenn der erfindungsgemäße Mikroorganismus eine vierte genetische Modifikation aufweist, so dass er verglichen zu seinem Wildtyp eine gesteigerte Aktivität mindestens eines Enzymes E₃ aufweist, ausgewählt aus mindestens einem der Proteine aufweisend die Polypeptidsequenzen WP_010966572.1, AAA23206.1, WP_034583347.1, WP_010966571.1, WP_034583350.1, WP_015616006.1, WP_026881898.1, WP_021655913.1, WP_010235517.1, WP_004455109.1, WP_026881497.1, WP_003486653.1, KG012652.1, WP_030034651.1, WP_003491005.1, WP_021106349.1, WP_012342913.1, WP_003362274.1, WP_003358614.1, WP_003401772.1, WP_014520521.1, WP_029452360.1, WP_007063868.1, WP_011949005.1, WP_011988245.1, WP_021284504.1, WP_025775882.1, WP_031369326.1, WP_031368191.1, WP_032077777.1, WP_023437247.1, WP_011098713.1, WP_033064364.1, WP_017415560.1, WP_017752786.1, WP_039657965.1, WP_007785525.1, WP_027638413.1, WP_014554180.1, WP_039636789.1, WP_009167516.1, WP_035786301.1, WP_027098116.1, WP_017352673.1, WP_012424272.1, WP_039769752.1, WP_035285659.1, WP_017213095.1, EEH98657.1, WP_026886109.1, WP_012058635.1, WP_041084633.1, WP_012449920.1, AHF06812.1, WP_040191544.1, WP_003371527.1, WP_021135040.1, WP_039231326.1, WP_008689032.1, WP_039221935.1, WP_003380845.1, WP_003375593.1, WP_008978928.1, WP_039229275.1, WP_039281897.1, WP_003363691.1, WP_039238494.1, WP_039256218.1, WP_039250085.1, WP_011721631.1, WP_013724912.1, WP_029169607.1, WP_039257472.1, WP_022420904.1, WP_009274567.1, WP_004798344.1, WP_039310701.1, WP_039277494.1, WP_039259348.1, WP_002596985.1, WP_022156508.1, WP_009269781.1, WP_008818561.1, WP_002607351.1, WP_006440315.1, WP_003500280.1, WP_021640844.1, WP_003509166.1, WP_024739697.1, WP_009298920.1, WP_007788299.1, WP_007049187.1, WP_035146363.1, WP_038322630.1, WP_006189666.1, WP_016518391.1, WP_012200477.1, WP_016524303.1, WP_010076251.1 und AIW89961.1, sowie Polypeptidsequenzen bei denen bis zu 10%, bevorzugt bis zu 5%, insbesondere bis zu 3%, besonders bevorzugt bis zu 2%, insbesondere besonders bevorzugt bis zu 1%, der Aminosäurereste gegenüber den vorgenannter Accession Nummern durch Deletion, Insertion, Substitution oder eine Kombination davon verändert sind
und die noch mindestens 50%, bevorzugt noch mindestens 65%, besonders bevorzugt noch mindestens 80%, insbesondere noch mindestens 90 %, der enzymatischen Aktivität des Enzyms mit der jeweiligen vorgenannten Accession Nummer besitzen,
Unter der enzymatischen Aktivität für ein Enzym E₃ wird insbesondere die Fähigkeit verstanden, Butanol, Wasser und NAD+, NADP+ oder oxidiertes PQQ zu Butanal und NADH, NADPH oder reduziertem PQQ umzusetzen.

Die im Zusammenhang mit der vorliegenden Erfindung angeführten Accession-Nummern entsprechen den ProteinBank Datenbank-Einträgen des NCBI mit Datum vom 10.02.2015; in der Regel wird vorliegend die Versionsnummer des Eintrages durch ".Ziffer" wie beispielsweise ".1", kenntlich gemacht.

Zur weiteren Ausbeutesteigerung kann es vorteilhaft sein und ist daher bevorzugt, wenn der erfindungsgemäße Mikroorganismus eine fünfte genetische Modifikation aufweist, so dass er verglichen zu seinem Wildtyp eine gesteigerte Aktivität eines mindestens Enzymes E₄ aufweist, welches den Import eines Alkans mit einer Kettenlänge von 2 bis 9, insbesondere 4 bis 6, bevorzugt 4, Kohlenstoffatomen, insbesondere Butan, in den Mikroorganismus hinein katalysiert. Bevorzugte Enzyme E₄ sind ausgewählt aus der Gruppe der Polypeptidsequenzen kodiert durch ein alkl Gen.

Unter dem Begriff "alkL-Gen" werden im Zusammenhang mit der vorliegenden Erfindung Gene verstanden, die für Proteine kodieren, die mindestens eine der nachfolgenden beiden Bedingungen erfüllen:
1.) Das Protein wird als ein Mitglied der Superfamilie der OmpW-Proteine (Proteinfamilie 3922 in der "Conserved Domain Database" (CDD) des "National Center for Biotechnology Information" (NCBI)) identifiziert, wobei diese Zuordnung durch ein Alignment der Aminosäuresequenz des Proteins mit den in der CDD des NCBI vorhandenen Datenbankeinträgen, die bis zum 22.03.2010 hinterlegt wurden, unter Nutzung der Standardsuchparameter, einem E-Wert (englisch "e-value") kleiner 0,01 und unter Verwendung des Algorithmus "blastp 2.2.23+", erfolgt,
2.) bei einer Suche nach in der betreffenden Aminosäuresequenz enthaltenen konservierten Proteindomänen in der NCBI CDD (Version 2.20) mittels RPS-BLAST wird die Präsenz der konservierten Domäne "OmpW, Outer membrane protein W" (COG3047) mit einem E-Wert (englisch "e-value") kleiner 1 x 10⁻⁵ festgestellt (englisch "domain hit").

Besonders bevorzugte Enzyme E₄ sind ausgewählt aus der Gruppe der Polypeptidsequenzen kodiert von einem alkL-Gen aus Organismen ausgewählt aus der Gruppe der gram-negativen Bakterien, insbesondere der Gruppe enthaltend, bevorzugt bestehend aus *Pseudomonas* sp., *Azotobacter* sp., *Desulfitobacterium* sp., *Burkholderia* sp., bevorzugt *Burkholderia cepacia, Xanthomonas* sp., *Rhodobacter* sp., *Ralstonia* sp., *Delftia* sp.und *Rickettsia* sp., *Oceanicaulis* sp., *Caulobacter* sp., *Marinobacter* sp. und *Rhodopseudomonas* sp., bevorzugt *Pseudomonas putida, Oceanicaulis alexandrii, Marinobacter aquaeolei,* insbesondere *Pseudomonas putida* GPo1 und P1, *Oceanicaulis alexandrii* HTCC2633, *Caulobacter* sp. K31 und *Marinobacter aquaeolei* VT8, ganz besonders bevorzugt aus *Pseudomonas putida* GPo1 und P1 und aus Proteinen aufweisend die Polypeptidsequenzen YP_009076010.1, WP_032489564.1, WP_022963140.1, CAB69081.1, WP_020798869.1, WP_040110242.1, AJA11745.1, EXS68633.1, AHI32885.1, WP_022991857.1, WP_011783980.1, WP_040613339.1, EAP90277.1, WP_008170303.1, WP_012275116.1, WP_023008031.1, WP_036784183.1, WP_037483133.1 und WP_023010670.1, sowie den Polypeptidsequenzen bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber den vorgenannter Accession Nummern durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der vorgenannter Accession Nummer besitzen, wobei unter enzymatischer Aktivität für ein Enzym E₄ die Fähigkeit verstanden wird, ein Alkan mit einer Kettenlänge von 2 bis 9, insbesondere 4 bis 6, bevorzugt 4, Kohlenstoffatomen, insbesondere Butan, in den Mikroorganismus zu importieren.

Ein weiterer Gegenstand ist ein Verfahren zur Herstellung von Aldehyden mit einer Kettenlänge von 2 bis 9, insbesondere 4 bis 6, bevorzugt 4, Kohlenstoffatomen umfassend die Verfahrensschritte.
A) In Kontakt Bringen der erfindungsgemäßen Mikroorganismen mit einem Medium enthaltend ein Substrat mit einer Kettenlänge von 2 bis 9, insbesondere 4 bis 6, bevorzugt 4, Kohlenstoffatomen unter Umwandlung des Substrates zu Aldehyd und gegebenenfalls
B) Aufreinigen des Aldehyds.

Bevorzugt eingesetzte Substrate sind Alkane mit einer Kettenlänge von 2 bis 9, insbesondere 4 bis 6, bevorzugt 4, Kohlenstoffatomen.

Das erfindungsgemäße Verfahren wird insbesondere zur Herstellung von n-Butanal eingesetzt. In diesem Zusammenhang wird als Substrat bevorzugt Butan, insbesondere wenn der eingesetzte Mikroorganismus über die dritte erfindungsgemäße genetische Modifikation verfügt, und/oder Butanol eingesetzt.

Die erfindungsgemäße Verfahren kann hinsichtlich des in Kontakt Bringens kontinuierlich oder diskontinuierlich im *batch*-Verfahren (Satzkultivierung) oder im *fed-batch*-Verfahren (Zulaufverfahren) oder *repeated-fed-batch*-Verfahren (repetitives Zulaufverfahren) zum Zwecke der Produktion von Aldehyden durchgeführt werden. Eine Zusammenfassung über bekannte Kultivierungsmethoden sind im Lehrbuch von Chmiel ("Bioprozesstechnik 1. Einführung in die Bioverfahrenstechnik" (Gustav Fischer Verlag, Stuttgart, 1991)) oder im Lehrbuch von Storhas

### ("Bioreaktoren und periphere Einrichtungen", Vieweg Verlag, Braunschweig/Wiesbaden, 1994) beschrieben.

Das in dem erfindungsgemäßen Verfahren eingesetzte Medium muss in geeigneter Weise den Ansprüchen der jeweiligen Stämme genügen. Beschreibungen von Kulturmedien verschiedenerMikroorganismen sind im Handbuch " Manual of Methods for General Bacteriology" der American Society for Bacteriology (Washington D. C., USA, 1981) enthalten.

Als Kohlenstoffquelle können Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Methanol, Kohlenwasserstoffe wie Methan, Aminosäuren wie L-Glutamat oder L-Valin oder organische Säuren wie z. B. Essigsäure verwendet werden. Diese Stoffe können einzeln oder als Mischung verwendet werden. Besonders bevorzugt ist der Einsatz von Kohlehydraten, insbesondere Monosaccharide, Oligosaccharide oder Polysaccharide, wie dies in US 6,01,494 und US 6,136,576 beschrieben ist, von C5-Zuckern oder von Glycerin.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden.

Das erfindungsgemäßen Verfahrens kann bei Atmosphärendruck durchgeführt. Im Falle des Einsatzes gasförmiger Substrate kann es jedoch vorteilhaft sein, Verfahrensschritt A) bei erhöhtem Druck durchzuführen. In einer bevorzugten Ausführungsform beträgt der Druck in Verfahrensschritt A) mehr als 1,5, 2, 3 oder 4 bar. In einer weiteren Ausführungsform beträgt der Druck in Verfahrensschritt A) 2 bis 20, bevorzugt 1,5 bis 5, am bevorzugtesten 1,2 bis 2 bar.

Es ist erfindungsgemäß bevorzugt, wenn Verfahrensschritt A) in einem Temperaturbereich von 20 °C bis 50 °C, bevorzugt von30 °C bis 37 °C, durchgeführt wird.

Es kann vorteilhaft sein, in dem erfindungsgemäß Verfahren dem Medium ein weiteres organisches Lösungsmittel zuzusetzen. Dieses kann in Verfahrensschritt B) vorteilhaft dazu eingesetzt werden, das Aldehyd zu extrahieren. Eine Führung als *in-situ* Extraktion ist ebenfalls möglich und erfindungsgemäß bevorzugt. Bevorzugte organische Lösungsmittel in diesem Zusammenhang sind Hexadekan, 1-Octanol, 5-Methylfurfural und Furfural.

Verfahrensschritt B) erfolgt vorzugsweise kontinuierlich, wobei es in diesem Zusammenhang weiterhin bevorzugt ist, auch Verfahrensschritt A) kontinuierlich durchzuführen, so dass der gesamte Prozess von der Herstellung des Aldehyds bis zur deren Aufreinigung aus dem Medium kontinuierlich durchgeführt werden kann. Zur kontinuierlichen Aufreinigung der Aldehyde aus dem Medium wird dieses kontinuierlich über eine Vorrichtung zur Abtrennung der im Verfahren eingesetzten Mikroorganismen, vorzugsweise über einen Filter mit einer Ausschlussgröße in einem Bereich von 20 bis 200 kDa geführt, in dem eine Fest/Flüssig-Trennung stattfindet. Denkbar ist auch der Einsatz einer Zentrifuge, einer geeigneten Sedimentationsvorrichtung oder eine Kombination dieser Vorrichtungen, wobei es besonders bevorzugt ist, zumindest einen Teil der Mikroorganismen zunächst durch Sedimentation abzutrennen und anschließend die von den Mikroorganismen teilweise befreites Medium einer Ultrafiltration oder Zentrifugationsvorrichtung zuzuführen.

Das hinsichtlich des Aldehyds angereicherte Erzeugnis wird nach der Abtrennung der Mikroorganismen einer vorzugsweise mehrstufigen Trennanlage zugeführt. In dieser Trennanlage sind mehrere hintereinander geschaltete Trennstufen vorgesehen, aus denen bevorzugt jeweils Rückführleitungen ausmünden, die zum Fermentationstank zurückgeführt sind. Weiterhin bevorzugt führen aus den jeweiligen Trennstufen Ableitungen heraus. Die einzelnen Trennstufen können nach dem Prinzip der Elektrodialyse, der Umkehrosmose, der Ultrafiltration oder der Nanofiltration arbeiten. In der Regel handelt es sich um Membran-Trenneinrichtungen in den einzelnen Trennstufen. Die Auswahl der einzelnen Trennstufen ergibt sich aus Art und Umfang der Gärungsnebenprodukte und Substratreste.

Verfahrensschritt B) kann auch in der Form einer Elektrodialyse, Umkehrosmose, Ultrafiltration oder Nano-Filtration, in deren Verlauf als Endprodukt eine wässrige Aldehyd-Lösung erhalten wird, ausgeführt werden.

Das erfindungsgemäße Verfahren lässt sich vorteilhaft zu einem Verfahren zur Herstellung von verzweigten Alkoholen mit einer Kettenlänge von 4 bis 18, insbesondere 8 bis 12, bevorzugt 8, Kohlenstoffatomen, insbesondere 2-Ethylhexanol, ausbauen, indem es um die Verfahrensschritte umfassend
C) Aldolkondensation des Aldehyds unter Wasserabspaltung
D) katalytischen Hydrierung
ergänzt wird.

Als Katalysator der Aldolkondensation in Verfahrensschritt C) des erfindungsgemäßen Verfahrens werden bevorzugt wasserlösliche, basische Verbindungen, wie z.B. Hydroxide, Hydrogencarbonate, Carbonate, Carboxylate oder ihre Gemische in Form ihrer Alkali-oder Erdalkaliverbindungen eingesetzt.

Vorzugsweise kommen Alkalihydroxide, wie NaOH, zum Einsatz. Der Katalysator wird bevorzugt gelöst in Wasser eingesetzt, wobei die wässrige Base neben dem organischen Edukt-/Produktgemisch eine zweite flüssige Phase bildet. Der Volumenanteil der wässrigen Phase in Verfahrensschritt C) des erfindungsgemäßen Verfahrens beträgt zwischen 60% und 90%, bevorzugt zwischen 70- 80%, besonders bevorzugt 75% bezogen auf das Gesamtvolumen des zweiphasigen Reaktionsgemisches im Reaktionsraum . Die Konzentration des Alkalikatalysators in der wässrigen Phase in Verfahrensschritt C) liegt vorzugsweise in einem Bereich von 0,1% bis 15 Gew.-%, besonders bevorzugt von 0,2 - 5 Gew-%, insbesondere von 1-3 Gew.-% bezogen auf die gesamte wässrige Phase. Das während der Reaktion freigesetzte Reaktionswasser reichert sich in der schwereren, wässrigen Phase an. Die organischen Edukte und Produkte bilden die leichtere, organische Phase. Die Reaktionstemperatur der Aldolkondensation liegt typischerweise im Bereich von 80 bis 180 °C. Die Reaktion erfolgt in der Regel unter erhöhten Druck, der durch die Zusammensetzung der organischen und wässrigen Phase im Reaktor bei vorliegender Temperatur bestimmt wird und liegt im Bereich von 1,5 bis 20 bar. Während der Reaktion wird Wärme frei, die aus dem Prozess abgeführt werden muss. Die Reaktionszeit beträgt bevorzugt zwischen 10 bis 20 Minuten. Nach Ablauf der Reaktion wird die Reaktionsmischung bevorzugt in einem Absetzgefäß in eine wässrige Phase und in eine organische Phase durch Phasentrennung aufgetrennt. Das nach Abtrennung der Katalysatorphase erhaltene Produkt kann durch bekannte Verfahren (z.B. Destillation) gereinigt werden.

Das gereinigte Produkt wir anschließend in Verfahrensschritt D) des erfindungsgemäßen Verfahrens bevorzugt in der Flüssigphase hydriert. Die katalytische Hydrierung wird vorzugsweise bei Temperaturen zwischen 50 und 200 °C durchgeführt. Als Katalysator können geeignete Hydrierkatalysatoren eingesetzt werden, beispielsweise geträgerte Cr-Cu oder Ni-Cu Katalysatoren. Bevorzugt wird die katalytische Hydrierung in Reaktoren bei Drücken von 1 bis 100 bar durchgeführt.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

## Patentansprüche

1. Mikroorganismus aufweisend eine erste genetische Modifikation, so dass er verglichen zu seinem Wildtyp eine erniedrigte Aktivität eines Enzymes E₁ aufweist, ausgewählt aus mindestens einem der Gruppe der Proteine aufweisend die Polypeptidsequenzen Seq ID Nr. 2, Seq ID Nr. 4, Seq ID Nr. 6, Seq ID Nr. 8, Seq ID Nr. 10, Seq ID Nr. 12, Seq ID Nr. 14, Seq ID Nr. 16, Seq ID Nr. 18, Seq ID Nr. 20, Seq ID Nr. 22, Seq ID Nr. 24, Seq ID Nr. 26 und Seq ID Nr. 28,
sowie Polypeptidsequenzen bei denen bis zu 5% der Aminosäurereste gegenüber Seq ID Nr. 2, Seq ID Nr. 6, Seq ID Nr. 8, Seq ID Nr. 18, Seq ID Nr. 22, Seq ID Nr. 24, und Seq ID Nr. 28,
sowie bei denen bis zu 2% der Aminosäurereste gegenüber Seq ID Nr. 4, Seq ID Nr. 10, Seq ID Nr. 12, Seq ID Nr. 14, Seq ID Nr. 16 und Seq ID Nr. 26 durch Deletion, Insertion, Substitution oder eine Kombination davon verändert sind
und die noch mindestens 50% der enzymatischen Aktivität des Enzyms mit der jeweiligen Bezugssequenz Seq ID Nr. 2, Seq ID Nr. 4, Seq ID Nr. 6, Seq ID Nr. 8, Seq ID Nr. 10, Seq ID Nr. 12, Seq ID Nr. 14, Seq ID Nr. 16, Seq ID Nr. 18, Seq ID Nr. 20, Seq ID Nr. 22, Seq ID Nr. 24, Seq ID Nr. 26 und Seq ID Nr. 28 besitzen,
wobei unter enzymatische Aktivität für ein Enzym E₁ die Fähigkeit verstanden wird, Butanal, Wasser und NAD+, NADP+, FAD oder oxidiertes PQQ zu n-Butansäure und NADH, NADPH, FADH oder reduziertem PQQ umzusetzen.

2. Mikroorganismus gemäß Anspruch 1, **dadurch gekennzeichnet, dass** er eine zweite genetische Modifikation aufweist, so dass er verglichen zu seinem Wildtyp eine erniedrigte Aktivität eines Enzymes E₁ aufweist, ausgewählt aus mindestens einem der Proteine aufweisend die Polypeptidsequenzen Seq ID Nr. 30 und Seq ID Nr. 32,
sowie Polypeptidsequenzen bei denen bis zu 10% der Aminosäurereste gegenüber Seq ID Nr. 30 und Seq ID Nr. 32 durch Deletion, Insertion, Substitution oder eine Kombination davon verändert sind
und die noch mindestens 50% der enzymatischen Aktivität des Enzyms mit der jeweiligen Bezugssequenz Seq ID Nr. 30 und Seq ID Nr. 32 besitzen,

3. Mikroorganismus gemäß Anspruch 1 oder 2, **dadurch gekennzeichnet, dass** er ausgewählt ist aus der Gruppe umfassend, bevorzugt bestehend aus, Hefen und Bakterien, bevorzugt gram negativen Bakterien, insbesondere ausgewählt aus der Gattung *Thauera.*

4. Mikroorganismus gemäß mindestens einem der vorherigen Ansprüche, **dadurch gekennzeichnet, dass** er eine dritte genetische Modifikation aufweist, so dass er verglichen zu seinem Wildtyp eine gesteigerte Aktivität mindestens eines Enzymes E₂ aufweist, ausgewählt aus der Gruppe der Alkanmonooxygenasen der EC 1.14.15.3, wobei unter der enzymatischen Aktivität für ein Enzym E₂ insbesondere die Fähigkeit verstanden wird, Butan, Wasser und NAD+, NADP+ oder oxidiertes PQQ zu Butanol und NADH, NADPH oder reduziertem PQQ umzusetzen.

5. Mikroorganismus gemäß Anspruch 4, **dadurch gekennzeichnet, dass** die Alkanmonooxygenase ausgewählt ist aus der Gruppe der Oxidoreduktasen vom AlkB-Typ,

6. Mikroorganismus gemäß mindestens einem der vorherigen Ansprüche, **dadurch gekennzeichnet, dass** er eine vierte genetische Modifikation aufweist, so dass er verglichen zu seinem Wildtyp eine gesteigerte Aktivität mindestens eines Enzymes E₃ aufweist, ausgewählt aus mindestens einem der Proteine aufweisend die Polypeptidsequenzen WP_010966572.1, AAA23206.1, WP_034583347.1, WP_010966571.1, WP_034583350.1, WP_015616006.1, WP_026881898.1, WP_021655913.1, WP_010235517.1, WP_004455109.1, WP_026881497.1, WP_003486653.1, KGO12652.1, WP_030034651.1, WP_003491005.1, WP_021106349.1, WP_012342913.1, WP_003362274.1, WP_003358614.1, WP_003401772.1, WP_014520521.1, WP_029452360.1, WP_007063868.1, WP_011949005.1, WP_011988245.1, WP_021284504.1, WP_025775882.1, WP_031369326.1, WP_031368191.1, WP_032077777.1, WP_023437247.1, WP_011098713.1, WP_033064364.1, WP_017415560.1, WP_017752786.1, WP_039657965.1, WP_007785525.1, WP_027638413.1, WP_014554180.1, WP_039636789.1, WP_009167516.1, WP_035786301.1, WP_027098116.1, WP_017352673.1, WP_012424272.1, WP_039769752.1, WP_035285659.1, WP_017213095.1, EEH98657.1, WP_026886109.1, WP_012058635.1, WP_041084633.1, WP_012449920.1, AHF06812.1, WP_040191544.1, WP_003371527.1, WP_021135040.1, WP_039231326.1, WP_008689032.1, WP_039221935.1, WP_003380845.1, WP_003375593.1, WP_008978928.1, WP_039229275.1, WP_039281897.1, WP_003363691.1, WP_039238494.1, WP_039256218.1, WP_039250085.1, WP_011721631.1, WP_013724912.1, WP_029169607.1, WP_039257472.1, WP_022420904.1, WP_009274567.1, WP_004798344.1, WP_039310701.1, WP_039277494.1, WP_039259348.1, WP_002596985.1, WP_022156508.1, WP_009269781.1, WP_008818561.1, WP_002607351.1, WP_006440315.1, WP_003500280.1, WP_021640844.1, WP_003509166.1, WP_024739697.1, WP_009298920.1, WP_007788299.1, WP_007049187.1, WP_035146363.1, WP_038322630.1, WP_006189666.1, WP_016518391.1, WP_012200477.1, WP_016524303.1, WP_010076251.1 und AIW89961.1,
sowie Polypeptidsequenzen bei denen bis zu 10% der Aminosäurereste gegenüber den vorgenannter Accession Nummern durch Deletion, Insertion, Substitution oder eine Kombination davon verändert sind und die noch mindestens 50% der enzymatischen Aktivität des Enzyms mit der jeweiligen vorgenannten Accession Nummer besitzen.

7. Mikroorganismus gemäß mindestens einem der vorherigen Ansprüche, **dadurch gekennzeichnet, dass** er eine fünfte genetische Modifikation aufweist, so dass er verglichen zu seinem Wildtyp eine gesteigerte Aktivität eines mindestens Enzymes E₄ aufweist, welches den Import eines Alkans mit einer Kettenlänge von 2 bis 9, insbesondere 4 bis 6, bevorzugt 4, Kohlenstoffatomen, insbesondere Butan, in den Mikroorganismus hinein katalysiert.

8. Verfahren zur Herstellung von Aldehyden mit einer Kettenlänge von 2 bis 9, insbesondere 4 bis 6, bevorzugt 4, Kohlenstoffatomen umfassend die Verfahrensschritte.
A) In Kontakt Bringen der Mikroorganismen gemäß mindestens einem der Ansprüche 1 bis 4 mit einem Medium enthaltend ein Substrat mit einer Kettenlänge von 2 bis 9, insbesondere 4 bis 6, bevorzugt 4, Kohlenstoffatomen unter Umwandlung des Substrates zu Aldehyd und gegebenenfalls
B Aufreinigen des Aldehyds.

9. Verfahren gemäß Anspruch 8, **dadurch gekennzeichnet, dass** als Substrat ein Alkan eingesetzt wird.

10. Verfahren gemäß Anspruch 8 oder 9, **dadurch gekennzeichnet, dass** der Druck in Verfahrensschritt A) mehr als 1,5, bar beträgt.

11. Verfahren gemäß mindestens einem der Ansprüche 8 bis 10, **dadurch gekennzeichnet, dass** Verfahrensschritt A) in einem Temperaturbereich von 20 °C bis 50 °C durchgeführt wird.

12. Verfahren gemäß mindestens einem der Ansprüche 8 bis 10, **dadurch gekennzeichnet, dass** es zur Herstellung von verzweigten Alkoholen mit einer Kettenlänge von 4 bis 18, insbesondere 8 bis 12, bevorzugt 8, Kohlenstoffatomen, insbesondere 2-Ethylhexanol, ausgebaut ist, zusätzlich umfassend die Verfahrensschritte
C) Aldolkondensation des Aldehyds unter Wasserabspaltung
D) katalytischen Hydrierung.


SEQUENCE LISTING

<110>  Evonik Industries AG

<120>  Mikroorganismen mit reduzierter Enzymaktivität

<130>  201400362

<160>  32

<170>  PatentIn version 3.5

<210>  1
<211>  1437
<212>  DNA
<213>  Thauera butanivorans

<220>
<221>  CDS
<222>  (1)..(1437)

<400>  1
gtg acg aaa gag atc atc acc acc gcc gcg ggc ctg gcc ggc ctg ctc      48
Val Thr Lys Glu Ile Ile Thr Thr Ala Ala Gly Leu Ala Gly Leu Leu
1               5                   10                  15

cag caa cag cgc aag gcc ttc gct gcc gct ggc gcc gtg agc gcc gcc      96
Gln Gln Gln Arg Lys Ala Phe Ala Ala Ala Gly Ala Val Ser Ala Ala
            20                  25                  30

gag cgc agg cgc cgc atc cag acc gcg atc gac ctg ctg gtg aaa cac      144
Glu Arg Arg Arg Arg Ile Gln Thr Ala Ile Asp Leu Leu Val Lys His
        35                  40                  45

cac aag tcc ctg gcc gaa gca atg gac gcc gac ttc ggc ggc cgc ccc      192
His Lys Ser Leu Ala Glu Ala Met Asp Ala Asp Phe Gly Gly Arg Pro
    50                  55                  60

agg ggc tat tca ctg atg aac gac gtg ctc ggt tcg ctg acc tcg ctg      240
Arg Gly Tyr Ser Leu Met Asn Asp Val Leu Gly Ser Leu Thr Ser Leu
65                  70                  75                  80

aag tac gcc cgc gat cat ttc gaa ccg tgg atg gcg agc gag ccg cgc      288
Lys Tyr Ala Arg Asp His Phe Glu Pro Trp Met Ala Ser Glu Pro Arg
                85                  90                  95

acg ccc ttt ccg ccc tac gac cag ctc ggc gcc cag gcc tgg gtg atg      336
Thr Pro Phe Pro Pro Tyr Asp Gln Leu Gly Ala Gln Ala Trp Val Met
            100                 105                 110

tac cag ccc aag ggc acg gtg ggc atc atc ggc acc tgg aac gcg ccg      384
Tyr Gln Pro Lys Gly Thr Val Gly Ile Ile Gly Thr Trp Asn Ala Pro
        115                 120                 125

ctg ttc acg ctg ttc agc ccc ttg gcg agc gcg ctg gcc gcc ggc aac      432
Leu Phe Thr Leu Phe Ser Pro Leu Ala Ser Ala Leu Ala Ala Gly Asn
    130                 135                 140

cgg gcg atc ctg aaa cct tcc gaa gtc gtg ccg cgt act gcg cag ata      480
Arg Ala Ile Leu Lys Pro Ser Glu Val Val Pro Arg Thr Ala Gln Ile
145                 150                 155                 160

gtg gcc gag gcc gtg gcc gag ctg ttc gat ccg ctc gaa gtc ggc gtc 528
Val Ala Glu Ala Val Ala Glu Leu Phe Asp Pro Leu Glu Val Gly Val
                165                 170                 175

gtc acc ggc ggg ccg gaa atg ggc gaa gcc ttc acg tcc cag cct ttc 576
Val Thr Gly Gly Pro Glu Met Gly Glu Ala Phe Thr Ser Gln Pro Phe
            180                 185                 190

gac cac atc gta ttc acc ggc agc acc cag gtg ggc aag gcg gtg atg 624
Asp His Ile Val Phe Thr Gly Ser Thr Gln Val Gly Lys Ala Val Met
        195                 200                 205

cgc aac gcc gcg cag aat ctg gtg ccg gtg acg ctg gag ctc ggc ggc 672
Arg Asn Ala Ala Gln Asn Leu Val Pro Val Thr Leu Glu Leu Gly Gly
    210                 215                 220

aag tcg ccg gcc atc gtc gcc cgc agc gcc gat ctc gcc acc gcg gcc 720
Lys Ser Pro Ala Ile Val Ala Arg Ser Ala Asp Leu Ala Thr Ala Ala
225                 230                 235                 240

ttc cgc atc gcc gct gcc aag gcg gcc aac gcc ggc cag ctc tgc gtc 768
Phe Arg Ile Ala Ala Ala Lys Ala Ala Asn Ala Gly Gln Leu Cys Val
                245                 250                 255

aat ccg gac gtg gtc tac gtc gcc cgt gag cag ctc gag gac ttc gtc 816
Asn Pro Asp Val Val Tyr Val Ala Arg Glu Gln Leu Glu Asp Phe Val
            260                 265                 270

gcc gcg ctc aag cgc agc ttc ggc gaa ctg ttc ccg agc gtg gcg ggc 864
Ala Ala Leu Lys Arg Ser Phe Gly Glu Leu Phe Pro Ser Val Ala Gly
        275                 280                 285

aat gcg gac atg gtg gcg gtg gtg aat gcc cgc cat ctg gcg cgt gtc 912
Asn Ala Asp Met Val Ala Val Val Asn Ala Arg His Leu Ala Arg Val
    290                 295                 300

gaa gcc tgc gtc ggc gac gcc gcc cgc gtc ggg gct cgc gtc gaa tgt 960
Glu Ala Cys Val Gly Asp Ala Ala Arg Val Gly Ala Arg Val Glu Cys
305                 310                 315                 320

gcg ccg gtc cct gca gcc gct gat gcg acg gac cgc cgc cgg ccg ctc 1008
Ala Pro Val Pro Ala Ala Ala Asp Ala Thr Asp Arg Arg Arg Pro Leu
                325                 330                 335

agc atc gtc atc gac ccg ccg gcg gac agc gcg atc atg cgc gag gaa 1056
Ser Ile Val Ile Asp Pro Pro Ala Asp Ser Ala Ile Met Arg Glu Glu
            340                 345                 350

atc ttc ggc ccc gcc gtc gtc gtc aaa ccc tac gac gcc atc gac gac 1104
Ile Phe Gly Pro Ala Val Val Val Lys Pro Tyr Asp Ala Ile Asp Asp
        355                 360                 365

gtg atc gcc gac atc aat gcc cgg ccg cgg ccg ctg gcc ttg tac tac 1152
Val Ile Ala Asp Ile Asn Ala Arg Pro Arg Pro Leu Ala Leu Tyr Tyr
    370                 375                 380

ttc ggc gag gac gag gcc gag cag cag cag gtg ctg gag cac acg ttg 1200
Phe Gly Glu Asp Glu Ala Glu Gln Gln Gln Val Leu Glu His Thr Leu
385                 390                 395                 400

tcg ggt ggc gtg acg atc aac gaa gcg atg ttc cat gcg gcg atg gag 1248
Ser Gly Gly Val Thr Ile Asn Glu Ala Met Phe His Ala Ala Met Glu
                405                 410                 415

gac gcg cct ttc ggc ggc atc ggc gat tcc ggc atg ggc cat tac cac          1296
Asp Ala Pro Phe Gly Gly Ile Gly Asp Ser Gly Met Gly His Tyr His
            420                 425                 430

ggc cgc gaa ggc ttc ctc gaa ttc agc cac acg cgc acg gtg ttc aag          1344
Gly Arg Glu Gly Phe Leu Glu Phe Ser His Thr Arg Thr Val Phe Lys
        435                 440                 445

gcg ccg gcc tgc gat ccg cgc cgc gaa tgg ggc ctg ctg ccg ccg tat          1392
Ala Pro Ala Cys Asp Pro Arg Arg Glu Trp Gly Leu Leu Pro Pro Tyr
    450                 455                 460

tcc gat cag ttc ctg gcg atg atg gag gcg cag gtc acg ccc tga              1437
Ser Asp Gln Phe Leu Ala Met Met Glu Ala Gln Val Thr Pro
465                 470                 475


<210>  2
<211>  478
<212>  PRT
<213>  Thauera butanivorans

<400>  2

Val Thr Lys Glu Ile Ile Thr Thr Ala Ala Gly Leu Ala Gly Leu Leu
1               5                   10                  15


Gln Gln Gln Arg Lys Ala Phe Ala Ala Ala Gly Ala Val Ser Ala Ala
            20                  25                  30


Glu Arg Arg Arg Arg Ile Gln Thr Ala Ile Asp Leu Leu Val Lys His
        35                  40                  45


His Lys Ser Leu Ala Glu Ala Met Asp Ala Asp Phe Gly Gly Arg Pro
    50                  55                  60


Arg Gly Tyr Ser Leu Met Asn Asp Val Leu Gly Ser Leu Thr Ser Leu
65                  70                  75                  80


Lys Tyr Ala Arg Asp His Phe Glu Pro Trp Met Ala Ser Glu Pro Arg
                85                  90                  95


Thr Pro Phe Pro Pro Tyr Asp Gln Leu Gly Ala Gln Ala Trp Val Met
            100                 105                 110


Tyr Gln Pro Lys Gly Thr Val Gly Ile Ile Gly Thr Trp Asn Ala Pro
        115                 120                 125


Leu Phe Thr Leu Phe Ser Pro Leu Ala Ser Ala Leu Ala Ala Gly Asn
    130                 135                 140


Arg Ala Ile Leu Lys Pro Ser Glu Val Val Pro Arg Thr Ala Gln Ile
145                 150                 155                 160

Val Ala Glu Ala Val Ala Glu Leu Phe Asp Pro Leu Glu Val Gly Val
                165                 170                 175

Val Thr Gly Gly Pro Glu Met Gly Glu Ala Phe Thr Ser Gln Pro Phe
            180                 185                 190

Asp His Ile Val Phe Thr Gly Ser Thr Gln Val Gly Lys Ala Val Met
        195                 200                 205

Arg Asn Ala Ala Gln Asn Leu Val Pro Val Thr Leu Glu Leu Gly Gly
    210                 215                 220

Lys Ser Pro Ala Ile Val Ala Arg Ser Ala Asp Leu Ala Thr Ala Ala
225                 230                 235                 240

Phe Arg Ile Ala Ala Ala Lys Ala Ala Asn Ala Gly Gln Leu Cys Val
                245                 250                 255

Asn Pro Asp Val Val Tyr Val Ala Arg Glu Gln Leu Glu Asp Phe Val
            260                 265                 270

Ala Ala Leu Lys Arg Ser Phe Gly Glu Leu Phe Pro Ser Val Ala Gly
        275                 280                 285

Asn Ala Asp Met Val Ala Val Val Asn Ala Arg His Leu Ala Arg Val
    290                 295                 300

Glu Ala Cys Val Gly Asp Ala Ala Arg Val Gly Ala Arg Val Glu Cys
305                 310                 315                 320

Ala Pro Val Pro Ala Ala Ala Asp Ala Thr Asp Arg Arg Arg Pro Leu
                325                 330                 335

Ser Ile Val Ile Asp Pro Pro Ala Asp Ser Ala Ile Met Arg Glu Glu
            340                 345                 350

Ile Phe Gly Pro Ala Val Val Val Lys Pro Tyr Asp Ala Ile Asp Asp
        355                 360                 365

Val Ile Ala Asp Ile Asn Ala Arg Pro Arg Pro Leu Ala Leu Tyr Tyr
    370                 375                 380

Phe Gly Glu Asp Glu Ala Glu Gln Gln Gln Val Leu Glu His Thr Leu
385                 390                 395                 400

Ser Gly Gly Val Thr Ile Asn Glu Ala Met Phe His Ala Ala Met Glu

405                 410                 415

Asp Ala Pro Phe Gly Gly Ile Gly Asp Ser Gly Met Gly His Tyr His
            420                 425                 430

Gly Arg Glu Gly Phe Leu Glu Phe Ser His Thr Arg Thr Val Phe Lys
        435                 440                 445

Ala Pro Ala Cys Asp Pro Arg Arg Glu Trp Gly Leu Leu Pro Pro Tyr
    450                 455                 460

Ser Asp Gln Phe Leu Ala Met Met Glu Ala Gln Val Thr Pro
465                 470                 475

<210>  3
<211>  1455
<212>  DNA
<213>  Thauera butanivorans

<220>
<221>  CDS
<222>  (1)..(1455)

<400>  3
atg aaa cag atc cac aat ttc atc aat ggc gaa ttc gtc gcc acc gcc      48
Met Lys Gln Ile His Asn Phe Ile Asn Gly Glu Phe Val Ala Thr Ala
1               5                   10                  15

aag tcc ttc gac aag ttc tcg ccg ctg gac aac gcc ctg atc gcc aag      96
Lys Ser Phe Asp Lys Phe Ser Pro Leu Asp Asn Ala Leu Ile Ala Lys
            20                  25                  30

gtg cat gag gcg ggc aag gcc gaa gtc gat gcc gcc gtc gcc gcc gcg      144
Val His Glu Ala Gly Lys Ala Glu Val Asp Ala Ala Val Ala Ala Ala
        35                  40                  45

cgt gcc gcc ttg tcg ggg ccg tgg ggc cgc atg acc gtg gcc gac cgc      192
Arg Ala Ala Leu Ser Gly Pro Trp Gly Arg Met Thr Val Ala Asp Arg
    50                  55                  60

gtc gat ctg ctc aac gcg gtg gcc gac ggc atc aac aag cgt ttc gat      240
Val Asp Leu Leu Asn Ala Val Ala Asp Gly Ile Asn Lys Arg Phe Asp
65                  70                  75                  80

gat ttc ctc gcc gcc gaa tgc gcc gat acc ggc aag ccg cgc agc ctc      288
Asp Phe Leu Ala Ala Glu Cys Ala Asp Thr Gly Lys Pro Arg Ser Leu
                85                  90                  95

gcc agt cat atc gac att ccg cgc ggc gcg gcc aac ttc aag gtg ttc      336
Ala Ser His Ile Asp Ile Pro Arg Gly Ala Ala Asn Phe Lys Val Phe
            100                 105                 110

gcg gac gtc atc aag agc gtg ccg acc gag ttc ttc gag atg gcg acg      384
Ala Asp Val Ile Lys Ser Val Pro Thr Glu Phe Phe Glu Met Ala Thr
        115                 120                 125

ccg gac ggc cgc ggt gcg ctg aac tat gcc gtg cgc cgc ccg gtg ggc      432

Pro Asp Gly Arg Gly Ala Leu Asn Tyr Ala Val Arg Arg Pro Val Gly
    130                 135                 140

gtg gtc ggt gtc atc tgt ccg tgg aac ctg ccc ctg ctg ttg atg acc     480
Val Val Gly Val Ile Cys Pro Trp Asn Leu Pro Leu Leu Leu Met Thr
145                 150                 155                 160

tgg aag gtc ggg cct gcg ctg gcc tgc ggc aac acc gtg gtg gtg aag     528
Trp Lys Val Gly Pro Ala Leu Ala Cys Gly Asn Thr Val Val Val Lys
                165                 170                 175

cct tcg gag gaa acc ccg cag acg gcg gcc ctg ctc ggc gag gtg atg     576
Pro Ser Glu Glu Thr Pro Gln Thr Ala Ala Leu Leu Gly Glu Val Met
            180                 185                 190

aac gag gtc ggc atg ccg aag ggc gtc tac aac gtc gtg cac ggc ttt     624
Asn Glu Val Gly Met Pro Lys Gly Val Tyr Asn Val Val His Gly Phe
        195                 200                 205

ggc ccg ggc tcg gcg ggc gaa ttc gtc acc gcg cat ccg aac gtc gat     672
Gly Pro Gly Ser Ala Gly Glu Phe Val Thr Ala His Pro Asn Val Asp
    210                 215                 220

gcc atc acc ttc acc ggc gag acc cgc acc ggc gcg gcg atc atg aag     720
Ala Ile Thr Phe Thr Gly Glu Thr Arg Thr Gly Ala Ala Ile Met Lys
225                 230                 235                 240

gcg gca gcg gaa ggt gcg cgc ccg gtg tcg ctc gag atg ggc ggc aag     768
Ala Ala Ala Glu Gly Ala Arg Pro Val Ser Leu Glu Met Gly Gly Lys
                245                 250                 255

aat ccg gcg atc gtg ttc gcc gat tgc gat ttc gac gcc gcc atc gaa     816
Asn Pro Ala Ile Val Phe Ala Asp Cys Asp Phe Asp Ala Ala Ile Glu
            260                 265                 270

ggc acg atg cgc tcg gtc ttc gcc aac tgc ggc cag gtc tgc ctg ggc     864
Gly Thr Met Arg Ser Val Phe Ala Asn Cys Gly Gln Val Cys Leu Gly
        275                 280                 285

acc gag cgt gtc tat gtc gaa cgg ccg atc ttc gag cgt ttc gtc gag     912
Thr Glu Arg Val Tyr Val Glu Arg Pro Ile Phe Glu Arg Phe Val Glu
    290                 295                 300

gcg ctg aag aag ggc gcg gaa ggg ctc agg ctc ggc cgc ccc gaa gat     960
Ala Leu Lys Lys Gly Ala Glu Gly Leu Arg Leu Gly Arg Pro Glu Asp
305                 310                 315                 320

gcc gcc acc ggc ctc ggc ccg ctg atc tcg cag gag cac aag cag aag     1008
Ala Ala Thr Gly Leu Gly Pro Leu Ile Ser Gln Glu His Lys Gln Lys
                325                 330                 335

gtg ctg tcc tac tac gag aag gcc agg gcg ctg ggc gcc acc gtc gtc     1056
Val Leu Ser Tyr Tyr Glu Lys Ala Arg Ala Leu Gly Ala Thr Val Val
            340                 345                 350

acc ggc ggc ggc gtg ccc gtg atg ccg ggg gaa ctg gcg aac ggc gcc     1104
Thr Gly Gly Gly Val Pro Val Met Pro Gly Glu Leu Ala Asn Gly Ala
        355                 360                 365

tgg gtg cag ccg acg atc tgg acc ggg ctg gac gac agc tcg ccg atc     1152
Trp Val Gln Pro Thr Ile Trp Thr Gly Leu Asp Asp Ser Ser Pro Ile
    370                 375                 380

gcg cgc gag gaa atc ttc ggt ccc tgc acg ctg atc cag ccg ttc gac          1200
Ala Arg Glu Glu Ile Phe Gly Pro Cys Thr Leu Ile Gln Pro Phe Asp
385                 390                 395                 400

gac gag gac gaa gtg gtc cgc cgt gcc aac gat acc gac tac ggc ctg          1248
Asp Glu Asp Glu Val Val Arg Arg Ala Asn Asp Thr Asp Tyr Gly Leu
                405                 410                 415

gcc tgc tcg gtg tgg acc cgc gat ctg gta cgc gcc cac cgc gtc gcg          1296
Ala Cys Ser Val Trp Thr Arg Asp Leu Val Arg Ala His Arg Val Ala
            420                 425                 430

ggc cgc atc gag gcc ggc ctg gtg tgg gtg aat tcc tgg ttc ctg cgc          1344
Gly Arg Ile Glu Ala Gly Leu Val Trp Val Asn Ser Trp Phe Leu Arg
        435                 440                 445

gac ctg cgc acg cct ttc ggc ggc tcg aag cag tcc ggc atc ggc cgc          1392
Asp Leu Arg Thr Pro Phe Gly Gly Ser Lys Gln Ser Gly Ile Gly Arg
    450                 455                 460

gaa ggc ggc gtg cat tcg ctc gag ttc tat acc gag ctg aag aac atc          1440
Glu Gly Gly Val His Ser Leu Glu Phe Tyr Thr Glu Leu Lys Asn Ile
465                 470                 475                 480

tgc atc aag ctg tga                                                      1455
Cys Ile Lys Leu

<210>  4
<211>  484
<212>  PRT
<213>  Thauera butanivorans

<400>  4

Met Lys Gln Ile His Asn Phe Ile Asn Gly Glu Phe Val Ala Thr Ala
1               5                   10                  15

Lys Ser Phe Asp Lys Phe Ser Pro Leu Asp Asn Ala Leu Ile Ala Lys
            20                  25                  30

Val His Glu Ala Gly Lys Ala Glu Val Asp Ala Ala Val Ala Ala Ala
        35                  40                  45

Arg Ala Ala Leu Ser Gly Pro Trp Gly Arg Met Thr Val Ala Asp Arg
    50                  55                  60

Val Asp Leu Leu Asn Ala Val Ala Asp Gly Ile Asn Lys Arg Phe Asp
65                  70                  75                  80

Asp Phe Leu Ala Ala Glu Cys Ala Asp Thr Gly Lys Pro Arg Ser Leu
                85                  90                  95

Ala Ser His Ile Asp Ile Pro Arg Gly Ala Ala Asn Phe Lys Val Phe
            100                 105                 110

Ala Asp Val Ile Lys Ser Val Pro Thr Glu Phe Phe Glu Met Ala Thr
        115                 120                 125

Pro Asp Gly Arg Gly Ala Leu Asn Tyr Ala Val Arg Arg Pro Val Gly
    130                 135                 140

Val Val Gly Val Ile Cys Pro Trp Asn Leu Pro Leu Leu Leu Met Thr
145                 150                 155                 160

Trp Lys Val Gly Pro Ala Leu Ala Cys Gly Asn Thr Val Val Val Lys
                165                 170                 175

Pro Ser Glu Glu Thr Pro Gln Thr Ala Ala Leu Leu Gly Glu Val Met
            180                 185                 190

Asn Glu Val Gly Met Pro Lys Gly Val Tyr Asn Val Val His Gly Phe
        195                 200                 205

Gly Pro Gly Ser Ala Gly Glu Phe Val Thr Ala His Pro Asn Val Asp
    210                 215                 220

Ala Ile Thr Phe Thr Gly Glu Thr Arg Thr Gly Ala Ala Ile Met Lys
225                 230                 235                 240

Ala Ala Ala Glu Gly Ala Arg Pro Val Ser Leu Glu Met Gly Gly Lys
                245                 250                 255

Asn Pro Ala Ile Val Phe Ala Asp Cys Asp Phe Asp Ala Ala Ile Glu
            260                 265                 270

Gly Thr Met Arg Ser Val Phe Ala Asn Cys Gly Gln Val Cys Leu Gly
        275                 280                 285

Thr Glu Arg Val Tyr Val Glu Arg Pro Ile Phe Glu Arg Phe Val Glu
    290                 295                 300

Ala Leu Lys Lys Gly Ala Glu Gly Leu Arg Leu Gly Arg Pro Glu Asp
305                 310                 315                 320

Ala Ala Thr Gly Leu Gly Pro Leu Ile Ser Gln Glu His Lys Gln Lys
                325                 330                 335

Val Leu Ser Tyr Tyr Glu Lys Ala Arg Ala Leu Gly Ala Thr Val Val
            340                 345                 350

Thr Gly Gly Gly Val Pro Val Met Pro Gly Glu Leu Ala Asn Gly Ala
        355                 360                 365

Trp Val Gln Pro Thr Ile Trp Thr Gly Leu Asp Asp Ser Ser Pro Ile
    370                 375                 380

Ala Arg Glu Glu Ile Phe Gly Pro Cys Thr Leu Ile Gln Pro Phe Asp
385                 390                 395                 400

Asp Glu Asp Glu Val Val Arg Arg Ala Asn Asp Thr Asp Tyr Gly Leu
                405                 410                 415

Ala Cys Ser Val Trp Thr Arg Asp Leu Val Arg Ala His Arg Val Ala
            420                 425                 430

Gly Arg Ile Glu Ala Gly Leu Val Trp Val Asn Ser Trp Phe Leu Arg
        435                 440                 445

Asp Leu Arg Thr Pro Phe Gly Gly Ser Lys Gln Ser Gly Ile Gly Arg
    450                 455                 460

Glu Gly Gly Val His Ser Leu Glu Phe Tyr Thr Glu Leu Lys Asn Ile
465                 470                 475                 480

Cys Ile Lys Leu

<210>  5
<211>  1461
<212>  DNA
<213>  Thauera butanivorans

<220>
<221>  CDS
<222>  (1)..(1461)

<400>  5
atg ctt gca gac aag atc ctc aac ttc atc ggc ggc gag tac gtc gcc      48
Met Leu Ala Asp Lys Ile Leu Asn Phe Ile Gly Gly Glu Tyr Val Ala
1               5                   10                  15

acc gac aaa tgg ttc gac aac cgg acg ccg atc tcc aat gcc gtc atc      96
Thr Asp Lys Trp Phe Asp Asn Arg Thr Pro Ile Ser Asn Ala Val Ile
            20                  25                  30

ggc cag gtc gcc gaa gcc ggc aag gcc gag gtc gat gcc gcg gtg gcc      144
Gly Gln Val Ala Glu Ala Gly Lys Ala Glu Val Asp Ala Ala Val Ala
        35                  40                  45

gcc gcg cgc gcc gcg ctg aaa ggc ccc tgg gga acg atg ccg ctg gcg      192
Ala Ala Arg Ala Ala Leu Lys Gly Pro Trp Gly Thr Met Pro Leu Ala
    50                  55                  60

cgc cgc gtc gag ctg ctc gaa gcc ctg gtc gcc gaa atc aat gcc cgc      240
Arg Arg Val Glu Leu Leu Glu Ala Leu Val Ala Glu Ile Asn Ala Arg

65                  70                  75                  80

ttc gac gag ttc ctc gag gcc gaa tgc gcc gac acc ggc aag ccg cgg      288
Phe Asp Glu Phe Leu Glu Ala Glu Cys Ala Asp Thr Gly Lys Pro Arg
                85                  90                  95

agc ctc gca tcg cat atc gac att ccg cgc ggc gcg gcg aac ttc agg      336
Ser Leu Ala Ser His Ile Asp Ile Pro Arg Gly Ala Ala Asn Phe Arg
            100                 105                 110

gtc ttc gcc gac atg atc aag aac gcc tcc acc gaa ttc ttc gag atg      384
Val Phe Ala Asp Met Ile Lys Asn Ala Ser Thr Glu Phe Phe Glu Met
        115                 120                 125

gcc acg ccc gac ggc agt tcg tgc atc aac tac ggc tat cgc cgt ccg      432
Ala Thr Pro Asp Gly Ser Ser Cys Ile Asn Tyr Gly Tyr Arg Arg Pro
    130                 135                 140

gtc ggc gtg atc ggt gtg atc tgc ccg tgg aac ctg ccg ctg ctg ctg      480
Val Gly Val Ile Gly Val Ile Cys Pro Trp Asn Leu Pro Leu Leu Leu
145                 150                 155                 160

atg acc tgg aaa gtc ggc ccg gcg ctg gcc tgc ggc aac acc gta gtg      528
Met Thr Trp Lys Val Gly Pro Ala Leu Ala Cys Gly Asn Thr Val Val
                165                 170                 175

gtc aaa ccc tcg gaa gag acg ccg cgc acc gcg gcg ctg ctg ggc gaa      576
Val Lys Pro Ser Glu Glu Thr Pro Arg Thr Ala Ala Leu Leu Gly Glu
            180                 185                 190

gtg atg aac aag gtc ggc atc ccc aag ggg gtg tac aac gtc gtc cac      624
Val Met Asn Lys Val Gly Ile Pro Lys Gly Val Tyr Asn Val Val His
        195                 200                 205

ggc ttc ggc ccg aac tcg gcg ggc gaa ttc gtc act tcg cac ccc gac      672
Gly Phe Gly Pro Asn Ser Ala Gly Glu Phe Val Thr Ser His Pro Asp
    210                 215                 220

gtc gat gcc atc acc ttt acc ggc gag acc cgc acc ggc gag atc atc      720
Val Asp Ala Ile Thr Phe Thr Gly Glu Thr Arg Thr Gly Glu Ile Ile
225                 230                 235                 240

aac cgt gcc gcc gcc atc ggc tcg cgc ccg gtt tcg ctg gaa atg ggc      768
Asn Arg Ala Ala Ala Ile Gly Ser Arg Pro Val Ser Leu Glu Met Gly
                245                 250                 255

ggc aag aac gcg gcg atc gtg ttt gcc gac tgc gat ttc gat gcc gcg      816
Gly Lys Asn Ala Ala Ile Val Phe Ala Asp Cys Asp Phe Asp Ala Ala
            260                 265                 270

atc gaa ggc acg ctg cgc tcg gcc ttc gcc aac tgc ggc cag gtc tgc      864
Ile Glu Gly Thr Leu Arg Ser Ala Phe Ala Asn Cys Gly Gln Val Cys
        275                 280                 285

ctg ggc acc gaa cgg gtg tat gtc gag cgc ccg atc ttc gac agc ttc      912
Leu Gly Thr Glu Arg Val Tyr Val Glu Arg Pro Ile Phe Asp Ser Phe
    290                 295                 300

gtc gcc gcg atg aag gcg ggt gcc gag gcg atg aag atc ggt gtg ccg      960
Val Ala Ala Met Lys Ala Gly Ala Glu Ala Met Lys Ile Gly Val Pro
305                 310                 315                 320

gac gat gcg gcg acc gac atg ggg ccg ctg atc agc aag gag cac cag     1008

Asp Asp Ala Ala Thr Asp Met Gly Pro Leu Ile Ser Lys Glu His Gln
                325                 330                 335

gaa aag gtg ctg tcc tac tgc cgc ctg gcg gtg gaa gag ggc gcc acg      1056
Glu Lys Val Leu Ser Tyr Cys Arg Leu Ala Val Glu Glu Gly Ala Thr
            340                 345                 350

gtg gtg acc ggc ggc ggc gtg ccc gag atg gcc ggc gag ctg aag gac      1104
Val Val Thr Gly Gly Gly Val Pro Glu Met Ala Gly Glu Leu Lys Asp
        355                 360                 365

ggc tgc tgg gtg cag ccg acg atc tgg acc ggc ctg ccc gag acc gcg      1152
Gly Cys Trp Val Gln Pro Thr Ile Trp Thr Gly Leu Pro Glu Thr Ala
    370                 375                 380

cgc gtg atc aag gaa gag atc ttc ggg ccc tgc tgc cac atc gcg ccg      1200
Arg Val Ile Lys Glu Glu Ile Phe Gly Pro Cys Cys His Ile Ala Pro
385                 390                 395                 400

ttc gac acc gaa gcg gaa gtg ctg gag aag gcc aac gac aac aag tac      1248
Phe Asp Thr Glu Ala Glu Val Leu Glu Lys Ala Asn Asp Asn Lys Tyr
                405                 410                 415

ggc ctg gcc acc gcg atc tgg acc cgc gac gta gcg cgt gcc aac cgc      1296
Gly Leu Ala Thr Ala Ile Trp Thr Arg Asp Val Ala Arg Ala Asn Arg
            420                 425                 430

gtc gcg cag aag atg gaa gtc ggc atc tcc tgg gtt aac agc tgg ttc      1344
Val Ala Gln Lys Met Glu Val Gly Ile Ser Trp Val Asn Ser Trp Phe
        435                 440                 445

ctg cgc gat ctg cgc acc ccg ttc ggc ggt tcc aag cag tcg ggc atc      1392
Leu Arg Asp Leu Arg Thr Pro Phe Gly Gly Ser Lys Gln Ser Gly Ile
    450                 455                 460

ggc cgt gag ggc ggg ctg cac tcg ctc gag ttc tac acc gat ctc aag      1440
Gly Arg Glu Gly Gly Leu His Ser Leu Glu Phe Tyr Thr Asp Leu Lys
465                 470                 475                 480

aac gtc tgc atc aag ctg tga                                          1461
Asn Val Cys Ile Lys Leu
                485

<210>  6
<211>  486
<212>  PRT
<213>  Thauera butanivorans

<400>  6

Met Leu Ala Asp Lys Ile Leu Asn Phe Ile Gly Gly Glu Tyr Val Ala
1               5                   10                  15

Thr Asp Lys Trp Phe Asp Asn Arg Thr Pro Ile Ser Asn Ala Val Ile
            20                  25                  30

Gly Gln Val Ala Glu Ala Gly Lys Ala Glu Val Asp Ala Ala Val Ala
        35                  40                  45

Ala Ala Arg Ala Ala Leu Lys Gly Pro Trp Gly Thr Met Pro Leu Ala
    50                  55                  60

Arg Arg Val Glu Leu Leu Glu Ala Leu Val Ala Glu Ile Asn Ala Arg
65                  70                  75                  80

Phe Asp Glu Phe Leu Glu Ala Glu Cys Ala Asp Thr Gly Lys Pro Arg
                85                  90                  95

Ser Leu Ala Ser His Ile Asp Ile Pro Arg Gly Ala Ala Asn Phe Arg
            100                 105                 110

Val Phe Ala Asp Met Ile Lys Asn Ala Ser Thr Glu Phe Phe Glu Met
        115                 120                 125

Ala Thr Pro Asp Gly Ser Ser Cys Ile Asn Tyr Gly Tyr Arg Arg Pro
    130                 135                 140

Val Gly Val Ile Gly Val Ile Cys Pro Trp Asn Leu Pro Leu Leu Leu
145                 150                 155                 160

Met Thr Trp Lys Val Gly Pro Ala Leu Ala Cys Gly Asn Thr Val Val
                165                 170                 175

Val Lys Pro Ser Glu Glu Thr Pro Arg Thr Ala Ala Leu Leu Gly Glu
            180                 185                 190

Val Met Asn Lys Val Gly Ile Pro Lys Gly Val Tyr Asn Val Val His
        195                 200                 205

Gly Phe Gly Pro Asn Ser Ala Gly Glu Phe Val Thr Ser His Pro Asp
    210                 215                 220

Val Asp Ala Ile Thr Phe Thr Gly Glu Thr Arg Thr Gly Glu Ile Ile
225                 230                 235                 240

Asn Arg Ala Ala Ala Ile Gly Ser Arg Pro Val Ser Leu Glu Met Gly
                245                 250                 255

Gly Lys Asn Ala Ala Ile Val Phe Ala Asp Cys Asp Phe Asp Ala Ala
            260                 265                 270

Ile Glu Gly Thr Leu Arg Ser Ala Phe Ala Asn Cys Gly Gln Val Cys
        275                 280                 285

Leu Gly Thr Glu Arg Val Tyr Val Glu Arg Pro Ile Phe Asp Ser Phe
    290                 295                 300

Val Ala Ala Met Lys Ala Gly Ala Glu Ala Met Lys Ile Gly Val Pro
305                 310                 315                 320

Asp Asp Ala Ala Thr Asp Met Gly Pro Leu Ile Ser Lys Glu His Gln
                325                 330                 335

Glu Lys Val Leu Ser Tyr Cys Arg Leu Ala Val Glu Glu Gly Ala Thr
            340                 345                 350

Val Val Thr Gly Gly Gly Val Pro Glu Met Ala Gly Glu Leu Lys Asp
        355                 360                 365

Gly Cys Trp Val Gln Pro Thr Ile Trp Thr Gly Leu Pro Glu Thr Ala
    370                 375                 380

Arg Val Ile Lys Glu Glu Ile Phe Gly Pro Cys Cys His Ile Ala Pro
385                 390                 395                 400

Phe Asp Thr Glu Ala Glu Val Leu Glu Lys Ala Asn Asp Asn Lys Tyr
                405                 410                 415

Gly Leu Ala Thr Ala Ile Trp Thr Arg Asp Val Ala Arg Ala Asn Arg
            420                 425                 430

Val Ala Gln Lys Met Glu Val Gly Ile Ser Trp Val Asn Ser Trp Phe
        435                 440                 445

Leu Arg Asp Leu Arg Thr Pro Phe Gly Gly Ser Lys Gln Ser Gly Ile
    450                 455                 460

Gly Arg Glu Gly Gly Leu His Ser Leu Glu Phe Tyr Thr Asp Leu Lys
465                 470                 475                 480

Asn Val Cys Ile Lys Leu
                485

<210>  7
<211>  1512
<212>  DNA
<213>  Thauera butanivorans

<220>
<221>  CDS
<222>  (1)..(1512)

<400>  7
atg cag gac tat gca agc aca ttg aag gcc ccg atc acc gac gcc acc      48
Met Gln Asp Tyr Ala Ser Thr Leu Lys Ala Pro Ile Thr Asp Ala Thr
1               5                   10                  15

cgc cgt ttc ctt gcc cag ccc aag cgc ctg ctg atc ggc ggg gcc tgg     96
Arg Arg Phe Leu Ala Gln Pro Lys Arg Leu Leu Ile Gly Gly Ala Trp
        20                  25                  30

gtg gag gcc agc gac ggc gcc acg ctg gac gtg ttc gac ccg gcc acc     144
Val Glu Ala Ser Asp Gly Ala Thr Leu Asp Val Phe Asp Pro Ala Thr
    35                  40                  45

ggc cag gcc ttc gcc aag gtg ccg gcc ggc ggc cag gcc gac atc gac     192
Gly Gln Ala Phe Ala Lys Val Pro Ala Gly Gly Gln Ala Asp Ile Asp
    50                  55                  60

cgt gcg gtg gcc gcc gcg cgc cag gcc ttc gaa ggc ggc gac tgg gcg     240
Arg Ala Val Ala Ala Ala Arg Gln Ala Phe Glu Gly Gly Asp Trp Ala
65                  70                  75                  80

gcg atg cgc ccg gtc gat cgc gag cgc ctg ctg atg aag ctg gcc gac     288
Ala Met Arg Pro Val Asp Arg Glu Arg Leu Leu Met Lys Leu Ala Asp
                85                  90                  95

ctg gtc gaa gcc aac gcc cag gaa ctg gcc gaa ctg gaa gcg ctg gac     336
Leu Val Glu Ala Asn Ala Gln Glu Leu Ala Glu Leu Glu Ala Leu Asp
            100                 105                 110

aac ggc aag ccg gtg acg gtg gcg cgc ttc gcc gac gtc gcg ctg gtg     384
Asn Gly Lys Pro Val Thr Val Ala Arg Phe Ala Asp Val Ala Leu Val
        115                 120                 125

gtc gac tcg ctg cgc tac atg gcc ggc tgg gcg acc aag ctc ggc ggc     432
Val Asp Ser Leu Arg Tyr Met Ala Gly Trp Ala Thr Lys Leu Gly Gly
    130                 135                 140

gag acc atg cag ccc tcg gtg ccg atc atc cgc gag cgc gaa ttc tgg     480
Glu Thr Met Gln Pro Ser Val Pro Ile Ile Arg Glu Arg Glu Phe Trp
145                 150                 155                 160

ggc ttc acc cgg cgc gag ccg gtg ggc gtg gtc ggc gcc atc atc ccg     528
Gly Phe Thr Arg Arg Glu Pro Val Gly Val Val Gly Ala Ile Ile Pro
                165                 170                 175

tgg aac ttc ccg ctg ctg atg gcg gcg tgg aag atg ggg ccg gcg ctg     576
Trp Asn Phe Pro Leu Leu Met Ala Ala Trp Lys Met Gly Pro Ala Leu
            180                 185                 190

gcc agc ggc tgc acg atg gtg ctc aag ccg gcc gag gaa acg ccg ctg     624
Ala Ser Gly Cys Thr Met Val Leu Lys Pro Ala Glu Glu Thr Pro Leu
        195                 200                 205

agc gcg ctg cgc ctg gcc gaa ctg gtg cag gaa gcc ggc tac ccg gcc     672
Ser Ala Leu Arg Leu Ala Glu Leu Val Gln Glu Ala Gly Tyr Pro Ala
    210                 215                 220

ggc gtg ttc aac gtc gtc acc ggc ctg ggc cat gtc gcc ggc gcg gcg     720
Gly Val Phe Asn Val Val Thr Gly Leu Gly His Val Ala Gly Ala Ala
225                 230                 235                 240

ctg tcc tcg cac ccg ggc atc gac aag ctc gcc ttc acc ggc tcg acc     768
Leu Ser Ser His Pro Gly Ile Asp Lys Leu Ala Phe Thr Gly Ser Thr
                245                 250                 255

gag atc ggc aag ctg gtg ggc aag gcc gcg ctc gac aac atg acg cgc     816
Glu Ile Gly Lys Leu Val Gly Lys Ala Ala Leu Asp Asn Met Thr Arg

260                 265                 270

atc tcg ctg gag ctg ggc ggc aag agc ccg atg atc gtg ctc gac gac     864
Ile Ser Leu Glu Leu Gly Gly Lys Ser Pro Met Ile Val Leu Asp Asp
        275                 280                 285

gcc gac gtc gac gcg gtg gcc gcc ggt gcc gca cac ggc atc ttc ttc     912
Ala Asp Val Asp Ala Val Ala Ala Gly Ala Ala His Gly Ile Phe Phe
    290                 295                 300

aac tcc ggc cag gtc tgc acc gcc ggc tcg cgc ctg ttc gtg cac aag     960
Asn Ser Gly Gln Val Cys Thr Ala Gly Ser Arg Leu Phe Val His Lys
305                 310                 315                 320

aag aag ttc gac cag gtg gtc gaa ggc ctg tcc aac att gcc gcc ggc     1008
Lys Lys Phe Asp Gln Val Val Glu Gly Leu Ser Asn Ile Ala Ala Gly
                325                 330                 335

atg aag gtc ggc gcc ggc ctc gac ccc gac acc cag ctc ggc ccg ctg     1056
Met Lys Val Gly Ala Gly Leu Asp Pro Asp Thr Gln Leu Gly Pro Leu
            340                 345                 350

gtc tcg gcg gtg cag cag gaa cgg gtg atg ggc tac atc cgc agc ggc     1104
Val Ser Ala Val Gln Gln Glu Arg Val Met Gly Tyr Ile Arg Ser Gly
        355                 360                 365

ttc gaa gaa ggc gcg cgt gcc gcg gtc ggc ggc gaa gcg ggc gaa ggc     1152
Phe Glu Glu Gly Ala Arg Ala Ala Val Gly Gly Glu Ala Gly Glu Gly
    370                 375                 380

ggc ggc tac ttc gtc aag ccg acg gtg ctg gtc gat gcg cgc gac gac     1200
Gly Gly Tyr Phe Val Lys Pro Thr Val Leu Val Asp Ala Arg Asp Asp
385                 390                 395                 400

atg cgg gtg gtg cgc gag gaa atc ttc ggc ccg gtg gtg gtg gcg atg     1248
Met Arg Val Val Arg Glu Glu Ile Phe Gly Pro Val Val Val Ala Met
                405                 410                 415

ccg tac gac gat ctg gac gag gtc gcg cgc cgc gcc aac gac acg ccc     1296
Pro Tyr Asp Asp Leu Asp Glu Val Ala Arg Arg Ala Asn Asp Thr Pro
            420                 425                 430

tac ggc ctg gcg gcg agc atc tgg tcg aac gac ctg acg cgg gtg cat     1344
Tyr Gly Leu Ala Ala Ser Ile Trp Ser Asn Asp Leu Thr Arg Val His
        435                 440                 445

cgg ctg att ccg cgg atc aag gcc ggc acg gtg tgg gtg aac tgc cac     1392
Arg Leu Ile Pro Arg Ile Lys Ala Gly Thr Val Trp Val Asn Cys His
    450                 455                 460

acg gtg ctg gac aac tcg atg ccg ttc ggc ggc ttc aag cag tcg ggc     1440
Thr Val Leu Asp Asn Ser Met Pro Phe Gly Gly Phe Lys Gln Ser Gly
465                 470                 475                 480

atc ggc cgc gag atg ggg cgc gcg gta ttc gac cag tac acc gag atc     1488
Ile Gly Arg Glu Met Gly Arg Ala Val Phe Asp Gln Tyr Thr Glu Ile
                485                 490                 495

aag tcg gtg ctg atg gcg ctg tga                                     1512
Lys Ser Val Leu Met Ala Leu
            500

<210>  8
<211>  503
<212>  PRT
<213>  Thauera butanivorans

<400>  8

Met Gln Asp Tyr Ala Ser Thr Leu Lys Ala Pro Ile Thr Asp Ala Thr
1               5                   10                  15

Arg Arg Phe Leu Ala Gln Pro Lys Arg Leu Leu Ile Gly Gly Ala Trp
            20                  25                  30

Val Glu Ala Ser Asp Gly Ala Thr Leu Asp Val Phe Asp Pro Ala Thr
        35                  40                  45

Gly Gln Ala Phe Ala Lys Val Pro Ala Gly Gly Gln Ala Asp Ile Asp
    50                  55                  60

Arg Ala Val Ala Ala Ala Arg Gln Ala Phe Glu Gly Gly Asp Trp Ala
65                  70                  75                  80

Ala Met Arg Pro Val Asp Arg Glu Arg Leu Leu Met Lys Leu Ala Asp
                85                  90                  95

Leu Val Glu Ala Asn Ala Gln Glu Leu Ala Glu Leu Glu Ala Leu Asp
            100                 105                 110

Asn Gly Lys Pro Val Thr Val Ala Arg Phe Ala Asp Val Ala Leu Val
        115                 120                 125

Val Asp Ser Leu Arg Tyr Met Ala Gly Trp Ala Thr Lys Leu Gly Gly
    130                 135                 140

Glu Thr Met Gln Pro Ser Val Pro Ile Ile Arg Glu Arg Glu Phe Trp
145                 150                 155                 160

Gly Phe Thr Arg Arg Glu Pro Val Gly Val Val Gly Ala Ile Ile Pro
                165                 170                 175

Trp Asn Phe Pro Leu Leu Met Ala Ala Trp Lys Met Gly Pro Ala Leu
            180                 185                 190

Ala Ser Gly Cys Thr Met Val Leu Lys Pro Ala Glu Glu Thr Pro Leu
        195                 200                 205

Ser Ala Leu Arg Leu Ala Glu Leu Val Gln Glu Ala Gly Tyr Pro Ala
    210                 215                 220

Gly Val Phe Asn Val Val Thr Gly Leu Gly His Val Ala Gly Ala Ala
225                 230                 235                 240

Leu Ser Ser His Pro Gly Ile Asp Lys Leu Ala Phe Thr Gly Ser Thr
                245                 250                 255

Glu Ile Gly Lys Leu Val Gly Lys Ala Ala Leu Asp Asn Met Thr Arg
            260                 265                 270

Ile Ser Leu Glu Leu Gly Gly Lys Ser Pro Met Ile Val Leu Asp Asp
        275                 280                 285

Ala Asp Val Asp Ala Val Ala Ala Gly Ala Ala His Gly Ile Phe Phe
    290                 295                 300

Asn Ser Gly Gln Val Cys Thr Ala Gly Ser Arg Leu Phe Val His Lys
305                 310                 315                 320

Lys Lys Phe Asp Gln Val Val Glu Gly Leu Ser Asn Ile Ala Ala Gly
                325                 330                 335

Met Lys Val Gly Ala Gly Leu Asp Pro Asp Thr Gln Leu Gly Pro Leu
            340                 345                 350

Val Ser Ala Val Gln Gln Glu Arg Val Met Gly Tyr Ile Arg Ser Gly
        355                 360                 365

Phe Glu Glu Gly Ala Arg Ala Ala Val Gly Gly Glu Ala Gly Glu Gly
    370                 375                 380

Gly Gly Tyr Phe Val Lys Pro Thr Val Leu Val Asp Ala Arg Asp Asp
385                 390                 395                 400

Met Arg Val Val Arg Glu Glu Ile Phe Gly Pro Val Val Val Ala Met
                405                 410                 415

Pro Tyr Asp Asp Leu Asp Glu Val Ala Arg Arg Ala Asn Asp Thr Pro
            420                 425                 430

Tyr Gly Leu Ala Ala Ser Ile Trp Ser Asn Asp Leu Thr Arg Val His
        435                 440                 445

Arg Leu Ile Pro Arg Ile Lys Ala Gly Thr Val Trp Val Asn Cys His
    450                 455                 460

Thr Val Leu Asp Asn Ser Met Pro Phe Gly Gly Phe Lys Gln Ser Gly
465                 470                 475                 480

Ile Gly Arg Glu Met Gly Arg Ala Val Phe Asp Gln Tyr Thr Glu Ile
                485                 490                 495

Lys Ser Val Leu Met Ala Leu
            500

<210>  9
<211>  1500
<212>  DNA
<213>  Thauera butanivorans

<220>
<221>  CDS
<222>  (1)..(1500)

<400>  9
atg gcc gag ctg ctg act gcc gcc gaa tac gcc gcg atc gcg aag aac        48
Met Ala Glu Leu Leu Thr Ala Ala Glu Tyr Ala Ala Ile Ala Lys Asn
1               5                   10                  15

atc cgc ttc ccg agg cag gcc ttc atc gat gga gct ttc cgc gac tcg        96
Ile Arg Phe Pro Arg Gln Ala Phe Ile Asp Gly Ala Phe Arg Asp Ser
            20                  25                  30

ctg tcg ggc aag acc ttc gtc acc acc aac ccc gcc acc ggc gaa gtg       144
Leu Ser Gly Lys Thr Phe Val Thr Thr Asn Pro Ala Thr Gly Glu Val
        35                  40                  45

ctg gcc gag gtc acc gcc tgc aat tcg cag gac gtc gat ctg gcg gtg       192
Leu Ala Glu Val Thr Ala Cys Asn Ser Gln Asp Val Asp Leu Ala Val
    50                  55                  60

gcc aac gcc aag gct gcg ttc gac gat ggc cgc tgg cag cgc ctg ccg       240
Ala Asn Ala Lys Ala Ala Phe Asp Asp Gly Arg Trp Gln Arg Leu Pro
65                  70                  75                  80

ccg tcg cag cgc aag acc gtg ctg gtg cgt ttc gcc gag ctg ctg gaa       288
Pro Ser Gln Arg Lys Thr Val Leu Val Arg Phe Ala Glu Leu Leu Glu
                85                  90                  95

gag cat gcg cac gaa ctg gcg gtg atg gaa agc ctg gac agc ggc aag       336
Glu His Ala His Glu Leu Ala Val Met Glu Ser Leu Asp Ser Gly Lys
            100                 105                 110

ccg gtg cgc gag tgc cag act gcc gac gtg ccg gaa gcg atc cat acc       384
Pro Val Arg Glu Cys Gln Thr Ala Asp Val Pro Glu Ala Ile His Thr
        115                 120                 125

atc cgc tgg cac gcc gaa ctg atc gac aag atc tac gac aac acc gcg       432
Ile Arg Trp His Ala Glu Leu Ile Asp Lys Ile Tyr Asp Asn Thr Ala
    130                 135                 140

ccc gtg ggc tcg aac gcg ctg gcg ctg gtg gtg cgc gag ccg atc ggc       480
Pro Val Gly Ser Asn Ala Leu Ala Leu Val Val Arg Glu Pro Ile Gly
145                 150                 155                 160

gtg gtc ggc ctg gtg ctg ccg tgg aac ttc ccg ttg ctg atg ctg gca       528
Val Val Gly Leu Val Leu Pro Trp Asn Phe Pro Leu Leu Met Leu Ala
                165                 170                 175

tgg aag atc ggt ccc tcg ctg gcg gcc ggt tgc tcg ata gtg gtc aag 576
Trp Lys Ile Gly Pro Ser Leu Ala Ala Gly Cys Ser Ile Val Val Lys
            180                 185                 190

ccg gcc aag gaa acg acg ctg acc gcg ctg cgc gtg gcc gaa ctc gcc 624
Pro Ala Lys Glu Thr Thr Leu Thr Ala Leu Arg Val Ala Glu Leu Ala
        195                 200                 205

cac cag gcc ggc gtg ccc gcc ggc gtg ttc aac gtg ctg ccg ggc ggc 672
His Gln Ala Gly Val Pro Ala Gly Val Phe Asn Val Leu Pro Gly Gly
    210                 215                 220

ggt cgc gaa gtc ggt gag ccg ctg ggc aag cac aag gac gtc acc atg 720
Gly Arg Glu Val Gly Glu Pro Leu Gly Lys His Lys Asp Val Thr Met
225                 230                 235                 240

gtc agc ttc acc ggc tcg acc gac acc ggc cgg ctg ttc ctg cag tat 768
Val Ser Phe Thr Gly Ser Thr Asp Thr Gly Arg Leu Phe Leu Gln Tyr
                245                 250                 255

gcg gcc gag tcg aac ctg aag cgc gtg gtg ctg gag tgc ggc ggc aag 816
Ala Ala Glu Ser Asn Leu Lys Arg Val Val Leu Glu Cys Gly Gly Lys
            260                 265                 270

aac ccc gcc gtg gtg ctg aag gac gtc gaa gac ctc gac gtg gtc gcg 864
Asn Pro Ala Val Val Leu Lys Asp Val Glu Asp Leu Asp Val Val Ala
        275                 280                 285

caa cag gtg gtc gcc ggc gcg ttc tgg aac atg ggc gag aac tgc tcg 912
Gln Gln Val Val Ala Gly Ala Phe Trp Asn Met Gly Glu Asn Cys Ser
    290                 295                 300

gct tcg tcg cgc ctg atc gtc cat gcc gac atc aag gac gcg ctg ctc 960
Ala Ser Ser Arg Leu Ile Val His Ala Asp Ile Lys Asp Ala Leu Leu
305                 310                 315                 320

gaa cgc atg gcc gtg gcg gtc aag gag tgg aag atg ggc aac ccg ctc 1008
Glu Arg Met Ala Val Ala Val Lys Glu Trp Lys Met Gly Asn Pro Leu
                325                 330                 335

gat ccg gag cac cgg ctg ggt tcg ctc gtc agc aag tcc cac ttc gac 1056
Asp Pro Glu His Arg Leu Gly Ser Leu Val Ser Lys Ser His Phe Asp
            340                 345                 350

aag gtc cgt tcc tac ctc gat cag gct gcg gcg gaa aag ctg cgc gtg 1104
Lys Val Arg Ser Tyr Leu Asp Gln Ala Ala Ala Glu Lys Leu Arg Val
        355                 360                 365

gtc ttc ggc ggc aac gcc agc gaa ggc aac ttc gtc gag ccg acg gtg 1152
Val Phe Gly Gly Asn Ala Ser Glu Gly Asn Phe Val Glu Pro Thr Val
    370                 375                 380

gtg gac ggc gtc ggg ccg gac agc ctg ctg ttc gtc gag gaa atc ttc 1200
Val Asp Gly Val Gly Pro Asp Ser Leu Leu Phe Val Glu Glu Ile Phe
385                 390                 395                 400

ggg ccg atc ctg tcg gtc acg acc ttc aag gat gtc gat gaa gcg atc 1248
Gly Pro Ile Leu Ser Val Thr Thr Phe Lys Asp Val Asp Glu Ala Ile
                405                 410                 415

gcg ctg gcc aac gac tcg gtc tat ggt ctg gcg gcc tcg gtc tac acc 1296
Ala Leu Ala Asn Asp Ser Val Tyr Gly Leu Ala Ala Ser Val Tyr Thr

420                 425                 430

agc aac ctg aac aac gcg atc cgt atc tcg cgt gcg atc cgc gcg ggc      1344
Ser Asn Leu Asn Asn Ala Ile Arg Ile Ser Arg Ala Ile Arg Ala Gly
        435                 440                 445

gtg gtg acc gtg aac tgc ttc ggc gaa ggt gac atc acc acg ccg ttt      1392
Val Val Thr Val Asn Cys Phe Gly Glu Gly Asp Ile Thr Thr Pro Phe
    450                 455                 460

ggc ggt tac aag gag tcg ggt ttc ggc ggc cgc gac aag tcg atc tgg      1440
Gly Gly Tyr Lys Glu Ser Gly Phe Gly Gly Arg Asp Lys Ser Ile Trp
465                 470                 475                 480

gct cac gac caa tac acc gag ctc aag acc atc tgg atc gac gtg ccg      1488
Ala His Asp Gln Tyr Thr Glu Leu Lys Thr Ile Trp Ile Asp Val Pro
                485                 490                 495

ggc gcc ggc tga                                                      1500
Gly Ala Gly

<210>  10
<211>  499
<212>  PRT
<213>  Thauera butanivorans

<400>  10

Met Ala Glu Leu Leu Thr Ala Ala Glu Tyr Ala Ala Ile Ala Lys Asn
1               5                   10                  15

Ile Arg Phe Pro Arg Gln Ala Phe Ile Asp Gly Ala Phe Arg Asp Ser
            20                  25                  30

Leu Ser Gly Lys Thr Phe Val Thr Thr Asn Pro Ala Thr Gly Glu Val
        35                  40                  45

Leu Ala Glu Val Thr Ala Cys Asn Ser Gln Asp Val Asp Leu Ala Val
    50                  55                  60

Ala Asn Ala Lys Ala Ala Phe Asp Asp Gly Arg Trp Gln Arg Leu Pro
65                  70                  75                  80

Pro Ser Gln Arg Lys Thr Val Leu Val Arg Phe Ala Glu Leu Leu Glu
                85                  90                  95

Glu His Ala His Glu Leu Ala Val Met Glu Ser Leu Asp Ser Gly Lys
            100                 105                 110

Pro Val Arg Glu Cys Gln Thr Ala Asp Val Pro Glu Ala Ile His Thr
        115                 120                 125

Ile Arg Trp His Ala Glu Leu Ile Asp Lys Ile Tyr Asp Asn Thr Ala

130                 135                 140

Pro Val Gly Ser Asn Ala Leu Ala Leu Val Val Arg Glu Pro Ile Gly
145                 150                 155                 160

Val Val Gly Leu Val Leu Pro Trp Asn Phe Pro Leu Leu Met Leu Ala
                165                 170                 175

Trp Lys Ile Gly Pro Ser Leu Ala Ala Gly Cys Ser Ile Val Val Lys
            180                 185                 190

Pro Ala Lys Glu Thr Thr Leu Thr Ala Leu Arg Val Ala Glu Leu Ala
        195                 200                 205

His Gln Ala Gly Val Pro Ala Gly Val Phe Asn Val Leu Pro Gly Gly
    210                 215                 220

Gly Arg Glu Val Gly Glu Pro Leu Gly Lys His Lys Asp Val Thr Met
225                 230                 235                 240

Val Ser Phe Thr Gly Ser Thr Asp Thr Gly Arg Leu Phe Leu Gln Tyr
                245                 250                 255

Ala Ala Glu Ser Asn Leu Lys Arg Val Val Leu Glu Cys Gly Gly Lys
            260                 265                 270

Asn Pro Ala Val Val Leu Lys Asp Val Glu Asp Leu Asp Val Val Ala
        275                 280                 285

Gln Gln Val Val Ala Gly Ala Phe Trp Asn Met Gly Glu Asn Cys Ser
    290                 295                 300

Ala Ser Ser Arg Leu Ile Val His Ala Asp Ile Lys Asp Ala Leu Leu
305                 310                 315                 320

Glu Arg Met Ala Val Ala Val Lys Glu Trp Lys Met Gly Asn Pro Leu
                325                 330                 335

Asp Pro Glu His Arg Leu Gly Ser Leu Val Ser Lys Ser His Phe Asp
            340                 345                 350

Lys Val Arg Ser Tyr Leu Asp Gln Ala Ala Ala Glu Lys Leu Arg Val
        355                 360                 365

Val Phe Gly Gly Asn Ala Ser Glu Gly Asn Phe Val Glu Pro Thr Val
    370                 375                 380

Val Asp Gly Val Gly Pro Asp Ser Leu Leu Phe Val Glu Glu Ile Phe
385                 390                 395                 400

Gly Pro Ile Leu Ser Val Thr Thr Phe Lys Asp Val Asp Glu Ala Ile
                405                 410                 415

Ala Leu Ala Asn Asp Ser Val Tyr Gly Leu Ala Ala Ser Val Tyr Thr
            420                 425                 430

Ser Asn Leu Asn Asn Ala Ile Arg Ile Ser Arg Ala Ile Arg Ala Gly
        435                 440                 445

Val Val Thr Val Asn Cys Phe Gly Glu Gly Asp Ile Thr Thr Pro Phe
    450                 455                 460

Gly Gly Tyr Lys Glu Ser Gly Phe Gly Gly Arg Asp Lys Ser Ile Trp
465                 470                 475                 480

Ala His Asp Gln Tyr Thr Glu Leu Lys Thr Ile Trp Ile Asp Val Pro
                485                 490                 495

Gly Ala Gly

<210>  11
<211>  1515
<212>  DNA
<213>  Thauera butanivorans

<220>
<221>  CDS
<222>  (1)..(1515)

<400>  11
atg aat acg acc caa agc agc acc atc ccc acc gtc aaa ctg ctg atc      48
Met Asn Thr Thr Gln Ser Ser Thr Ile Pro Thr Val Lys Leu Leu Ile
1               5                   10                  15

aac ggc gaa ttc gtc gaa tcg cgc acc acc gaa tgg cgc gac gtg gtc      96
Asn Gly Glu Phe Val Glu Ser Arg Thr Thr Glu Trp Arg Asp Val Val
            20                  25                  30

aac ccc gcc acc cag cag gtg ctg gcc cgc gtg ccg atg gcc acc gca      144
Asn Pro Ala Thr Gln Gln Val Leu Ala Arg Val Pro Met Ala Thr Ala
        35                  40                  45

gaa gaa gtg gat gcc gcc gtc gcc gcg gcc agg aaa gcc ttc gaa agc      192
Glu Glu Val Asp Ala Ala Val Ala Ala Ala Arg Lys Ala Phe Glu Ser
    50                  55                  60

tgg agg aag acg ccc atc ggt gcc cgc gca cgc atc ttc ctg aag tac      240
Trp Arg Lys Thr Pro Ile Gly Ala Arg Ala Arg Ile Phe Leu Lys Tyr
65                  70                  75                  80

cag cag ttg atc cgc gag aac atg aag gaa gtg gcg gcc atc ctg acc 288
Gln Gln Leu Ile Arg Glu Asn Met Lys Glu Val Ala Ala Ile Leu Thr
                85                  90                  95

gcc gaa cag ggc aag acc ctg ccc gac gcc gag ggc gac gtc ttc cgc 336
Ala Glu Gln Gly Lys Thr Leu Pro Asp Ala Glu Gly Asp Val Phe Arg
            100                 105                 110

ggt ctg gag gtg gtg gag cac gct gcc aac atc ggc acg ctg cag ctc 384
Gly Leu Glu Val Val Glu His Ala Ala Asn Ile Gly Thr Leu Gln Leu
        115                 120                 125

ggc gga ctg gcc aac aac gtc gcc acc ggc gtc gat acc tac acc ctg 432
Gly Gly Leu Ala Asn Asn Val Ala Thr Gly Val Asp Thr Tyr Thr Leu
    130                 135                 140

ctg cag ccg atc ggc gtc tgc gcc ggc atc acg ccg ttc aac ttc ccg 480
Leu Gln Pro Ile Gly Val Cys Ala Gly Ile Thr Pro Phe Asn Phe Pro
145                 150                 155                 160

gcg atg atc ccg ctg tgg atg ttc ccg atg gcg atc gcc tgc ggc aac 528
Ala Met Ile Pro Leu Trp Met Phe Pro Met Ala Ile Ala Cys Gly Asn
                165                 170                 175

acc ttc gtg ctc aag cct tcc gaa cag gac ccg ctg tcc acg atg cgg 576
Thr Phe Val Leu Lys Pro Ser Glu Gln Asp Pro Leu Ser Thr Met Arg
            180                 185                 190

ctg gtc gaa ctg gcg atc gag gcc ggc att ccg gcc ggc gtg ctc aac 624
Leu Val Glu Leu Ala Ile Glu Ala Gly Ile Pro Ala Gly Val Leu Asn
        195                 200                 205

gtc gtg cat ggt gcg gtc gac gtg gtc aac ggc atc tgc gac cac ccg 672
Val Val His Gly Ala Val Asp Val Val Asn Gly Ile Cys Asp His Pro
    210                 215                 220

gac atc aag gcg gtg tcc ttc gtc ggc tcg acc cgc gtc ggc acc cac 720
Asp Ile Lys Ala Val Ser Phe Val Gly Ser Thr Arg Val Gly Thr His
225                 230                 235                 240

gtc tac aac cgt gcc agc ctc gcc ggc aag cgc gcg cag tgc atg atg 768
Val Tyr Asn Arg Ala Ser Leu Ala Gly Lys Arg Ala Gln Cys Met Met
                245                 250                 255

ggc gcc aag aac cat gtc gtc gtg ctg ccc gac gcg cac aag gaa cag 816
Gly Ala Lys Asn His Val Val Val Leu Pro Asp Ala His Lys Glu Gln
            260                 265                 270

acg ctg aac gcg ctg gcg ggc gcc ggc ttc ggc gcc gcc ggc cag cgc 864
Thr Leu Asn Ala Leu Ala Gly Ala Gly Phe Gly Ala Ala Gly Gln Arg
        275                 280                 285

tgc atg gcc tcg tcg gtg gtg gtc ttc gtc ggc gaa gcg gcg cag tgg 912
Cys Met Ala Ser Ser Val Val Val Phe Val Gly Glu Ala Ala Gln Trp
    290                 295                 300

ctg ccg gaa ctg gtc gag cgc gcc cgc gcg ctg aag gtc aat gcc ggc 960
Leu Pro Glu Leu Val Glu Arg Ala Arg Ala Leu Lys Val Asn Ala Gly
305                 310                 315                 320

cac gag gcg ggc acc gac gtc ggc ccg gtg atc tcg cgc gcc gcc agg 1008
His Glu Ala Gly Thr Asp Val Gly Pro Val Ile Ser Arg Ala Ala Arg
                325                 330                 335

gcc cgc atc ctg gaa ctg atc gaa gcc ggc gtg cag gaa ggc gcg acg     1056
Ala Arg Ile Leu Glu Leu Ile Glu Ala Gly Val Gln Glu Gly Ala Thr
            340                 345                 350

ctg gaa ctg gac ggc cgc gcc ccg cag gtg ctc ggc tac gcc gac ggc     1104
Leu Glu Leu Asp Gly Arg Ala Pro Gln Val Leu Gly Tyr Ala Asp Gly
        355                 360                 365

aac ttc gtc gcc ccc acg ctg ttc tcc ggc gtc aag ccc ggc atg cgc     1152
Asn Phe Val Ala Pro Thr Leu Phe Ser Gly Val Lys Pro Gly Met Arg
    370                 375                 380

att tac gac gag gag atc ttc ggc ccg gtg ctg tgc acg ctg gcg gtg     1200
Ile Tyr Asp Glu Glu Ile Phe Gly Pro Val Leu Cys Thr Leu Ala Val
385                 390                 395                 400

gac tcg ctc gac gag gcc atc gcg ctg atc aac gcc aac ccc aac ggc     1248
Asp Ser Leu Asp Glu Ala Ile Ala Leu Ile Asn Ala Asn Pro Asn Gly
                405                 410                 415

aac ggc acc ggg gtg ttc acc cag tcg ggc gcg gcg gcg cgc aag ttc     1296
Asn Gly Thr Gly Val Phe Thr Gln Ser Gly Ala Ala Ala Arg Lys Phe
            420                 425                 430

cag gag gag atc gac gtc ggc cag gtg ggc atc aac gtg ccc atc ccg     1344
Gln Glu Glu Ile Asp Val Gly Gln Val Gly Ile Asn Val Pro Ile Pro
        435                 440                 445

gta ccg gtg ccg ctg ttc tcc ttc acc ggc tcg cgc gcc tcc aag ctg     1392
Val Pro Val Pro Leu Phe Ser Phe Thr Gly Ser Arg Ala Ser Lys Leu
    450                 455                 460

ggc gac ctc ggc ccc tac ggc cag cag gtg gtc ctg ttc tac acc cag     1440
Gly Asp Leu Gly Pro Tyr Gly Gln Gln Val Val Leu Phe Tyr Thr Gln
465                 470                 475                 480

acc aag acc gtc acc gcg cgc tgg ttc gac gac ggc gtg gtg gcg ccg     1488
Thr Lys Thr Val Thr Ala Arg Trp Phe Asp Asp Gly Val Val Ala Pro
                485                 490                 495

gtc aac acc acg atc agc ctc aag taa                                 1515
Val Asn Thr Thr Ile Ser Leu Lys
            500

<210>  12
<211>  504
<212>  PRT
<213>  Thauera butanivorans

<400>  12

Met Asn Thr Thr Gln Ser Ser Thr Ile Pro Thr Val Lys Leu Leu Ile
1               5                   10                  15

Asn Gly Glu Phe Val Glu Ser Arg Thr Thr Glu Trp Arg Asp Val Val
            20                  25                  30

Asn Pro Ala Thr Gln Gln Val Leu Ala Arg Val Pro Met Ala Thr Ala
        35                  40                  45

Glu Glu Val Asp Ala Ala Val Ala Ala Ala Arg Lys Ala Phe Glu Ser
    50                  55                  60

Trp Arg Lys Thr Pro Ile Gly Ala Arg Ala Arg Ile Phe Leu Lys Tyr
65                  70                  75                  80

Gln Gln Leu Ile Arg Glu Asn Met Lys Glu Val Ala Ala Ile Leu Thr
                85                  90                  95

Ala Glu Gln Gly Lys Thr Leu Pro Asp Ala Glu Gly Asp Val Phe Arg
            100                 105                 110

Gly Leu Glu Val Val Glu His Ala Ala Asn Ile Gly Thr Leu Gln Leu
        115                 120                 125

Gly Gly Leu Ala Asn Asn Val Ala Thr Gly Val Asp Thr Tyr Thr Leu
    130                 135                 140

Leu Gln Pro Ile Gly Val Cys Ala Gly Ile Thr Pro Phe Asn Phe Pro
145                 150                 155                 160

Ala Met Ile Pro Leu Trp Met Phe Pro Met Ala Ile Ala Cys Gly Asn
                165                 170                 175

Thr Phe Val Leu Lys Pro Ser Glu Gln Asp Pro Leu Ser Thr Met Arg
            180                 185                 190

Leu Val Glu Leu Ala Ile Glu Ala Gly Ile Pro Ala Gly Val Leu Asn
        195                 200                 205

Val Val His Gly Ala Val Asp Val Val Asn Gly Ile Cys Asp His Pro
    210                 215                 220

Asp Ile Lys Ala Val Ser Phe Val Gly Ser Thr Arg Val Gly Thr His
225                 230                 235                 240

Val Tyr Asn Arg Ala Ser Leu Ala Gly Lys Arg Ala Gln Cys Met Met
                245                 250                 255

Gly Ala Lys Asn His Val Val Val Leu Pro Asp Ala His Lys Glu Gln
            260                 265                 270

Thr Leu Asn Ala Leu Ala Gly Ala Gly Phe Gly Ala Ala Gly Gln Arg
        275                 280                 285

Cys Met Ala Ser Ser Val Val Val Phe Val Gly Glu Ala Ala Gln Trp

290                 295                 300

Leu Pro Glu Leu Val Glu Arg Ala Arg Ala Leu Lys Val Asn Ala Gly
305                 310                 315                 320

His Glu Ala Gly Thr Asp Val Gly Pro Val Ile Ser Arg Ala Ala Arg
                325                 330                 335

Ala Arg Ile Leu Glu Leu Ile Glu Ala Gly Val Gln Glu Gly Ala Thr
            340                 345                 350

Leu Glu Leu Asp Gly Arg Ala Pro Gln Val Leu Gly Tyr Ala Asp Gly
        355                 360                 365

Asn Phe Val Ala Pro Thr Leu Phe Ser Gly Val Lys Pro Gly Met Arg
    370                 375                 380

Ile Tyr Asp Glu Glu Ile Phe Gly Pro Val Leu Cys Thr Leu Ala Val
385                 390                 395                 400

Asp Ser Leu Asp Glu Ala Ile Ala Leu Ile Asn Ala Asn Pro Asn Gly
                405                 410                 415

Asn Gly Thr Gly Val Phe Thr Gln Ser Gly Ala Ala Ala Arg Lys Phe
            420                 425                 430

Gln Glu Glu Ile Asp Val Gly Gln Val Gly Ile Asn Val Pro Ile Pro
        435                 440                 445

Val Pro Val Pro Leu Phe Ser Phe Thr Gly Ser Arg Ala Ser Lys Leu
    450                 455                 460

Gly Asp Leu Gly Pro Tyr Gly Gln Gln Val Val Leu Phe Tyr Thr Gln
465                 470                 475                 480

Thr Lys Thr Val Thr Ala Arg Trp Phe Asp Asp Gly Val Val Ala Pro
                485                 490                 495

Val Asn Thr Thr Ile Ser Leu Lys
            500

<210>  13
<211>  1461
<212>  DNA
<213>  Thauera butanivorans

<220>
<221>  CDS

<222>  (1)..(1461)

<400>  13
atg cgt ctt gta gac aac ttc atc gcg ggc gaa tac cgg gcg ccg gcc      48
Met Arg Leu Val Asp Asn Phe Ile Ala Gly Glu Tyr Arg Ala Pro Ala
1               5                   10                  15

tcg ggc cgc cgc ttc gac aaa cgc tcg ccg ctg gac aac cgt gtg atc      96
Ser Gly Arg Arg Phe Asp Lys Arg Ser Pro Leu Asp Asn Arg Val Ile
            20                  25                  30

gcc tcg gtc gcc gag gcg ggg cgc ggc gag atc gat gcc gcg gtc ggc      144
Ala Ser Val Ala Glu Ala Gly Arg Gly Glu Ile Asp Ala Ala Val Gly
        35                  40                  45

gcc gcc cgc gcg gcg ctg gcg ggc gag tgg ggc gtg ttg acg acg gag      192
Ala Ala Arg Ala Ala Leu Ala Gly Glu Trp Gly Val Leu Thr Thr Glu
    50                  55                  60

cag cgc gtc gat ctg ctc cat ggc gtc gcc aac gag atc acc tgc cgc      240
Gln Arg Val Asp Leu Leu His Gly Val Ala Asn Glu Ile Thr Cys Arg
65                  70                  75                  80

ttc gag gac ttc gtc gcc gcc gag atg gcc gat acc ggg cag ccg ggc      288
Phe Glu Asp Phe Val Ala Ala Glu Met Ala Asp Thr Gly Gln Pro Gly
                85                  90                  95

cat gtc atg cgc cat gtg ttc atc ccg cgc ggc gcg gcc aat ttc aag      336
His Val Met Arg His Val Phe Ile Pro Arg Gly Ala Ala Asn Phe Lys
            100                 105                 110

gtg ttc gcc gac gtc gtc aag aac gtg gcc tcg gag tcc ttc cgc atg      384
Val Phe Ala Asp Val Val Lys Asn Val Ala Ser Glu Ser Phe Arg Met
        115                 120                 125

gcg acg ccg gac ggg cag ggc gcg ctg aac tac gcg atc cgc gtg ccc      432
Ala Thr Pro Asp Gly Gln Gly Ala Leu Asn Tyr Ala Ile Arg Val Pro
    130                 135                 140

aag ggc gtg atc ggc gtc atc tgt ccg tgg aac gcg cct ttc atg ctg      480
Lys Gly Val Ile Gly Val Ile Cys Pro Trp Asn Ala Pro Phe Met Leu
145                 150                 155                 160

atg acc tgg aag gtg ggg ccg gcg ctg gcc tgc ggc aac acc gtg gtg      528
Met Thr Trp Lys Val Gly Pro Ala Leu Ala Cys Gly Asn Thr Val Val
                165                 170                 175

gtc aag ccg tcg gag gag tcg ccg cag acg gcg gcc ctg ctc ggc gag      576
Val Lys Pro Ser Glu Glu Ser Pro Gln Thr Ala Ala Leu Leu Gly Glu
            180                 185                 190

gtg atg aac gcc gtc ggc gtg ccg aag ggc gtc tac aac gtg gtg cag      624
Val Met Asn Ala Val Gly Val Pro Lys Gly Val Tyr Asn Val Val Gln
        195                 200                 205

ggt ttc ggg ccg gac tcg gcc ggc gaa ttc ctg acc cag cac ccc ggc      672
Gly Phe Gly Pro Asp Ser Ala Gly Glu Phe Leu Thr Gln His Pro Gly
    210                 215                 220

gtc gat gcg atc acc ttc acc ggc gag acg cgc acc ggc acg gcg atc      720
Val Asp Ala Ile Thr Phe Thr Gly Glu Thr Arg Thr Gly Thr Ala Ile
225                 230                 235                 240

atg aag gcc gcc tcc gaa ggc atg cgc gac gtg tcc ttc gaa ctg ggg 768
Met Lys Ala Ala Ser Glu Gly Met Arg Asp Val Ser Phe Glu Leu Gly
                245                 250                 255

ggc aag aac gcg gcc atc gtc ttc gcc gac tgc gat ttc gac gcc gcg 816
Gly Lys Asn Ala Ala Ile Val Phe Ala Asp Cys Asp Phe Asp Ala Ala
            260                 265                 270

gtc gag ggc atc ttc cgc tcg gcc ttc ctc aat acc ggg cag gtc tgc 864
Val Glu Gly Ile Phe Arg Ser Ala Phe Leu Asn Thr Gly Gln Val Cys
        275                 280                 285

ctg ggc acc gag cgc gtc tat gtc gag cgg ccg atc ttc gag cgc ttc 912
Leu Gly Thr Glu Arg Val Tyr Val Glu Arg Pro Ile Phe Glu Arg Phe
    290                 295                 300

gtg cag gcg ctc agg tcc agg gcc gag gcg gtg aaa tac ggc cgc ccg 960
Val Gln Ala Leu Arg Ser Arg Ala Glu Ala Val Lys Tyr Gly Arg Pro
305                 310                 315                 320

gag gat cgc aat gcc gac tac ggg cca ctg atc agc cag gcg cac cgc 1008
Glu Asp Arg Asn Ala Asp Tyr Gly Pro Leu Ile Ser Gln Ala His Arg
                325                 330                 335

cag aag gtg ctg tcc tac tac cgc aag gcg gtg gaa gag ggc gcc acc 1056
Gln Lys Val Leu Ser Tyr Tyr Arg Lys Ala Val Glu Glu Gly Ala Thr
            340                 345                 350

gtg gtc acc ggc ggc ggc gtg ccc gag atg gcg gaa gaa ctc gcc gac 1104
Val Val Thr Gly Gly Gly Val Pro Glu Met Ala Glu Glu Leu Ala Asp
        355                 360                 365

ggc gcc tgg gtg cag ccg acc atc tgg acc ggc ctg gcc gac gat gcg 1152
Gly Ala Trp Val Gln Pro Thr Ile Trp Thr Gly Leu Ala Asp Asp Ala
    370                 375                 380

gcc gtg gtg cgc gag gaa atc ttc ggg ccg tgc tgc cac atc cgc ccg 1200
Ala Val Val Arg Glu Glu Ile Phe Gly Pro Cys Cys His Ile Arg Pro
385                 390                 395                 400

ttc gat tcc gag gac gaa gtg gtc ggc ctc gcc aac gac acc gtg tat 1248
Phe Asp Ser Glu Asp Glu Val Val Gly Leu Ala Asn Asp Thr Val Tyr
                405                 410                 415

ggc ctg tcc acg acg atc tgg acc acc agc ctg gcg cgc gcg cac cgc 1296
Gly Leu Ser Thr Thr Ile Trp Thr Thr Ser Leu Ala Arg Ala His Arg
            420                 425                 430

gtc gcc gcg cgt gtc gag gtg ggc atc acc tgg gtg aat agc tgg ttc 1344
Val Ala Ala Arg Val Glu Val Gly Ile Thr Trp Val Asn Ser Trp Phe
        435                 440                 445

ctg cgc gac ctg cgc acg cct ttc ggc ggc tcg aag cag tcc ggc atc 1392
Leu Arg Asp Leu Arg Thr Pro Phe Gly Gly Ser Lys Gln Ser Gly Ile
    450                 455                 460

ggg cgc gag ggc ggg gtg cat tcc ctg gag ttc tac acc gag acg cgc 1440
Gly Arg Glu Gly Gly Val His Ser Leu Glu Phe Tyr Thr Glu Thr Arg
465                 470                 475                 480

aac atc tgc gtc aag ctc tga 1461
Asn Ile Cys Val Lys Leu
                485

<210>  14
<211>  486
<212>  PRT
<213>  Thauera butanivorans

<400>  14

Met Arg Leu Val Asp Asn Phe Ile Ala Gly Glu Tyr Arg Ala Pro Ala
1               5                   10                  15

Ser Gly Arg Arg Phe Asp Lys Arg Ser Pro Leu Asp Asn Arg Val Ile
            20                  25                  30

Ala Ser Val Ala Glu Ala Gly Arg Gly Glu Ile Asp Ala Ala Val Gly
        35                  40                  45

Ala Ala Arg Ala Ala Leu Ala Gly Glu Trp Gly Val Leu Thr Thr Glu
    50                  55                  60

Gln Arg Val Asp Leu Leu His Gly Val Ala Asn Glu Ile Thr Cys Arg
65                  70                  75                  80

Phe Glu Asp Phe Val Ala Ala Glu Met Ala Asp Thr Gly Gln Pro Gly
                85                  90                  95

His Val Met Arg His Val Phe Ile Pro Arg Gly Ala Ala Asn Phe Lys
            100                 105                 110

Val Phe Ala Asp Val Val Lys Asn Val Ala Ser Glu Ser Phe Arg Met
        115                 120                 125

Ala Thr Pro Asp Gly Gln Gly Ala Leu Asn Tyr Ala Ile Arg Val Pro
    130                 135                 140

Lys Gly Val Ile Gly Val Ile Cys Pro Trp Asn Ala Pro Phe Met Leu
145                 150                 155                 160

Met Thr Trp Lys Val Gly Pro Ala Leu Ala Cys Gly Asn Thr Val Val
                165                 170                 175

Val Lys Pro Ser Glu Glu Ser Pro Gln Thr Ala Ala Leu Leu Gly Glu
            180                 185                 190

Val Met Asn Ala Val Gly Val Pro Lys Gly Val Tyr Asn Val Val Gln
        195                 200                 205

Gly Phe Gly Pro Asp Ser Ala Gly Glu Phe Leu Thr Gln His Pro Gly
    210                 215                 220

Val Asp Ala Ile Thr Phe Thr Gly Glu Thr Arg Thr Gly Thr Ala Ile
225                 230                 235                 240

Met Lys Ala Ala Ser Glu Gly Met Arg Asp Val Ser Phe Glu Leu Gly
                245                 250                 255

Gly Lys Asn Ala Ala Ile Val Phe Ala Asp Cys Asp Phe Asp Ala Ala
            260                 265                 270

Val Glu Gly Ile Phe Arg Ser Ala Phe Leu Asn Thr Gly Gln Val Cys
        275                 280                 285

Leu Gly Thr Glu Arg Val Tyr Val Glu Arg Pro Ile Phe Glu Arg Phe
    290                 295                 300

Val Gln Ala Leu Arg Ser Arg Ala Glu Ala Val Lys Tyr Gly Arg Pro
305                 310                 315                 320

Glu Asp Arg Asn Ala Asp Tyr Gly Pro Leu Ile Ser Gln Ala His Arg
                325                 330                 335

Gln Lys Val Leu Ser Tyr Tyr Arg Lys Ala Val Glu Glu Gly Ala Thr
            340                 345                 350

Val Val Thr Gly Gly Gly Val Pro Glu Met Ala Glu Glu Leu Ala Asp
        355                 360                 365

Gly Ala Trp Val Gln Pro Thr Ile Trp Thr Gly Leu Ala Asp Asp Ala
    370                 375                 380

Ala Val Val Arg Glu Glu Ile Phe Gly Pro Cys Cys His Ile Arg Pro
385                 390                 395                 400

Phe Asp Ser Glu Asp Glu Val Val Gly Leu Ala Asn Asp Thr Val Tyr
                405                 410                 415

Gly Leu Ser Thr Thr Ile Trp Thr Thr Ser Leu Ala Arg Ala His Arg
            420                 425                 430

Val Ala Ala Arg Val Glu Val Gly Ile Thr Trp Val Asn Ser Trp Phe
        435                 440                 445

Leu Arg Asp Leu Arg Thr Pro Phe Gly Gly Ser Lys Gln Ser Gly Ile
    450                 455                 460

Gly Arg Glu Gly Gly Val His Ser Leu Glu Phe Tyr Thr Glu Thr Arg

465                 470                 475                 480

Asn Ile Cys Val Lys Leu
                485

<210>  15
<211>  1521
<212>  DNA
<213>  Thauera butanivorans

<220>
<221>  CDS
<222>  (1)..(1521)

<400>  15
atg atc tac gca atg ccc ggc cag tcc ggt gcc ccg gtg cag cac aag      48
Met Ile Tyr Ala Met Pro Gly Gln Ser Gly Ala Pro Val Gln His Lys
1               5                   10                  15

gcg cgc tac gac aac ttc atc ggc ggc cgt tgg gtt tcg ccg gtc aag      96
Ala Arg Tyr Asp Asn Phe Ile Gly Gly Arg Trp Val Ser Pro Val Lys
            20                  25                  30

ggc cag tac ttc gac gtg gtg acg ccg atc agc ggc cag gtc tat acg      144
Gly Gln Tyr Phe Asp Val Val Thr Pro Ile Ser Gly Gln Val Tyr Thr
        35                  40                  45

cag gcg gca cgc tcc acc gcc gag gac atc gag ctg gcg ctg gac gcg      192
Gln Ala Ala Arg Ser Thr Ala Glu Asp Ile Glu Leu Ala Leu Asp Ala
    50                  55                  60

gcg cac gcg gcg ttt ccg gcc tgg agc aag acg ccg gcg gcc gag cgg      240
Ala His Ala Ala Phe Pro Ala Trp Ser Lys Thr Pro Ala Ala Glu Arg
65                  70                  75                  80

gcc aac att ctg ctc cgg atc gcc gag cgc atc gaa cag aac ctg gaa      288
Ala Asn Ile Leu Leu Arg Ile Ala Glu Arg Ile Glu Gln Asn Leu Glu
                85                  90                  95

cgg ctg gcc tac gcc gag acg gtc gac aac ggc aag gcg atc cgc gag      336
Arg Leu Ala Tyr Ala Glu Thr Val Asp Asn Gly Lys Ala Ile Arg Glu
            100                 105                 110

acg ctc aat gcc gac att ccg ctc acc gtc gat cac ttc cgc tac ttc      384
Thr Leu Asn Ala Asp Ile Pro Leu Thr Val Asp His Phe Arg Tyr Phe
        115                 120                 125

gcc ggc tgc ctg cgc gcg cag gaa ggc agc atc agc gag atc gac gag      432
Ala Gly Cys Leu Arg Ala Gln Glu Gly Ser Ile Ser Glu Ile Asp Glu
    130                 135                 140

aac acc gtc gcc tac cac ctg cac gaa ccg ctg ggc gtg gtc ggg cag      480
Asn Thr Val Ala Tyr His Leu His Glu Pro Leu Gly Val Val Gly Gln
145                 150                 155                 160

atc atc ccg tgg aac ttc ccg atc ctg atg gcg gcc tgg aag ctc gcg      528
Ile Ile Pro Trp Asn Phe Pro Ile Leu Met Ala Ala Trp Lys Leu Ala
                165                 170                 175

ccg gcg ctg ggc gcg ggc aac tgc gtg gtg ttg aag ccc gcc gaa tcg      576

Pro Ala Leu Gly Ala Gly Asn Cys Val Val Leu Lys Pro Ala Glu Ser
        180                 185                 190

acg ccg gtc tcg atc atg gtg ctg gtc gaa ctg atc gcc gac ctg ctg     624
Thr Pro Val Ser Ile Met Val Leu Val Glu Leu Ile Ala Asp Leu Leu
        195                 200                 205

ccg ccg ggc gtg ctc aac atc gtc aac ggc ttc ggc cgc gaa gcc ggc     672
Pro Pro Gly Val Leu Asn Ile Val Asn Gly Phe Gly Arg Glu Ala Gly
    210                 215                 220

atg ccg ctg gcc acc agc aag cgc atc gcc aag atc gcc ttc acc ggc     720
Met Pro Leu Ala Thr Ser Lys Arg Ile Ala Lys Ile Ala Phe Thr Gly
225                 230                 235                 240

tcg acc tcg acc ggg cgg gtg atc gcc cag gcc gcg gcc aac aac ctg     768
Ser Thr Ser Thr Gly Arg Val Ile Ala Gln Ala Ala Ala Asn Asn Leu
                245                 250                 255

atc ccg gcc acg ctg gag ctg ggc ggc aag tcg ccc aac atc ttc ttc     816
Ile Pro Ala Thr Leu Glu Leu Gly Gly Lys Ser Pro Asn Ile Phe Phe
            260                 265                 270

gcc gac gtc atg gac cgc gac gac gcc ttc ctc gac aag gcc atc gaa     864
Ala Asp Val Met Asp Arg Asp Asp Ala Phe Leu Asp Lys Ala Ile Glu
        275                 280                 285

ggc atg gtg ctg ttc gcc ttc aac cag ggc gaa gtg tgc acc tgc ccg     912
Gly Met Val Leu Phe Ala Phe Asn Gln Gly Glu Val Cys Thr Cys Pro
    290                 295                 300

agc cgg gcg ctg atc cag gaa agc atc tac gag cgc ttc atc gag cgc     960
Ser Arg Ala Leu Ile Gln Glu Ser Ile Tyr Glu Arg Phe Ile Glu Arg
305                 310                 315                 320

gtg ctc gag cgc gtg gcg gcg atc aag cac ggc cac ccg ctc gac acc     1008
Val Leu Glu Arg Val Ala Ala Ile Lys His Gly His Pro Leu Asp Thr
                325                 330                 335

gag tcg atg atg ggc gcg cag gcc tcg cgc gag caa atg agc aag atc     1056
Glu Ser Met Met Gly Ala Gln Ala Ser Arg Glu Gln Met Ser Lys Ile
            340                 345                 350

cag tcc tac ctg gcg ctg ggc aag gaa gaa ggg gcc gaa gtg ctg atc     1104
Gln Ser Tyr Leu Ala Leu Gly Lys Glu Glu Gly Ala Glu Val Leu Ile
        355                 360                 365

ggc ggc gac aag gcg aat ctg ggc ggc gaa ctg agc gag ggc tac tac     1152
Gly Gly Asp Lys Ala Asn Leu Gly Gly Glu Leu Ser Glu Gly Tyr Tyr
    370                 375                 380

atc cag ccc acg ctg ttc aag ggc cac aac aag atg cgc atc ttc cag     1200
Ile Gln Pro Thr Leu Phe Lys Gly His Asn Lys Met Arg Ile Phe Gln
385                 390                 395                 400

gaa gag atc ttc ggc ccg gtg ctg gcg gtg acc acc ttc aag gac gaa     1248
Glu Glu Ile Phe Gly Pro Val Leu Ala Val Thr Thr Phe Lys Asp Glu
                405                 410                 415

gcc gag gcg ctg gcg atc gcc aac gac acg ctg tac ggg ctg ggt gcg     1296
Ala Glu Ala Leu Ala Ile Ala Asn Asp Thr Leu Tyr Gly Leu Gly Ala
            420                 425                 430

ggg gtg tgg agc cgc aac ggc aac gtg gcc tac cgc atg ggc cgg gcg 1344
Gly Val Trp Ser Arg Asn Gly Asn Val Ala Tyr Arg Met Gly Arg Ala
        435                 440                 445

atc cag gcc ggg cgg gtg tgg acc aac tgc tac cac gcc tat ccg gcg 1392
Ile Gln Ala Gly Arg Val Trp Thr Asn Cys Tyr His Ala Tyr Pro Ala
    450                 455                 460

cat gcg gcc ttc ggc ggc tac aag gag tcg ggc atc ggg cgc gag acc 1440
His Ala Ala Phe Gly Gly Tyr Lys Glu Ser Gly Ile Gly Arg Glu Thr
465                 470                 475                 480

cac aag atg atg ctc gac cac tac cag cag acc aag aac ctg ctc gtc 1488
His Lys Met Met Leu Asp His Tyr Gln Gln Thr Lys Asn Leu Leu Val
                485                 490                 495

agc tac agc gag aac aag ctc ggg ttc ttc tga 1521
Ser Tyr Ser Glu Asn Lys Leu Gly Phe Phe
            500                 505

<210> 16
<211> 506
<212> PRT
<213> Thauera butanivorans

<400> 16

Met Ile Tyr Ala Met Pro Gly Gln Ser Gly Ala Pro Val Gln His Lys
1               5                   10                  15

Ala Arg Tyr Asp Asn Phe Ile Gly Gly Arg Trp Val Ser Pro Val Lys
            20                  25                  30

Gly Gln Tyr Phe Asp Val Val Thr Pro Ile Ser Gly Gln Val Tyr Thr
        35                  40                  45

Gln Ala Ala Arg Ser Thr Ala Glu Asp Ile Glu Leu Ala Leu Asp Ala
    50                  55                  60

Ala His Ala Ala Phe Pro Ala Trp Ser Lys Thr Pro Ala Ala Glu Arg
65                  70                  75                  80

Ala Asn Ile Leu Leu Arg Ile Ala Glu Arg Ile Glu Gln Asn Leu Glu
                85                  90                  95

Arg Leu Ala Tyr Ala Glu Thr Val Asp Asn Gly Lys Ala Ile Arg Glu
            100                 105                 110

Thr Leu Asn Ala Asp Ile Pro Leu Thr Val Asp His Phe Arg Tyr Phe
        115                 120                 125

Ala Gly Cys Leu Arg Ala Gln Glu Gly Ser Ile Ser Glu Ile Asp Glu
    130                 135                 140

Asn Thr Val Ala Tyr His Leu His Glu Pro Leu Gly Val Val Gly Gln
145                 150                 155                 160

Ile Ile Pro Trp Asn Phe Pro Ile Leu Met Ala Ala Trp Lys Leu Ala
                165                 170                 175

Pro Ala Leu Gly Ala Gly Asn Cys Val Val Leu Lys Pro Ala Glu Ser
            180                 185                 190

Thr Pro Val Ser Ile Met Val Leu Val Glu Leu Ile Ala Asp Leu Leu
        195                 200                 205

Pro Pro Gly Val Leu Asn Ile Val Asn Gly Phe Gly Arg Glu Ala Gly
    210                 215                 220

Met Pro Leu Ala Thr Ser Lys Arg Ile Ala Lys Ile Ala Phe Thr Gly
225                 230                 235                 240

Ser Thr Ser Thr Gly Arg Val Ile Ala Gln Ala Ala Ala Asn Asn Leu
                245                 250                 255

Ile Pro Ala Thr Leu Glu Leu Gly Gly Lys Ser Pro Asn Ile Phe Phe
            260                 265                 270

Ala Asp Val Met Asp Arg Asp Asp Ala Phe Leu Asp Lys Ala Ile Glu
        275                 280                 285

Gly Met Val Leu Phe Ala Phe Asn Gln Gly Glu Val Cys Thr Cys Pro
    290                 295                 300

Ser Arg Ala Leu Ile Gln Glu Ser Ile Tyr Glu Arg Phe Ile Glu Arg
305                 310                 315                 320

Val Leu Glu Arg Val Ala Ala Ile Lys His Gly His Pro Leu Asp Thr
                325                 330                 335

Glu Ser Met Met Gly Ala Gln Ala Ser Arg Glu Gln Met Ser Lys Ile
            340                 345                 350

Gln Ser Tyr Leu Ala Leu Gly Lys Glu Glu Gly Ala Glu Val Leu Ile
        355                 360                 365

Gly Gly Asp Lys Ala Asn Leu Gly Gly Glu Leu Ser Glu Gly Tyr Tyr
    370                 375                 380

Ile Gln Pro Thr Leu Phe Lys Gly His Asn Lys Met Arg Ile Phe Gln
385                 390                 395                 400

Glu Glu Ile Phe Gly Pro Val Leu Ala Val Thr Thr Phe Lys Asp Glu
                405                 410                 415

Ala Glu Ala Leu Ala Ile Ala Asn Asp Thr Leu Tyr Gly Leu Gly Ala
            420                 425                 430

Gly Val Trp Ser Arg Asn Gly Asn Val Ala Tyr Arg Met Gly Arg Ala
        435                 440                 445

Ile Gln Ala Gly Arg Val Trp Thr Asn Cys Tyr His Ala Tyr Pro Ala
    450                 455                 460

His Ala Ala Phe Gly Gly Tyr Lys Glu Ser Gly Ile Gly Arg Glu Thr
465                 470                 475                 480

His Lys Met Met Leu Asp His Tyr Gln Gln Thr Lys Asn Leu Leu Val
                485                 490                 495

Ser Tyr Ser Glu Asn Lys Leu Gly Phe Phe
            500                 505

<210>  17
<211>  1458
<212>  DNA
<213>  Thauera butanivorans

<220>
<221>  CDS
<222>  (1)..(1458)

<400>  17
atg caa ctg acc gat tcc gcg ctg ctg cgc acc caa gcc tat atc gac      48
Met Gln Leu Thr Asp Ser Ala Leu Leu Arg Thr Gln Ala Tyr Ile Asp
1               5                   10                  15

ggc gcc tgg cgc gat gcg acc gac ggc agc cgc ttc gac gtg aac aat      96
Gly Ala Trp Arg Asp Ala Thr Asp Gly Ser Arg Phe Asp Val Asn Asn
            20                  25                  30

ccg gcc gac ggc cgc gtg ctg gcg agc gtg ccg gac atg ggg cgc acc      144
Pro Ala Asp Gly Arg Val Leu Ala Ser Val Pro Asp Met Gly Arg Thr
        35                  40                  45

gag acc cgg cag gcg atc gag gcc gca gcc gcc gcc ctg ccg gcc tgg      192
Glu Thr Arg Gln Ala Ile Glu Ala Ala Ala Ala Ala Leu Pro Ala Trp
    50                  55                  60

cgg gcg cgc acc gcc aag gaa cgc gcc gcc gtg ctg cgc aag tgg ttc      240
Arg Ala Arg Thr Ala Lys Glu Arg Ala Ala Val Leu Arg Lys Trp Phe
65                  70                  75                  80

gaa ctg atg ctc gcg cac cag gaa gac ctg gcg ctg ctg atg agc tcg      288
Glu Leu Met Leu Ala His Gln Glu Asp Leu Ala Leu Leu Met Ser Ser

85                  90                  95

gag cag ggc aag ccg ctg ccc gaa gcg cgc ggc gaa gtg gcc tat ggc        336
Glu Gln Gly Lys Pro Leu Pro Glu Ala Arg Gly Glu Val Ala Tyr Gly
            100                 105                 110

gcc tct ttc ctc gaa tgg ttc gcc gag gaa ggc aag cgc atc tac ggc        384
Ala Ser Phe Leu Glu Trp Phe Ala Glu Glu Gly Lys Arg Ile Tyr Gly
        115                 120                 125

gac gtg att ccc gcg cac ggc gcg gac aag cgc atc gtc gtg gtc aag        432
Asp Val Ile Pro Ala His Gly Ala Asp Lys Arg Ile Val Val Val Lys
    130                 135                 140

cag ccg atc ggc gtg gtg gcg gcg gtg acg ccg tgg aat ttc ccc atc        480
Gln Pro Ile Gly Val Val Ala Ala Val Thr Pro Trp Asn Phe Pro Ile
145                 150                 155                 160

gcg atg atc acg cgc aag gcc ggc ccg gcg ctg gcc gcc ggc tgc acg        528
Ala Met Ile Thr Arg Lys Ala Gly Pro Ala Leu Ala Ala Gly Cys Thr
                165                 170                 175

atg gtg atc aag ccg gcc gag gac acg ccg ctg tgc gcg ctg gcg ctg        576
Met Val Ile Lys Pro Ala Glu Asp Thr Pro Leu Cys Ala Leu Ala Leu
            180                 185                 190

gcc gag ctc ggt gag cgc gcc ggc ctg ccc agg ggc gtg ctc aac atc        624
Ala Glu Leu Gly Glu Arg Ala Gly Leu Pro Arg Gly Val Leu Asn Ile
        195                 200                 205

gtc acc acc gcg agg gcg gcc gaa gtc ggc ggc gaa ctc acc gcc aat        672
Val Thr Thr Ala Arg Ala Ala Glu Val Gly Gly Glu Leu Thr Ala Asn
    210                 215                 220

ccg ctg gtg cgc aag atc tcc ttc acc ggc tcg acc gaa gtc ggc aag        720
Pro Leu Val Arg Lys Ile Ser Phe Thr Gly Ser Thr Glu Val Gly Lys
225                 230                 235                 240

ctg ctg atg cgc cag tcc gcc gac aca gtc aag aaa gtg tcg ctc gaa        768
Leu Leu Met Arg Gln Ser Ala Asp Thr Val Lys Lys Val Ser Leu Glu
                245                 250                 255

ctt ggc ggc aat gca ccc ttc atc gtc ttc gac gat gcc gac ctg gat        816
Leu Gly Gly Asn Ala Pro Phe Ile Val Phe Asp Asp Ala Asp Leu Asp
            260                 265                 270

gcc gct gtc gcc ggc gcg atg gcc tcg aag tac cgc aac gcc ggc cag        864
Ala Ala Val Ala Gly Ala Met Ala Ser Lys Tyr Arg Asn Ala Gly Gln
        275                 280                 285

acc tgc gtc tgc gcc aac cgc ctg atc gtg cag gac ggc atc cat gac        912
Thr Cys Val Cys Ala Asn Arg Leu Ile Val Gln Asp Gly Ile His Asp
    290                 295                 300

gcc ttc gcc gcc aaa ctg gcc gaa gcg gtg gcg aag ctg aag gtc ggc        960
Ala Phe Ala Ala Lys Leu Ala Glu Ala Val Ala Lys Leu Lys Val Gly
305                 310                 315                 320

ccg gca ctg tcg ggc gat gtc cag caa ggc ccg ctg atc aac gag gac       1008
Pro Ala Leu Ser Gly Asp Val Gln Gln Gly Pro Leu Ile Asn Glu Asp
                325                 330                 335

gcg gtc gcc aag gtc gaa acc ctg ctc gcc gat gcg ctc gac aag ggc       1056

Ala Val Ala Lys Val Glu Thr Leu Leu Ala Asp Ala Leu Asp Lys Gly
            340                 345                 350

gcg cgg ctg gtc tgc ggc ggc agg cgc cat gcg ctc ggc ggc acc ttc      1104
Ala Arg Leu Val Cys Gly Gly Arg Arg His Ala Leu Gly Gly Thr Phe
        355                 360                 365

ttc gaa ccc acc atc gtc gcc gac gtg acg ccg gcg atg cgg gtg gcg      1152
Phe Glu Pro Thr Ile Val Ala Asp Val Thr Pro Ala Met Arg Val Ala
    370                 375                 380

cgc gag gaa atc ttc ggc ccg gtg gcg ccg ctg ttc cgc ttc cat gcc      1200
Arg Glu Glu Ile Phe Gly Pro Val Ala Pro Leu Phe Arg Phe His Ala
385                 390                 395                 400

gaa gaa gag gcg atc cgc atg gcc aac gac acc gag ttc ggc ctc gcg      1248
Glu Glu Glu Ala Ile Arg Met Ala Asn Asp Thr Glu Phe Gly Leu Ala
                405                 410                 415

gcg tac ttc tac gcc cgc gac atc ggc cgc gtg tgg cgt gtc gcc gag      1296
Ala Tyr Phe Tyr Ala Arg Asp Ile Gly Arg Val Trp Arg Val Ala Glu
            420                 425                 430

gcg ctg gaa tac ggc atc gtc ggc atc aac gaa ggc atc atc tcg acc      1344
Ala Leu Glu Tyr Gly Ile Val Gly Ile Asn Glu Gly Ile Ile Ser Thr
        435                 440                 445

gag gtc gca ccc ttc ggc ggc atc aag gag tcg ggc atc ggc cgc gaa      1392
Glu Val Ala Pro Phe Gly Gly Ile Lys Glu Ser Gly Ile Gly Arg Glu
    450                 455                 460

ggc tcg cgc tac ggc atc gag gac ttc gtc gag atc aag tac ctg tgc      1440
Gly Ser Arg Tyr Gly Ile Glu Asp Phe Val Glu Ile Lys Tyr Leu Cys
465                 470                 475                 480

atg ggc ggc atc cgt tga                                              1458
Met Gly Gly Ile Arg
                485


<210>  18
<211>  485
<212>  PRT
<213>  Thauera butanivorans

<400>  18

Met Gln Leu Thr Asp Ser Ala Leu Leu Arg Thr Gln Ala Tyr Ile Asp
1               5                   10                  15


Gly Ala Trp Arg Asp Ala Thr Asp Gly Ser Arg Phe Asp Val Asn Asn
            20                  25                  30


Pro Ala Asp Gly Arg Val Leu Ala Ser Val Pro Asp Met Gly Arg Thr
        35                  40                  45


Glu Thr Arg Gln Ala Ile Glu Ala Ala Ala Ala Ala Leu Pro Ala Trp
    50                  55                  60

Arg Ala Arg Thr Ala Lys Glu Arg Ala Ala Val Leu Arg Lys Trp Phe
65                  70                  75                  80

Glu Leu Met Leu Ala His Gln Glu Asp Leu Ala Leu Leu Met Ser Ser
                85                  90                  95

Glu Gln Gly Lys Pro Leu Pro Glu Ala Arg Gly Glu Val Ala Tyr Gly
            100                 105                 110

Ala Ser Phe Leu Glu Trp Phe Ala Glu Glu Gly Lys Arg Ile Tyr Gly
        115                 120                 125

Asp Val Ile Pro Ala His Gly Ala Asp Lys Arg Ile Val Val Val Lys
    130                 135                 140

Gln Pro Ile Gly Val Val Ala Ala Val Thr Pro Trp Asn Phe Pro Ile
145                 150                 155                 160

Ala Met Ile Thr Arg Lys Ala Gly Pro Ala Leu Ala Ala Gly Cys Thr
                165                 170                 175

Met Val Ile Lys Pro Ala Glu Asp Thr Pro Leu Cys Ala Leu Ala Leu
            180                 185                 190

Ala Glu Leu Gly Glu Arg Ala Gly Leu Pro Arg Gly Val Leu Asn Ile
        195                 200                 205

Val Thr Thr Ala Arg Ala Ala Glu Val Gly Gly Glu Leu Thr Ala Asn
    210                 215                 220

Pro Leu Val Arg Lys Ile Ser Phe Thr Gly Ser Thr Glu Val Gly Lys
225                 230                 235                 240

Leu Leu Met Arg Gln Ser Ala Asp Thr Val Lys Lys Val Ser Leu Glu
                245                 250                 255

Leu Gly Gly Asn Ala Pro Phe Ile Val Phe Asp Asp Ala Asp Leu Asp
            260                 265                 270

Ala Ala Val Ala Gly Ala Met Ala Ser Lys Tyr Arg Asn Ala Gly Gln
        275                 280                 285

Thr Cys Val Cys Ala Asn Arg Leu Ile Val Gln Asp Gly Ile His Asp
    290                 295                 300

Ala Phe Ala Ala Lys Leu Ala Glu Ala Val Ala Lys Leu Lys Val Gly
305                 310                 315                 320

Pro Ala Leu Ser Gly Asp Val Gln Gln Gly Pro Leu Ile Asn Glu Asp
                325                 330                 335

Ala Val Ala Lys Val Glu Thr Leu Leu Ala Asp Ala Leu Asp Lys Gly
            340                 345                 350

Ala Arg Leu Val Cys Gly Gly Arg Arg His Ala Leu Gly Gly Thr Phe
        355                 360                 365

Phe Glu Pro Thr Ile Val Ala Asp Val Thr Pro Ala Met Arg Val Ala
    370                 375                 380

Arg Glu Glu Ile Phe Gly Pro Val Ala Pro Leu Phe Arg Phe His Ala
385                 390                 395                 400

Glu Glu Glu Ala Ile Arg Met Ala Asn Asp Thr Glu Phe Gly Leu Ala
                405                 410                 415

Ala Tyr Phe Tyr Ala Arg Asp Ile Gly Arg Val Trp Arg Val Ala Glu
            420                 425                 430

Ala Leu Glu Tyr Gly Ile Val Gly Ile Asn Glu Gly Ile Ile Ser Thr
        435                 440                 445

Glu Val Ala Pro Phe Gly Gly Ile Lys Glu Ser Gly Ile Gly Arg Glu
    450                 455                 460

Gly Ser Arg Tyr Gly Ile Glu Asp Phe Val Glu Ile Lys Tyr Leu Cys
465                 470                 475                 480

Met Gly Gly Ile Arg
                485

<210> 19
<211> 1029
<212> DNA
<213> Thauera butanivorans

<220>
<221> CDS
<222> (1)..(1029)

<400> 19
atg agc atc aag gtc gcc atc aac ggt ttc ggt cgt atc ggt cgc tgc 48
Met Ser Ile Lys Val Ala Ile Asn Gly Phe Gly Arg Ile Gly Arg Cys
1               5                   10                  15

acg ctg cgc gcc atc tac gag cag gga ctg cag aac gaa ttc gaa gtg 96
Thr Leu Arg Ala Ile Tyr Glu Gln Gly Leu Gln Asn Glu Phe Glu Val
            20                  25                  30

gtc gcc atc aac gct tcc ggc gat ctg gcc acc aat gcc cac ctg ttg 144
Val Ala Ile Asn Ala Ser Gly Asp Leu Ala Thr Asn Ala His Leu Leu
        35                  40                  45

aag tac gac acc acc cac ggc cgc ttc gcc acc tcg gtc gag acc gaa 192
Lys Tyr Asp Thr Thr His Gly Arg Phe Ala Thr Ser Val Glu Thr Glu
    50                  55                  60

ggc gag aac gtc atc atc atc gac ggc aag aag atc ccc ttc tac tcc 240
Gly Glu Asn Val Ile Ile Ile Asp Gly Lys Lys Ile Pro Phe Tyr Ser
65                  70                  75                  80

acc aag gac ccg aag ggc gtc aac tgg ggc agc cac ggc gtg gac gtg 288
Thr Lys Asp Pro Lys Gly Val Asn Trp Gly Ser His Gly Val Asp Val
                85                  90                  95

ctg ctg gaa tgc acc ggc gcc tac acc acc aag gcc aag gcc cag gcc 336
Leu Leu Glu Cys Thr Gly Ala Tyr Thr Thr Lys Ala Lys Ala Gln Ala
            100                 105                 110

ctg ctc gac cag ggt gcc cgc cgc gtg ctg atc tcg gcg ccg ggc ggc 384
Leu Leu Asp Gln Gly Ala Arg Arg Val Leu Ile Ser Ala Pro Gly Gly
        115                 120                 125

gac gac gtg gac acc acc atc gtc atg ggc gtc aat gaa gac gcg ctg 432
Asp Asp Val Asp Thr Thr Ile Val Met Gly Val Asn Glu Asp Ala Leu
    130                 135                 140

aag gct ggc atg acc gtc gtt tcc aac gcc tcg tgc acc acc aac tgc 480
Lys Ala Gly Met Thr Val Val Ser Asn Ala Ser Cys Thr Thr Asn Cys
145                 150                 155                 160

ctg gcc ccg gtg gcc aag atc ctg gcc gac agc gtg ggc atc aag cag 528
Leu Ala Pro Val Ala Lys Ile Leu Ala Asp Ser Val Gly Ile Lys Gln
                165                 170                 175

ggt ctg atg acc acc atc cac gcc tac acc aac gac cag gtc acc gtc 576
Gly Leu Met Thr Thr Ile His Ala Tyr Thr Asn Asp Gln Val Thr Val
            180                 185                 190

gac gtc cgc cac aag gac ctg cgc cgc gcc cgc gcc gcc gcc gcc aac 624
Asp Val Arg His Lys Asp Leu Arg Arg Ala Arg Ala Ala Ala Ala Asn
        195                 200                 205

atc atc ccg acc aag acc ggc gcc gcc aag gcc gtc ggc ctg gtg ctg 672
Ile Ile Pro Thr Lys Thr Gly Ala Ala Lys Ala Val Gly Leu Val Leu
    210                 215                 220

ccg caa ctg gcc ggc aag gtc gac ggc ttc gcc ctg cgc gtg ccg acg 720
Pro Gln Leu Ala Gly Lys Val Asp Gly Phe Ala Leu Arg Val Pro Thr
225                 230                 235                 240

atc aac gtc tcg ctg gtc gat ctg acc ttc acc gcc gag cgc gcc acc 768
Ile Asn Val Ser Leu Val Asp Leu Thr Phe Thr Ala Glu Arg Ala Thr
                245                 250                 255

acc aag gaa gag atc aac gag ctg atg agc gcc gcg gcc aac ggc ccg 816
Thr Lys Glu Glu Ile Asn Glu Leu Met Ser Ala Ala Ala Asn Gly Pro
            260                 265                 270

ctg aag ggc atc ctg gcg gtc aac acc gag ccg ctg gtg tct tcc gac 864
Leu Lys Gly Ile Leu Ala Val Asn Thr Glu Pro Leu Val Ser Ser Asp

275                 280                 285

ttc aac cac acc acc gtc tcc tcc acc ttc gac gcc acc cag acc cgc      912
Phe Asn His Thr Thr Val Ser Ser Thr Phe Asp Ala Thr Gln Thr Arg
    290                 295                 300

gtc atc aag ggc gag gac ggc tcg gtg ctg gtc aag gtg ctg gcc tgg      960
Val Ile Lys Gly Glu Asp Gly Ser Val Leu Val Lys Val Leu Ala Trp
305                 310                 315                 320

tac gac aac gag tgg ggc tat tcc tgc cgc atg ctc gac gcc gcc cgc     1008
Tyr Asp Asn Glu Trp Gly Tyr Ser Cys Arg Met Leu Asp Ala Ala Arg
                325                 330                 335

gcc ttc gtc gcc gca aag taa                                         1029
Ala Phe Val Ala Ala Lys
            340

<210>  20
<211>  342
<212>  PRT
<213>  Thauera butanivorans

<400>  20

Met Ser Ile Lys Val Ala Ile Asn Gly Phe Gly Arg Ile Gly Arg Cys
1               5                   10                  15

Thr Leu Arg Ala Ile Tyr Glu Gln Gly Leu Gln Asn Glu Phe Glu Val
            20                  25                  30

Val Ala Ile Asn Ala Ser Gly Asp Leu Ala Thr Asn Ala His Leu Leu
        35                  40                  45

Lys Tyr Asp Thr Thr His Gly Arg Phe Ala Thr Ser Val Glu Thr Glu
    50                  55                  60

Gly Glu Asn Val Ile Ile Ile Asp Gly Lys Lys Ile Pro Phe Tyr Ser
65                  70                  75                  80

Thr Lys Asp Pro Lys Gly Val Asn Trp Gly Ser His Gly Val Asp Val
                85                  90                  95

Leu Leu Glu Cys Thr Gly Ala Tyr Thr Thr Lys Ala Lys Ala Gln Ala
            100                 105                 110

Leu Leu Asp Gln Gly Ala Arg Arg Val Leu Ile Ser Ala Pro Gly Gly
        115                 120                 125

Asp Asp Val Asp Thr Thr Ile Val Met Gly Val Asn Glu Asp Ala Leu
    130                 135                 140

Lys Ala Gly Met Thr Val Val Ser Asn Ala Ser Cys Thr Thr Asn Cys

145                 150                 155                 160

Leu Ala Pro Val Ala Lys Ile Leu Ala Asp Ser Val Gly Ile Lys Gln
                165                 170                 175

Gly Leu Met Thr Thr Ile His Ala Tyr Thr Asn Asp Gln Val Thr Val
            180                 185                 190

Asp Val Arg His Lys Asp Leu Arg Arg Ala Arg Ala Ala Ala Ala Asn
        195                 200                 205

Ile Ile Pro Thr Lys Thr Gly Ala Ala Lys Ala Val Gly Leu Val Leu
    210                 215                 220

Pro Gln Leu Ala Gly Lys Val Asp Gly Phe Ala Leu Arg Val Pro Thr
225                 230                 235                 240

Ile Asn Val Ser Leu Val Asp Leu Thr Phe Thr Ala Glu Arg Ala Thr
                245                 250                 255

Thr Lys Glu Glu Ile Asn Glu Leu Met Ser Ala Ala Ala Asn Gly Pro
            260                 265                 270

Leu Lys Gly Ile Leu Ala Val Asn Thr Glu Pro Leu Val Ser Ser Asp
        275                 280                 285

Phe Asn His Thr Thr Val Ser Ser Thr Phe Asp Ala Thr Gln Thr Arg
    290                 295                 300

Val Ile Lys Gly Glu Asp Gly Ser Val Leu Val Lys Val Leu Ala Trp
305                 310                 315                 320

Tyr Asp Asn Glu Trp Gly Tyr Ser Cys Arg Met Leu Asp Ala Ala Arg
                325                 330                 335

Ala Phe Val Ala Ala Lys
            340

<210>  21
<211>  1503
<212>  DNA
<213>  Thauera butanivorans

<220>
<221>  CDS
<222>  (1)..(1503)

<400>  21
atg gat gct cac acg agt gaa ctg ctg cac gag aac acc cgg cgt ttc        48

Met Asp Ala His Thr Ser Glu Leu Leu His Glu Asn Thr Arg Arg Phe
1               5                   10                  15

ctt gcc cag ccc aag cgc ctg ctg atc ggc ggg gcc tgg gtg gag gcc      96
Leu Ala Gln Pro Lys Arg Leu Leu Ile Gly Gly Ala Trp Val Glu Ala
            20                  25                  30

agc gac ggc gcc acg ctg gac gtg ttc gac ccg gcc acc ggc cag tcc      144
Ser Asp Gly Ala Thr Leu Asp Val Phe Asp Pro Ala Thr Gly Gln Ser
        35                  40                  45

ttc gcc aag gtg ccg gcc ggc ggt cag gcc gac atc gac cgt gcg gtg      192
Phe Ala Lys Val Pro Ala Gly Gly Gln Ala Asp Ile Asp Arg Ala Val
    50                  55                  60

gcc gcc gcg cgc cag gcc ttc gaa ggc ggc gac tgg gcg gcg atg cgc      240
Ala Ala Ala Arg Gln Ala Phe Glu Gly Gly Asp Trp Ala Ala Met Arg
65                  70                  75                  80

ccg gtc gat cgc gag cgc ctg ctg atg aag ctg gcc gac ctg gtc gaa      288
Pro Val Asp Arg Glu Arg Leu Leu Met Lys Leu Ala Asp Leu Val Glu
                85                  90                  95

gcc aac gcc cag gaa ctg gcc gaa ctg gaa gcg ctg gac aac ggc aag      336
Ala Asn Ala Gln Glu Leu Ala Glu Leu Glu Ala Leu Asp Asn Gly Lys
            100                 105                 110

ccg gtg acg gtg gcg cgc ttc gcc gac gtc gcg ctg gtg gtc gac tcg      384
Pro Val Thr Val Ala Arg Phe Ala Asp Val Ala Leu Val Val Asp Ser
        115                 120                 125

ctg cgc tac atg gcc ggc tgg gcg acc aag ctc ggc ggc gag acc atg      432
Leu Arg Tyr Met Ala Gly Trp Ala Thr Lys Leu Gly Gly Glu Thr Met
    130                 135                 140

cag ccc tcg gtg ccg atc atc cgc gag cgc gaa ttc tgg ggc ttc acc      480
Gln Pro Ser Val Pro Ile Ile Arg Glu Arg Glu Phe Trp Gly Phe Thr
145                 150                 155                 160

cgg cgc gag ccg gtg ggc gtg gtc ggc gcc atc atc ccg tgg aac ttc      528
Arg Arg Glu Pro Val Gly Val Val Gly Ala Ile Ile Pro Trp Asn Phe
                165                 170                 175

ccg ctg ctg atg gcg gcg tgg aag atg ggg ccg gcg ctg gcc agc ggc      576
Pro Leu Leu Met Ala Ala Trp Lys Met Gly Pro Ala Leu Ala Ser Gly
            180                 185                 190

tgc acg atg gtg ctc aag ccg gcc gag gaa acg ccg ctg agc gcg ctg      624
Cys Thr Met Val Leu Lys Pro Ala Glu Glu Thr Pro Leu Ser Ala Leu
        195                 200                 205

cgc ctg gcc gaa ctg gtg cag gaa gcc ggc tac ccg gcc ggc gtg ttc      672
Arg Leu Ala Glu Leu Val Gln Glu Ala Gly Tyr Pro Ala Gly Val Phe
    210                 215                 220

aac gtc gtc acc ggc ctg ggc cat gtc gcc ggc gcg gcg ctg tcc tcg      720
Asn Val Val Thr Gly Leu Gly His Val Ala Gly Ala Ala Leu Ser Ser
225                 230                 235                 240

cac ccg ggc atc gac aag ctc gcc ttc acc ggc tcg acc gag atc ggc      768
His Pro Gly Ile Asp Lys Leu Ala Phe Thr Gly Ser Thr Glu Ile Gly
                245                 250                 255

aag ctg gtg ggc aag gcc gcg ctc gac aac atg acg cgc atc tcg ctg 816
Lys Leu Val Gly Lys Ala Ala Leu Asp Asn Met Thr Arg Ile Ser Leu
            260                 265                 270

gag ctg ggc ggc aag agc ccg atg atc gtg ctc gac gac gcc gac gtc 864
Glu Leu Gly Gly Lys Ser Pro Met Ile Val Leu Asp Asp Ala Asp Val
        275                 280                 285

gac gcg gtg gcc gcc ggt gcc gca cac ggc atc ttc ttc aac tcc ggc 912
Asp Ala Val Ala Ala Gly Ala Ala His Gly Ile Phe Phe Asn Ser Gly
    290                 295                 300

cag gtc tgc acc gcc ggc tcg cgc ctg ttc gtg cac aag aag aag ttc 960
Gln Val Cys Thr Ala Gly Ser Arg Leu Phe Val His Lys Lys Lys Phe
305                 310                 315                 320

gac cag gtg gtc gaa ggc ctg tcc aac att gcc gcc ggc atg aag gtc 1008
Asp Gln Val Val Glu Gly Leu Ser Asn Ile Ala Ala Gly Met Lys Val
                325                 330                 335

ggc gcc ggc ctc gac ccc gac acc cag ctc ggc ccg ctg gtc tcg gcg 1056
Gly Ala Gly Leu Asp Pro Asp Thr Gln Leu Gly Pro Leu Val Ser Ala
            340                 345                 350

gtg cag cag gaa cgg gtg atg ggc tac atc cgc agc ggc ttc gaa gaa 1104
Val Gln Gln Glu Arg Val Met Gly Tyr Ile Arg Ser Gly Phe Glu Glu
        355                 360                 365

ggc gcg cgt gcc gcg gtc ggc ggc gaa gcg ggc gaa ggc ggc ggc tac 1152
Gly Ala Arg Ala Ala Val Gly Gly Glu Ala Gly Glu Gly Gly Gly Tyr
    370                 375                 380

ttc gtc aag ccg acg gtg ctg gtc gat gcg cgc gac gac atg cgg gtg 1200
Phe Val Lys Pro Thr Val Leu Val Asp Ala Arg Asp Asp Met Arg Val
385                 390                 395                 400

gtg cgc gag gaa atc ttc ggc ccg gtg gtg gtg gcg atg ccg tac gac 1248
Val Arg Glu Glu Ile Phe Gly Pro Val Val Val Ala Met Pro Tyr Asp
                405                 410                 415

gat ctg gac gag gtc gcg cgc cgc gcc aac gac acg ccc tac ggc ctg 1296
Asp Leu Asp Glu Val Ala Arg Arg Ala Asn Asp Thr Pro Tyr Gly Leu
            420                 425                 430

gcg gcg agc atc tgg tcg aac gac ctg acg cgg gtg cat cgg ctg att 1344
Ala Ala Ser Ile Trp Ser Asn Asp Leu Thr Arg Val His Arg Leu Ile
        435                 440                 445

ccg cgg atc aag gcc ggc acg gtg tgg gtg aac tgc cac acg gtg ctg 1392
Pro Arg Ile Lys Ala Gly Thr Val Trp Val Asn Cys His Thr Val Leu
    450                 455                 460

gac aac tcg atg ccg ttc ggc ggc ttc aag cag tcg ggc atc ggc cgc 1440
Asp Asn Ser Met Pro Phe Gly Gly Phe Lys Gln Ser Gly Ile Gly Arg
465                 470                 475                 480

gag atg ggg cgc gcg gta ttc gac cag tac acc gag atc aag tcg gtg 1488
Glu Met Gly Arg Ala Val Phe Asp Gln Tyr Thr Glu Ile Lys Ser Val
                485                 490                 495

ctg atg gcc ttg tga 1503
Leu Met Ala Leu
            500

<210>  22
<211>  500
<212>  PRT
<213>  Thauera butanivorans

<400>  22

Met Asp Ala His Thr Ser Glu Leu Leu His Glu Asn Thr Arg Arg Phe
1               5                   10                  15

Leu Ala Gln Pro Lys Arg Leu Leu Ile Gly Gly Ala Trp Val Glu Ala
            20                  25                  30

Ser Asp Gly Ala Thr Leu Asp Val Phe Asp Pro Ala Thr Gly Gln Ser
        35                  40                  45

Phe Ala Lys Val Pro Ala Gly Gly Gln Ala Asp Ile Asp Arg Ala Val
    50                  55                  60

Ala Ala Ala Arg Gln Ala Phe Glu Gly Gly Asp Trp Ala Ala Met Arg
65                  70                  75                  80

Pro Val Asp Arg Glu Arg Leu Leu Met Lys Leu Ala Asp Leu Val Glu
                85                  90                  95

Ala Asn Ala Gln Glu Leu Ala Glu Leu Glu Ala Leu Asp Asn Gly Lys
            100                 105                 110

Pro Val Thr Val Ala Arg Phe Ala Asp Val Ala Leu Val Val Asp Ser
        115                 120                 125

Leu Arg Tyr Met Ala Gly Trp Ala Thr Lys Leu Gly Gly Glu Thr Met
    130                 135                 140

Gln Pro Ser Val Pro Ile Ile Arg Glu Arg Glu Phe Trp Gly Phe Thr
145                 150                 155                 160

Arg Arg Glu Pro Val Gly Val Val Gly Ala Ile Ile Pro Trp Asn Phe
                165                 170                 175

Pro Leu Leu Met Ala Ala Trp Lys Met Gly Pro Ala Leu Ala Ser Gly
            180                 185                 190

Cys Thr Met Val Leu Lys Pro Ala Glu Glu Thr Pro Leu Ser Ala Leu
        195                 200                 205

Arg Leu Ala Glu Leu Val Gln Glu Ala Gly Tyr Pro Ala Gly Val Phe
    210                 215                 220

Asn Val Val Thr Gly Leu Gly His Val Ala Gly Ala Ala Leu Ser Ser
225                 230                 235                 240

His Pro Gly Ile Asp Lys Leu Ala Phe Thr Gly Ser Thr Glu Ile Gly
                245                 250                 255

Lys Leu Val Gly Lys Ala Ala Leu Asp Asn Met Thr Arg Ile Ser Leu
            260                 265                 270

Glu Leu Gly Gly Lys Ser Pro Met Ile Val Leu Asp Asp Ala Asp Val
        275                 280                 285

Asp Ala Val Ala Ala Gly Ala Ala His Gly Ile Phe Phe Asn Ser Gly
    290                 295                 300

Gln Val Cys Thr Ala Gly Ser Arg Leu Phe Val His Lys Lys Lys Phe
305                 310                 315                 320

Asp Gln Val Val Glu Gly Leu Ser Asn Ile Ala Ala Gly Met Lys Val
                325                 330                 335

Gly Ala Gly Leu Asp Pro Asp Thr Gln Leu Gly Pro Leu Val Ser Ala
            340                 345                 350

Val Gln Gln Glu Arg Val Met Gly Tyr Ile Arg Ser Gly Phe Glu Glu
        355                 360                 365

Gly Ala Arg Ala Ala Val Gly Gly Glu Ala Gly Glu Gly Gly Gly Tyr
    370                 375                 380

Phe Val Lys Pro Thr Val Leu Val Asp Ala Arg Asp Asp Met Arg Val
385                 390                 395                 400

Val Arg Glu Glu Ile Phe Gly Pro Val Val Val Ala Met Pro Tyr Asp
                405                 410                 415

Asp Leu Asp Glu Val Ala Arg Arg Ala Asn Asp Thr Pro Tyr Gly Leu
            420                 425                 430

Ala Ala Ser Ile Trp Ser Asn Asp Leu Thr Arg Val His Arg Leu Ile
        435                 440                 445

Pro Arg Ile Lys Ala Gly Thr Val Trp Val Asn Cys His Thr Val Leu
    450                 455                 460

Asp Asn Ser Met Pro Phe Gly Gly Phe Lys Gln Ser Gly Ile Gly Arg

465                 470                 475                 480

Glu Met Gly Arg Ala Val Phe Asp Gln Tyr Thr Glu Ile Lys Ser Val
                485                 490                 495

Leu Met Ala Leu
            500

<210>  23
<211>  1668
<212>  DNA
<213>  Thauera butanivorans

<220>
<221>  CDS
<222>  (1)..(1668)

<400>  23
atg agc cac ccc ctt ctt gaa aag cac cgc ggc atc ctc gac aac gcg      48
Met Ser His Pro Leu Leu Glu Lys His Arg Gly Ile Leu Asp Asn Ala
1               5                   10                  15

ctg aac acg atc gcg acg cgc ggc tac tgg acg cct ttc ccg gaa atg      96
Leu Asn Thr Ile Ala Thr Arg Gly Tyr Trp Thr Pro Phe Pro Glu Met
            20                  25                  30

ccc agc ccc aag ctc tac ggc gaa acc gcg ccc gag gac ggc aag cgt      144
Pro Ser Pro Lys Leu Tyr Gly Glu Thr Ala Pro Glu Asp Gly Lys Arg
        35                  40                  45

gcc ttc gag gcc cac ctg ggc aag cac ttc gag ctg gac cag ccc ggc      192
Ala Phe Glu Ala His Leu Gly Lys His Phe Glu Leu Asp Gln Pro Gly
    50                  55                  60

cag acc ggc tgg ctc gac aag gaa acc tcg ccc tac ggc gcc acg ctc      240
Gln Thr Gly Trp Leu Asp Lys Glu Thr Ser Pro Tyr Gly Ala Thr Leu
65                  70                  75                  80

ggc gtc agc tac ccg gtg tgc gag ccc gag gca ctg atc gcc gcc ggc      288
Gly Val Ser Tyr Pro Val Cys Glu Pro Glu Ala Leu Ile Ala Ala Gly
                85                  90                  95

acc gaa gcc atg aag agc tgg cag gcc gtc ggc gcc gac ggc cgc acc      336
Thr Glu Ala Met Lys Ser Trp Gln Ala Val Gly Ala Asp Gly Arg Thr
            100                 105                 110

ggc atc tgc ctc gaa atc ctc acc cgc ctc aac gcc cag agc ttc gag      384
Gly Ile Cys Leu Glu Ile Leu Thr Arg Leu Asn Ala Gln Ser Phe Glu
        115                 120                 125

atc gcc cat gcg gtg atg atg acc acc ggc cag ggc tgg atg atg gcc      432
Ile Ala His Ala Val Met Met Thr Thr Gly Gln Gly Trp Met Met Ala
    130                 135                 140

ttc cag gcc ggc ggc ccg cac gcg cag gat cgc ggc ctg gag gcc gtc      480
Phe Gln Ala Gly Gly Pro His Ala Gln Asp Arg Gly Leu Glu Ala Val
145                 150                 155                 160

gcc tac gcc tgg cgc gag cag agc ttc gtg ccg gcc gaa acg agc tgg      528

Ala Tyr Ala Trp Arg Glu Gln Ser Phe Val Pro Ala Glu Thr Ser Trp
                165                 170                 175

gag aag ccg cag ggc aag aac ccg ccg ctg gtc atg aag aag cac tac 576
Glu Lys Pro Gln Gly Lys Asn Pro Pro Leu Val Met Lys Lys His Tyr
            180                 185                 190

cag atc gtc ggc cgc ggc gtc ggc ctg gtg gtc ggc tgc ggc acc ttc 624
Gln Ile Val Gly Arg Gly Val Gly Leu Val Val Gly Cys Gly Thr Phe
        195                 200                 205

ccg acc tgg aac acc tac ccc ggc ctg ttc gcc gcg ctc gcc acc ggc 672
Pro Thr Trp Asn Thr Tyr Pro Gly Leu Phe Ala Ala Leu Ala Thr Gly
    210                 215                 220

aac gcc gtc atc gtc aag ccg cac agc aac gcc gtc ctg ccg gcg gcg 720
Asn Ala Val Ile Val Lys Pro His Ser Asn Ala Val Leu Pro Ala Ala
225                 230                 235                 240

atc acc gtg cgc acg atc cgc gcc gtg ctc gcc gag aac ggc atc gac 768
Ile Thr Val Arg Thr Ile Arg Ala Val Leu Ala Glu Asn Gly Ile Asp
                245                 250                 255

ccc gac ctc gtc acg ctg tgc gtc gcg ccc cag cgc gcc gcc acc cag 816
Pro Asp Leu Val Thr Leu Cys Val Ala Pro Gln Arg Ala Ala Thr Gln
            260                 265                 270

gcg ctg gcc acc cat ccg gcg gtg aaa tcg atc gac ttc acc ggc agc 864
Ala Leu Ala Thr His Pro Ala Val Lys Ser Ile Asp Phe Thr Gly Ser
        275                 280                 285

aac gtc ttc ggc cag tgg ctg atc gac aac tgc cgc cag gcc caa gtc 912
Asn Val Phe Gly Gln Trp Leu Ile Asp Asn Cys Arg Gln Ala Gln Val
    290                 295                 300

tat gcc gag ctg gcc ggc gtc aac aac atc gtc atc gac tcc acc agc 960
Tyr Ala Glu Leu Ala Gly Val Asn Asn Ile Val Ile Asp Ser Thr Ser
305                 310                 315                 320

gcc tac aag gcg atg ctg ggc aac ctc gcc ttc acg ctg tcg ctg tac 1008
Ala Tyr Lys Ala Met Leu Gly Asn Leu Ala Phe Thr Leu Ser Leu Tyr
                325                 330                 335

tcc ggc cag atg tgc acc act tcg cag gcg atc ttc gtg ccg gcc ggc 1056
Ser Gly Gln Met Cys Thr Thr Ser Gln Ala Ile Phe Val Pro Ala Gly
            340                 345                 350

ggc atc gac acc gac gaa ggc cac aag agc tac gac gag gtc tgc gcc 1104
Gly Ile Asp Thr Asp Glu Gly His Lys Ser Tyr Asp Glu Val Cys Ala
        355                 360                 365

gac ctg gcg aag tcg atc gag cgc ttc ctg tcc aag ccc gaa gtc gcc 1152
Asp Leu Ala Lys Ser Ile Glu Arg Phe Leu Ser Lys Pro Glu Val Ala
    370                 375                 380

cat gcc gtg ctc ggc gcg atc cag tcc gcc gac acc gcc gaa cgc atc 1200
His Ala Val Leu Gly Ala Ile Gln Ser Ala Asp Thr Ala Glu Arg Ile
385                 390                 395                 400

gac atc gcc aac gcc ggc gag ctc ggc cgc gtc gtg ctg gcc tcg cag 1248
Asp Ile Ala Asn Ala Gly Glu Leu Gly Arg Val Val Leu Ala Ser Gln
                405                 410                 415

cgc ctc gac aac ccc gac ttc ccc gcc gcc acg gtg cgc acc ccg gtg 1296
Arg Leu Asp Asn Pro Asp Phe Pro Ala Ala Thr Val Arg Thr Pro Val
            420                 425                 430

ctg ctg gcc tgc gac gcc agc gac gag aaa gcc tac atg gaa gag cgc 1344
Leu Leu Ala Cys Asp Ala Ser Asp Glu Lys Ala Tyr Met Glu Glu Arg
        435                 440                 445

ttc ggc ccg atc agc ttc atc gtc aag gtc gcc gac acc gcc gcc gcg 1392
Phe Gly Pro Ile Ser Phe Ile Val Lys Val Ala Asp Thr Ala Ala Ala
    450                 455                 460

atc gcc ctg tcc gag cgc gtc gtc agc acc cac ggc gcc ctg acc gtc 1440
Ile Ala Leu Ser Glu Arg Val Val Ser Thr His Gly Ala Leu Thr Val
465                 470                 475                 480

ggc ctc tat tcg acg aag cag gag gtc atc gac gcg atg acc gaa gcc 1488
Gly Leu Tyr Ser Thr Lys Gln Glu Val Ile Asp Ala Met Thr Glu Ala
                485                 490                 495

acc tgg cgc agc aag gtc gcg ctg tcg atc aac ctc acc ggc ggc gtg 1536
Thr Trp Arg Ser Lys Val Ala Leu Ser Ile Asn Leu Thr Gly Gly Val
            500                 505                 510

ttc gtg aac cag tcg gcg gcc ttc tcc gac tac cac ggc acc ggc ggc 1584
Phe Val Asn Gln Ser Ala Ala Phe Ser Asp Tyr His Gly Thr Gly Gly
        515                 520                 525

aac ccg gcg gcc aat gcc tcg tat gcg gat tcg gcc ttc gtc gcc aac 1632
Asn Pro Ala Ala Asn Ala Ser Tyr Ala Asp Ser Ala Phe Val Ala Asn
    530                 535                 540

cgc ttc cgt gtc gtc cag cgt cgt tat cac gcc tga 1668
Arg Phe Arg Val Val Gln Arg Arg Tyr His Ala
545                 550                 555

<210> 24
<211> 555
<212> PRT
<213> Thauera butanivorans

<400> 24

Met Ser His Pro Leu Leu Glu Lys His Arg Gly Ile Leu Asp Asn Ala
1               5                   10                  15

Leu Asn Thr Ile Ala Thr Arg Gly Tyr Trp Thr Pro Phe Pro Glu Met
            20                  25                  30

Pro Ser Pro Lys Leu Tyr Gly Glu Thr Ala Pro Glu Asp Gly Lys Arg
        35                  40                  45

Ala Phe Glu Ala His Leu Gly Lys His Phe Glu Leu Asp Gln Pro Gly
    50                  55                  60

Gln Thr Gly Trp Leu Asp Lys Glu Thr Ser Pro Tyr Gly Ala Thr Leu
65                  70                  75                  80

Gly Val Ser Tyr Pro Val Cys Glu Pro Glu Ala Leu Ile Ala Ala Gly
                85                  90                  95

Thr Glu Ala Met Lys Ser Trp Gln Ala Val Gly Ala Asp Gly Arg Thr
            100                 105                 110

Gly Ile Cys Leu Glu Ile Leu Thr Arg Leu Asn Ala Gln Ser Phe Glu
        115                 120                 125

Ile Ala His Ala Val Met Met Thr Thr Gly Gln Gly Trp Met Met Ala
    130                 135                 140

Phe Gln Ala Gly Gly Pro His Ala Gln Asp Arg Gly Leu Glu Ala Val
145                 150                 155                 160

Ala Tyr Ala Trp Arg Glu Gln Ser Phe Val Pro Ala Glu Thr Ser Trp
                165                 170                 175

Glu Lys Pro Gln Gly Lys Asn Pro Pro Leu Val Met Lys Lys His Tyr
            180                 185                 190

Gln Ile Val Gly Arg Gly Val Gly Leu Val Val Gly Cys Gly Thr Phe
        195                 200                 205

Pro Thr Trp Asn Thr Tyr Pro Gly Leu Phe Ala Ala Leu Ala Thr Gly
    210                 215                 220

Asn Ala Val Ile Val Lys Pro His Ser Asn Ala Val Leu Pro Ala Ala
225                 230                 235                 240

Ile Thr Val Arg Thr Ile Arg Ala Val Leu Ala Glu Asn Gly Ile Asp
                245                 250                 255

Pro Asp Leu Val Thr Leu Cys Val Ala Pro Gln Arg Ala Ala Thr Gln
            260                 265                 270

Ala Leu Ala Thr His Pro Ala Val Lys Ser Ile Asp Phe Thr Gly Ser
        275                 280                 285

Asn Val Phe Gly Gln Trp Leu Ile Asp Asn Cys Arg Gln Ala Gln Val
    290                 295                 300

Tyr Ala Glu Leu Ala Gly Val Asn Asn Ile Val Ile Asp Ser Thr Ser
305                 310                 315                 320

Ala Tyr Lys Ala Met Leu Gly Asn Leu Ala Phe Thr Leu Ser Leu Tyr
                325                 330                 335

Ser Gly Gln Met Cys Thr Thr Ser Gln Ala Ile Phe Val Pro Ala Gly
            340                 345                 350

Gly Ile Asp Thr Asp Glu Gly His Lys Ser Tyr Asp Glu Val Cys Ala
        355                 360                 365

Asp Leu Ala Lys Ser Ile Glu Arg Phe Leu Ser Lys Pro Glu Val Ala
    370                 375                 380

His Ala Val Leu Gly Ala Ile Gln Ser Ala Asp Thr Ala Glu Arg Ile
385                 390                 395                 400

Asp Ile Ala Asn Ala Gly Glu Leu Gly Arg Val Val Leu Ala Ser Gln
                405                 410                 415

Arg Leu Asp Asn Pro Asp Phe Pro Ala Ala Thr Val Arg Thr Pro Val
            420                 425                 430

Leu Leu Ala Cys Asp Ala Ser Asp Glu Lys Ala Tyr Met Glu Glu Arg
        435                 440                 445

Phe Gly Pro Ile Ser Phe Ile Val Lys Val Ala Asp Thr Ala Ala Ala
    450                 455                 460

Ile Ala Leu Ser Glu Arg Val Val Ser Thr His Gly Ala Leu Thr Val
465                 470                 475                 480

Gly Leu Tyr Ser Thr Lys Gln Glu Val Ile Asp Ala Met Thr Glu Ala
                485                 490                 495

Thr Trp Arg Ser Lys Val Ala Leu Ser Ile Asn Leu Thr Gly Gly Val
            500                 505                 510

Phe Val Asn Gln Ser Ala Ala Phe Ser Asp Tyr His Gly Thr Gly Gly
        515                 520                 525

Asn Pro Ala Ala Asn Ala Ser Tyr Ala Asp Ser Ala Phe Val Ala Asn
    530                 535                 540

Arg Phe Arg Val Val Gln Arg Arg Tyr His Ala
545                 550                 555

<210>  25
<211>  1767
<212>  DNA
<213>  Thauera butanivorans

<220>
<221>  CDS
<222>  (1)..(1767)

<400>  25
atg gat ggg aac gtg gcg ctg gcg aag tgc atc gca ttg gaa cag gtg      48
Met Asp Gly Asn Val Ala Leu Ala Lys Cys Ile Ala Leu Glu Gln Val
1               5                   10                  15

tgc ggc aat gga aca ccc ggc gag gcc ggc ttt gag cgg ctc cgg atg      96
Cys Gly Asn Gly Thr Pro Gly Glu Ala Gly Phe Glu Arg Leu Arg Met
            20                  25                  30

ggc tgc tca gcc tgg gct gtt gcc agt tgc ctg aat gat aag aag aaa      144
Gly Cys Ser Ala Trp Ala Val Ala Ser Cys Leu Asn Asp Lys Lys Lys
        35                  40                  45

ttg aga atc tct gcg cag gcc tgc ggt tct ggc acg ctg cct gca gag      192
Leu Arg Ile Ser Ala Gln Ala Cys Gly Ser Gly Thr Leu Pro Ala Glu
    50                  55                  60

gag acg tgt aac gca cac ggc gtt cgt aac cac aag aga gaa gac agc      240
Glu Thr Cys Asn Ala His Gly Val Arg Asn His Lys Arg Glu Asp Ser
65                  70                  75                  80

gag atg atc tac gca atg ccc ggg caa tcc ggc gcc ccg gtc cag cac      288
Glu Met Ile Tyr Ala Met Pro Gly Gln Ser Gly Ala Pro Val Gln His
                85                  90                  95

aag gcg cgg tac gac aat ttc atc ggc ggc cgt tgg gta gcg cca gtc      336
Lys Ala Arg Tyr Asp Asn Phe Ile Gly Gly Arg Trp Val Ala Pro Val
            100                 105                 110

aag ggc cag tac ttc gac gtg gtg acg ccg atc agc ggc cag gtc tat      384
Lys Gly Gln Tyr Phe Asp Val Val Thr Pro Ile Ser Gly Gln Val Tyr
        115                 120                 125

acg cag gcg gcg cgc tcg acg gcc gag gac atc gag ctg gcg ctg gac      432
Thr Gln Ala Ala Arg Ser Thr Ala Glu Asp Ile Glu Leu Ala Leu Asp
    130                 135                 140

gcg gcg cac gcg gcg ttt ccg gcc tgg agc aag acg ccg gcg gcc gag      480
Ala Ala His Ala Ala Phe Pro Ala Trp Ser Lys Thr Pro Ala Ala Glu
145                 150                 155                 160

cgg gcc aac att ctg ctc agg atc gcc gag cgc atc gaa cag aac ctg      528
Arg Ala Asn Ile Leu Leu Arg Ile Ala Glu Arg Ile Glu Gln Asn Leu
                165                 170                 175

gaa cgg ctg gcc tac gcc gag acg gtc gac aac ggc aag gcg atc cgc      576
Glu Arg Leu Ala Tyr Ala Glu Thr Val Asp Asn Gly Lys Ala Ile Arg
            180                 185                 190

gag acg ctc aac gcc gac atc ccg ctc acc gtc gat cac ttc cgc tac      624
Glu Thr Leu Asn Ala Asp Ile Pro Leu Thr Val Asp His Phe Arg Tyr
        195                 200                 205

ttc gcc ggc tgc ctg cgc gcg cag gaa ggc agc atc agc gag atc gac      672
Phe Ala Gly Cys Leu Arg Ala Gln Glu Gly Ser Ile Ser Glu Ile Asp
    210                 215                 220

gag aac acc gtc gcc tac cac ctg cac gaa ccg ctg ggc gtg gtc ggg      720

Glu Asn Thr Val Ala Tyr His Leu His Glu Pro Leu Gly Val Val Gly
225                 230                 235                 240

cag atc atc ccg tgg aac ttc ccg atc ctg atg gcg gcc tgg aag ctc      768
Gln Ile Ile Pro Trp Asn Phe Pro Ile Leu Met Ala Ala Trp Lys Leu
                245                 250                 255

gcg ccg gcg ctg ggc gcg ggc aac tgc gtg gtg ttg aag ccc gcc gaa      816
Ala Pro Ala Leu Gly Ala Gly Asn Cys Val Val Leu Lys Pro Ala Glu
            260                 265                 270

tcg acg ccg gtc tcg atc atg gtg ctg gtc gaa ctg atc gcc gac ctg      864
Ser Thr Pro Val Ser Ile Met Val Leu Val Glu Leu Ile Ala Asp Leu
        275                 280                 285

ctg ccg ccg ggc gtg ctc aac atc gtc aac ggc ttc ggc cgc gaa gcc      912
Leu Pro Pro Gly Val Leu Asn Ile Val Asn Gly Phe Gly Arg Glu Ala
    290                 295                 300

ggc atg ccg ctg gcc acc agc aag cgc atc gcc aag atc gcc ttc acc      960
Gly Met Pro Leu Ala Thr Ser Lys Arg Ile Ala Lys Ile Ala Phe Thr
305                 310                 315                 320

ggc tcg acc tcg acc ggg cgg gtg atc gcc cag gcc gcg gcc aac aac     1008
Gly Ser Thr Ser Thr Gly Arg Val Ile Ala Gln Ala Ala Ala Asn Asn
                325                 330                 335

ctg atc ccg gcc acg ctg gag ctg ggc ggc aag tcg ccc aac atc ttc     1056
Leu Ile Pro Ala Thr Leu Glu Leu Gly Gly Lys Ser Pro Asn Ile Phe
            340                 345                 350

ttc gcc gac gtc atg gac cgc gac gac gcc ttc ctc gac aag gcc atc     1104
Phe Ala Asp Val Met Asp Arg Asp Asp Ala Phe Leu Asp Lys Ala Ile
        355                 360                 365

gaa ggc atg gtg ctg ttc gcc ttc aac cag ggc gaa gtg tgc acc tgc     1152
Glu Gly Met Val Leu Phe Ala Phe Asn Gln Gly Glu Val Cys Thr Cys
    370                 375                 380

ccg agc cgg gcg ctg atc cag gaa agc atc tac gag cgc ttc atc gag     1200
Pro Ser Arg Ala Leu Ile Gln Glu Ser Ile Tyr Glu Arg Phe Ile Glu
385                 390                 395                 400

cgc gtg ctc gag cgc gtg gcg gcg atc aag cac ggc cac ccg ctc gac     1248
Arg Val Leu Glu Arg Val Ala Ala Ile Lys His Gly His Pro Leu Asp
                405                 410                 415

acc gag tcg atg atg ggc gcg cag gcc tcg cgc gag caa atg agc aag     1296
Thr Glu Ser Met Met Gly Ala Gln Ala Ser Arg Glu Gln Met Ser Lys
            420                 425                 430

atc cag tcc tac ctg gcg ctg ggc aag gaa gaa ggg ccg aag tgc gtg     1344
Ile Gln Ser Tyr Leu Ala Leu Gly Lys Glu Glu Gly Pro Lys Cys Val
        435                 440                 445

ctg atc ggc ggc gac aag gcg aat ctg ggc ggc gaa ctg agc gag ggc     1392
Leu Ile Gly Gly Asp Lys Ala Asn Leu Gly Gly Glu Leu Ser Glu Gly
    450                 455                 460

tac tac atc cag ccc acg ctg ttc aag ggc cac aac aag atg cgc atc     1440
Tyr Tyr Ile Gln Pro Thr Leu Phe Lys Gly His Asn Lys Met Arg Ile
465                 470                 475                 480

ttc cag gaa gag atc ttc ggc ccg gtg ctg gcg gtg acc acc ttc aag 1488
Phe Gln Glu Glu Ile Phe Gly Pro Val Leu Ala Val Thr Thr Phe Lys
                485                 490                 495

gac gaa gcc gag gcg ctg gcg atc gcc aac gac acg ctg tac ggg ctg 1536
Asp Glu Ala Glu Ala Leu Ala Ile Ala Asn Asp Thr Leu Tyr Gly Leu
            500                 505                 510

ggt gcg ggg gtg tgg agc cgc aac ggc aac gtg gcc tac cgc atg ggc 1584
Gly Ala Gly Val Trp Ser Arg Asn Gly Asn Val Ala Tyr Arg Met Gly
        515                 520                 525

cgg gcg atc cag gcc ggg cgg gtg tgg acc aac tgc tac cac gcc tat 1632
Arg Ala Ile Gln Ala Gly Arg Val Trp Thr Asn Cys Tyr His Ala Tyr
    530                 535                 540

ccg gcg cat gcg gcc ttc ggc ggc tac aag gag tcg ggc atc ggg cgc 1680
Pro Ala His Ala Ala Phe Gly Gly Tyr Lys Glu Ser Gly Ile Gly Arg
545                 550                 555                 560

gag acc cac aag atg atg ctc gac cac tac cag cag acc aag aac ctg 1728
Glu Thr His Lys Met Met Leu Asp His Tyr Gln Gln Thr Lys Asn Leu
                565                 570                 575

ctc gtc agc tac agc gag aac aag ctc ggg ttc ttc tga 1767
Leu Val Ser Tyr Ser Glu Asn Lys Leu Gly Phe Phe
            580                 585

<210> 26
<211> 588
<212> PRT
<213> Thauera butanivorans

<400> 26

Met Asp Gly Asn Val Ala Leu Ala Lys Cys Ile Ala Leu Glu Gln Val
1               5                   10                  15

Cys Gly Asn Gly Thr Pro Gly Glu Ala Gly Phe Glu Arg Leu Arg Met
            20                  25                  30

Gly Cys Ser Ala Trp Ala Val Ala Ser Cys Leu Asn Asp Lys Lys Lys
        35                  40                  45

Leu Arg Ile Ser Ala Gln Ala Cys Gly Ser Gly Thr Leu Pro Ala Glu
    50                  55                  60

Glu Thr Cys Asn Ala His Gly Val Arg Asn His Lys Arg Glu Asp Ser
65                  70                  75                  80

Glu Met Ile Tyr Ala Met Pro Gly Gln Ser Gly Ala Pro Val Gln His
                85                  90                  95

Lys Ala Arg Tyr Asp Asn Phe Ile Gly Gly Arg Trp Val Ala Pro Val
            100                 105                 110

Lys Gly Gln Tyr Phe Asp Val Val Thr Pro Ile Ser Gly Gln Val Tyr
        115                 120                 125

Thr Gln Ala Ala Arg Ser Thr Ala Glu Asp Ile Glu Leu Ala Leu Asp
    130                 135                 140

Ala Ala His Ala Ala Phe Pro Ala Trp Ser Lys Thr Pro Ala Ala Glu
145                 150                 155                 160

Arg Ala Asn Ile Leu Leu Arg Ile Ala Glu Arg Ile Glu Gln Asn Leu
                165                 170                 175

Glu Arg Leu Ala Tyr Ala Glu Thr Val Asp Asn Gly Lys Ala Ile Arg
            180                 185                 190

Glu Thr Leu Asn Ala Asp Ile Pro Leu Thr Val Asp His Phe Arg Tyr
        195                 200                 205

Phe Ala Gly Cys Leu Arg Ala Gln Glu Gly Ser Ile Ser Glu Ile Asp
    210                 215                 220

Glu Asn Thr Val Ala Tyr His Leu His Glu Pro Leu Gly Val Val Gly
225                 230                 235                 240

Gln Ile Ile Pro Trp Asn Phe Pro Ile Leu Met Ala Ala Trp Lys Leu
                245                 250                 255

Ala Pro Ala Leu Gly Ala Gly Asn Cys Val Val Leu Lys Pro Ala Glu
            260                 265                 270

Ser Thr Pro Val Ser Ile Met Val Leu Val Glu Leu Ile Ala Asp Leu
        275                 280                 285

Leu Pro Pro Gly Val Leu Asn Ile Val Asn Gly Phe Gly Arg Glu Ala
    290                 295                 300

Gly Met Pro Leu Ala Thr Ser Lys Arg Ile Ala Lys Ile Ala Phe Thr
305                 310                 315                 320

Gly Ser Thr Ser Thr Gly Arg Val Ile Ala Gln Ala Ala Ala Asn Asn
                325                 330                 335

Leu Ile Pro Ala Thr Leu Glu Leu Gly Gly Lys Ser Pro Asn Ile Phe
            340                 345                 350

Phe Ala Asp Val Met Asp Arg Asp Asp Ala Phe Leu Asp Lys Ala Ile
        355                 360                 365

Glu Gly Met Val Leu Phe Ala Phe Asn Gln Gly Glu Val Cys Thr Cys
    370                 375                 380

Pro Ser Arg Ala Leu Ile Gln Glu Ser Ile Tyr Glu Arg Phe Ile Glu
385                 390                 395                 400

Arg Val Leu Glu Arg Val Ala Ala Ile Lys His Gly His Pro Leu Asp
                405                 410                 415

Thr Glu Ser Met Met Gly Ala Gln Ala Ser Arg Glu Gln Met Ser Lys
            420                 425                 430

Ile Gln Ser Tyr Leu Ala Leu Gly Lys Glu Glu Gly Pro Lys Cys Val
        435                 440                 445

Leu Ile Gly Gly Asp Lys Ala Asn Leu Gly Gly Glu Leu Ser Glu Gly
    450                 455                 460

Tyr Tyr Ile Gln Pro Thr Leu Phe Lys Gly His Asn Lys Met Arg Ile
465                 470                 475                 480

Phe Gln Glu Glu Ile Phe Gly Pro Val Leu Ala Val Thr Thr Phe Lys
                485                 490                 495

Asp Glu Ala Glu Ala Leu Ala Ile Ala Asn Asp Thr Leu Tyr Gly Leu
            500                 505                 510

Gly Ala Gly Val Trp Ser Arg Asn Gly Asn Val Ala Tyr Arg Met Gly
        515                 520                 525

Arg Ala Ile Gln Ala Gly Arg Val Trp Thr Asn Cys Tyr His Ala Tyr
    530                 535                 540

Pro Ala His Ala Ala Phe Gly Gly Tyr Lys Glu Ser Gly Ile Gly Arg
545                 550                 555                 560

Glu Thr His Lys Met Met Leu Asp His Tyr Gln Gln Thr Lys Asn Leu
                565                 570                 575

Leu Val Ser Tyr Ser Glu Asn Lys Leu Gly Phe Phe
            580                 585

<210>  27
<211>  1512
<212>  DNA
<213>  Thauera butanivorans

<220>
<221>  CDS
<222>  (1)..(1512)

<400>  27
atg agc ttt acc tac gcc tcg ccc caa agc cgc gtc ggc cac tgg atc      48
Met Ser Phe Thr Tyr Ala Ser Pro Gln Ser Arg Val Gly His Trp Ile
1               5                   10                  15

gac aac gaa ttc cgc ccc ggt gaa ggc ggc gag acc gcg gtt tac gat      96
Asp Asn Glu Phe Arg Pro Gly Glu Gly Gly Glu Thr Ala Val Tyr Asp
            20                  25                  30

ccc gcc ctg ggc aag gtt tcg cgc cag gtc gcg ctt gca acc cgg gcc      144
Pro Ala Leu Gly Lys Val Ser Arg Gln Val Ala Leu Ala Thr Arg Ala
        35                  40                  45

gaa gtc gat gcc gcc gtc gcc tcc gct gcc gcc gcc cag ccc gag tgg      192
Glu Val Asp Ala Ala Val Ala Ser Ala Ala Ala Ala Gln Pro Glu Trp
    50                  55                  60

ggc gcg ctg gcc gtc cag cgc cgc gcc cgc gtg ctg ttc cgg atg aag      240
Gly Ala Leu Ala Val Gln Arg Arg Ala Arg Val Leu Phe Arg Met Lys
65                  70                  75                  80

gaa ctg gtc gag cgc cac acc ggc gac ctc gcc cgt ctg gtc acc cgc      288
Glu Leu Val Glu Arg His Thr Gly Asp Leu Ala Arg Leu Val Thr Arg
                85                  90                  95

gag cac ggc aag acc ttc ccc gac gcc agg ggc gaa gtg cag cgc ggc      336
Glu His Gly Lys Thr Phe Pro Asp Ala Arg Gly Glu Val Gln Arg Gly
            100                 105                 110

ctg gaa atc atc gag ttc gcc tgc ggc att ccg cag ctt ctg aag ggc      384
Leu Glu Ile Ile Glu Phe Ala Cys Gly Ile Pro Gln Leu Leu Lys Gly
        115                 120                 125

cag tac tcc gac aac gtg ggc ggc ggc atc gcc aac tgg aac cag cgc      432
Gln Tyr Ser Asp Asn Val Gly Gly Gly Ile Ala Asn Trp Asn Gln Arg
    130                 135                 140

atg ccg ctg ggc gtc acc gcc ggc atc acc ccg ttc aat ttc ccc gtc      480
Met Pro Leu Gly Val Thr Ala Gly Ile Thr Pro Phe Asn Phe Pro Val
145                 150                 155                 160

atg gtg ccg atg tgg atg gcg ccg gtg tcg atc gcc tgc ggc aac gcc      528
Met Val Pro Met Trp Met Ala Pro Val Ser Ile Ala Cys Gly Asn Ala
                165                 170                 175

ttc gtg ctg aag ccc tcc gag cgc gac ccc tcg ccc tcg ctg ctg ctc      576
Phe Val Leu Lys Pro Ser Glu Arg Asp Pro Ser Pro Ser Leu Leu Leu
            180                 185                 190

ggc gag ctg ttc cgc gaa gcc ggc ctg ccg gca ggc gtg ttc aac gtc      624
Gly Glu Leu Phe Arg Glu Ala Gly Leu Pro Ala Gly Val Phe Asn Val
        195                 200                 205

gtg cag ggc gac aag acc gcg gtg gat gcg ctg ctg aac cac ccc gac      672
Val Gln Gly Asp Lys Thr Ala Val Asp Ala Leu Leu Asn His Pro Asp
    210                 215                 220

gtg cag gcg gtc agc ttc gtc ggc tcg acc ccg atc gcg aaa tac atc      720

Val Gln Ala Val Ser Phe Val Gly Ser Thr Pro Ile Ala Lys Tyr Ile
225                 230                 235                 240

tac gag acc gcc gcg cgc aac ggc aag cgc gcc cag gcc ctg ggc ggc      768
Tyr Glu Thr Ala Ala Arg Asn Gly Lys Arg Ala Gln Ala Leu Gly Gly
                245                 250                 255

gcc aag aac cac atg gtg gtg atg ccc gac gcc gac ctc gac caa gcg      816
Ala Lys Asn His Met Val Val Met Pro Asp Ala Asp Leu Asp Gln Ala
            260                 265                 270

gct gac gcg ctg atc ggc gcc gcc tac ggc tcg gcc ggc gaa cgc tgc      864
Ala Asp Ala Leu Ile Gly Ala Ala Tyr Gly Ser Ala Gly Glu Arg Cys
        275                 280                 285

atg gcg atc tcg gtc gcg gtc gcg gtc ggc agc atc gcc gac gag ctg      912
Met Ala Ile Ser Val Ala Val Ala Val Gly Ser Ile Ala Asp Glu Leu
    290                 295                 300

atc gcc aag gtg aag agc cgc gcc gaa aag ctc aag gtc gac gac ggc      960
Ile Ala Lys Val Lys Ser Arg Ala Glu Lys Leu Lys Val Asp Asp Gly
305                 310                 315                 320

gaa gcc agc ggt gcc gac atg ggc ccg gtg gtc acg cag gag gcc aag     1008
Glu Ala Ser Gly Ala Asp Met Gly Pro Val Val Thr Gln Glu Ala Lys
                325                 330                 335

gcg cgc atc gag gac tac atc gct tcc ggc gtc gaa cag ggc gcc agc     1056
Ala Arg Ile Glu Asp Tyr Ile Ala Ser Gly Val Glu Gln Gly Ala Ser
            340                 345                 350

ctg gtg ctc gac ggc cgc ggc ctg gtc gtg ccc ggc cgc gaa gac ggc     1104
Leu Val Leu Asp Gly Arg Gly Leu Val Val Pro Gly Arg Glu Asp Gly
        355                 360                 365

tac ttc ctc ggc gcc acg ctg ttc gac cac gtc aag ccc ggc atg aag     1152
Tyr Phe Leu Gly Ala Thr Leu Phe Asp His Val Lys Pro Gly Met Lys
    370                 375                 380

atc tac gac gag gaa atc ttc ggc ccg gtg ctg tgc gtg gtc cgc gtc     1200
Ile Tyr Asp Glu Glu Ile Phe Gly Pro Val Leu Cys Val Val Arg Val
385                 390                 395                 400

gat acc ttc gcc gag gcg gtg gcg atg atc aac gcc cac gcc ttc ggc     1248
Asp Thr Phe Ala Glu Ala Val Ala Met Ile Asn Ala His Ala Phe Gly
                405                 410                 415

aac ggc gtc gcc tgc tac acc cgc gac ggc cgc acg gcg cag gaa ttc     1296
Asn Gly Val Ala Cys Tyr Thr Arg Asp Gly Arg Thr Ala Gln Glu Phe
            420                 425                 430

gcc cag aag atc cag gtc ggc atg gtc ggc atc aac gtg ccg ctg ccg     1344
Ala Gln Lys Ile Gln Val Gly Met Val Gly Ile Asn Val Pro Leu Pro
        435                 440                 445

gtg ccg atg gcc tgg cat tcg ttc ggc ggc tgg aag cag agc ctg ttc     1392
Val Pro Met Ala Trp His Ser Phe Gly Gly Trp Lys Gln Ser Leu Phe
    450                 455                 460

ggc gac cac cac ggc tac ggc gag gaa ggc gtg cgc ttc tac acc cgc     1440
Gly Asp His His Gly Tyr Gly Glu Glu Gly Val Arg Phe Tyr Thr Arg
465                 470                 475                 480

tac aag agc atc atg cag cgc tgg ccc gac agc ggc tcc aag ggg ccc     1488
Tyr Lys Ser Ile Met Gln Arg Trp Pro Asp Ser Gly Ser Lys Gly Pro
                485                 490                 495

gag ttc gtg atg ccg gtc aac tga                                     1512
Glu Phe Val Met Pro Val Asn
            500

<210>  28
<211>  503
<212>  PRT
<213>  Thauera butanivorans

<400>  28

Met Ser Phe Thr Tyr Ala Ser Pro Gln Ser Arg Val Gly His Trp Ile
1               5                   10                  15

Asp Asn Glu Phe Arg Pro Gly Glu Gly Gly Glu Thr Ala Val Tyr Asp
            20                  25                  30

Pro Ala Leu Gly Lys Val Ser Arg Gln Val Ala Leu Ala Thr Arg Ala
        35                  40                  45

Glu Val Asp Ala Ala Val Ala Ser Ala Ala Ala Ala Gln Pro Glu Trp
    50                  55                  60

Gly Ala Leu Ala Val Gln Arg Arg Ala Arg Val Leu Phe Arg Met Lys
65                  70                  75                  80

Glu Leu Val Glu Arg His Thr Gly Asp Leu Ala Arg Leu Val Thr Arg
                85                  90                  95

Glu His Gly Lys Thr Phe Pro Asp Ala Arg Gly Glu Val Gln Arg Gly
            100                 105                 110

Leu Glu Ile Ile Glu Phe Ala Cys Gly Ile Pro Gln Leu Leu Lys Gly
        115                 120                 125

Gln Tyr Ser Asp Asn Val Gly Gly Gly Ile Ala Asn Trp Asn Gln Arg
    130                 135                 140

Met Pro Leu Gly Val Thr Ala Gly Ile Thr Pro Phe Asn Phe Pro Val
145                 150                 155                 160

Met Val Pro Met Trp Met Ala Pro Val Ser Ile Ala Cys Gly Asn Ala
                165                 170                 175

Phe Val Leu Lys Pro Ser Glu Arg Asp Pro Ser Pro Ser Leu Leu Leu
            180                 185                 190

Gly Glu Leu Phe Arg Glu Ala Gly Leu Pro Ala Gly Val Phe Asn Val
        195                 200                 205

Val Gln Gly Asp Lys Thr Ala Val Asp Ala Leu Leu Asn His Pro Asp
    210                 215                 220

Val Gln Ala Val Ser Phe Val Gly Ser Thr Pro Ile Ala Lys Tyr Ile
225                 230                 235                 240

Tyr Glu Thr Ala Ala Arg Asn Gly Lys Arg Ala Gln Ala Leu Gly Gly
                245                 250                 255

Ala Lys Asn His Met Val Val Met Pro Asp Ala Asp Leu Asp Gln Ala
            260                 265                 270

Ala Asp Ala Leu Ile Gly Ala Ala Tyr Gly Ser Ala Gly Glu Arg Cys
        275                 280                 285

Met Ala Ile Ser Val Ala Val Ala Val Gly Ser Ile Ala Asp Glu Leu
    290                 295                 300

Ile Ala Lys Val Lys Ser Arg Ala Glu Lys Leu Lys Val Asp Asp Gly
305                 310                 315                 320

Glu Ala Ser Gly Ala Asp Met Gly Pro Val Val Thr Gln Glu Ala Lys
                325                 330                 335

Ala Arg Ile Glu Asp Tyr Ile Ala Ser Gly Val Glu Gln Gly Ala Ser
            340                 345                 350

Leu Val Leu Asp Gly Arg Gly Leu Val Val Pro Gly Arg Glu Asp Gly
        355                 360                 365

Tyr Phe Leu Gly Ala Thr Leu Phe Asp His Val Lys Pro Gly Met Lys
    370                 375                 380

Ile Tyr Asp Glu Glu Ile Phe Gly Pro Val Leu Cys Val Val Arg Val
385                 390                 395                 400

Asp Thr Phe Ala Glu Ala Val Ala Met Ile Asn Ala His Ala Phe Gly
                405                 410                 415

Asn Gly Val Ala Cys Tyr Thr Arg Asp Gly Arg Thr Ala Gln Glu Phe
            420                 425                 430

Ala Gln Lys Ile Gln Val Gly Met Val Gly Ile Asn Val Pro Leu Pro
        435                 440                 445

Val Pro Met Ala Trp His Ser Phe Gly Gly Trp Lys Gln Ser Leu Phe
    450                 455                 460

Gly Asp His His Gly Tyr Gly Glu Glu Gly Val Arg Phe Tyr Thr Arg
465                 470                 475                 480

Tyr Lys Ser Ile Met Gln Arg Trp Pro Asp Ser Gly Ser Lys Gly Pro
                485                 490                 495

Glu Phe Val Met Pro Val Asn
            500

<210>  29
<211>  1872
<212>  DNA
<213>  Thauera butanivorans

<220>
<221>  CDS
<222>  (1)..(1872)

<400>  29
atg aaa aag tcg cat gca aag ccc ttc gcg ctg cgc gcc atc gtc gtc      48
Met Lys Lys Ser His Ala Lys Pro Phe Ala Leu Arg Ala Ile Val Val
1               5                   10                  15

gcc acc gca gcg gcg ttg tcg ctg ccg gcc gcc gca gtc acc gac gtc      96
Ala Thr Ala Ala Ala Leu Ser Leu Pro Ala Ala Ala Val Thr Asp Val
            20                  25                  30

acc tgg gag gac atc gcc aac gac cac aag acc acc ggc gac gtg ctg      144
Thr Trp Glu Asp Ile Ala Asn Asp His Lys Thr Thr Gly Asp Val Leu
        35                  40                  45

acc tac ggc ctg ggc ctg aag gcg cag cgc cac agc ccg ctc aag gcg      192
Thr Tyr Gly Leu Gly Leu Lys Ala Gln Arg His Ser Pro Leu Lys Ala
    50                  55                  60

atc aac acc gac aac gtc gcc aac ctg gtg ccg gcc tgg agc ttc tcc      240
Ile Asn Thr Asp Asn Val Ala Asn Leu Val Pro Ala Trp Ser Phe Ser
65                  70                  75                  80

ttc ggc ggt gaa aaa cag cgc ggc cag gaa gca cag gtt ctg gtg cac      288
Phe Gly Gly Glu Lys Gln Arg Gly Gln Glu Ala Gln Val Leu Val His
                85                  90                  95

gat ggc gtg atc tac gcc acc gcc tcc tac tcc cgc atc ttc gcc atc      336
Asp Gly Val Ile Tyr Ala Thr Ala Ser Tyr Ser Arg Ile Phe Ala Ile
            100                 105                 110

gac gcc cgc agc ggc aag cgc ctg tgg gaa tac aac gcc cgc ctg ccg      384
Asp Ala Arg Ser Gly Lys Arg Leu Trp Glu Tyr Asn Ala Arg Leu Pro
        115                 120                 125

gac gac atc cgc ccc tgc tgc gac gtg gtc aac cgc ggc gcc gcc atc      432
Asp Asp Ile Arg Pro Cys Cys Asp Val Val Asn Arg Gly Ala Ala Ile

130                 135                 140

tac ggc gac aag gtc ttc ttc ggc act ctg gat gcg gcc atg gtc gcg         480
Tyr Gly Asp Lys Val Phe Phe Gly Thr Leu Asp Ala Ala Met Val Ala
145                 150                 155                 160

ctc gac cgc aag acc ggc aag gtc gtg tgg cgg aag aag ttc ggc gac         528
Leu Asp Arg Lys Thr Gly Lys Val Val Trp Arg Lys Lys Phe Gly Asp
                165                 170                 175

cac aag gtg ggc tac acc atg acc ggc gcc ccc ttc gtc atc aag gac         576
His Lys Val Gly Tyr Thr Met Thr Gly Ala Pro Phe Val Ile Lys Asp
            180                 185                 190

cag aag tcc ggc cgc acg ctg ctg gtg cac ggc tca tcc ggc gac gaa         624
Gln Lys Ser Gly Arg Thr Leu Leu Val His Gly Ser Ser Gly Asp Glu
        195                 200                 205

ttc ggc gtg gtc ggc tgg ctg ttc gcg cgc gac ccg gac acc ggt gag         672
Phe Gly Val Val Gly Trp Leu Phe Ala Arg Asp Pro Asp Thr Gly Glu
    210                 215                 220

gaa gtc tgg gcc cgt ccg atg gtc gaa ggc cac atg ggc cgc ctg aac         720
Glu Val Trp Ala Arg Pro Met Val Glu Gly His Met Gly Arg Leu Asn
225                 230                 235                 240

ggc aag gac agc acc ccc acc ggc gac ccc aag gca ccg agc tgg ccg         768
Gly Lys Asp Ser Thr Pro Thr Gly Asp Pro Lys Ala Pro Ser Trp Pro
                245                 250                 255

gac gat ccc aac tcg ccc acc ggc aag gtc gaa gcc tgg agc cag ggc         816
Asp Asp Pro Asn Ser Pro Thr Gly Lys Val Glu Ala Trp Ser Gln Gly
            260                 265                 270

ggc ggc gca cca tgg cag acc gcc agc ttc gac gtc gaa aac aac atg         864
Gly Gly Ala Pro Trp Gln Thr Ala Ser Phe Asp Val Glu Asn Asn Met
        275                 280                 285

gtc gtc atc ggc gcc ggc aac ccc gca ccg tgg aac acc tgg aag cgc         912
Val Val Ile Gly Ala Gly Asn Pro Ala Pro Trp Asn Thr Trp Lys Arg
    290                 295                 300

acc gcg ccg ggc gac gac ccg cgc aac tgg gac agc ctg ttc acc tcc         960
Thr Ala Pro Gly Asp Asp Pro Arg Asn Trp Asp Ser Leu Phe Thr Ser
305                 310                 315                 320

ggc cag gcc tat gtc gac gcc agc acc ggc gag ctg aag ggc ttc tac        1008
Gly Gln Ala Tyr Val Asp Ala Ser Thr Gly Glu Leu Lys Gly Phe Tyr
                325                 330                 335

cag cac acc ccg aac gac gcc tgg gac ttt tcc ggc aac aac tcc gtc        1056
Gln His Thr Pro Asn Asp Ala Trp Asp Phe Ser Gly Asn Asn Ser Val
            340                 345                 350

gtg ctg ttc gag tac aag gac ccg aag acc ggc aag atg gtc aat gcc        1104
Val Leu Phe Glu Tyr Lys Asp Pro Lys Thr Gly Lys Met Val Asn Ala
        355                 360                 365

tcg gcc cac gcc gac cgc aac ggc ttc ttt ttc gtc acc gac cgc gac        1152
Ser Ala His Ala Asp Arg Asn Gly Phe Phe Phe Val Thr Asp Arg Asp
    370                 375                 380

atg ctg gcc aag ggc gcc ggc tat ccc aac aag ccg acc tcg ctg atc        1200

Met Leu Ala Lys Gly Ala Gly Tyr Pro Asn Lys Pro Thr Ser Leu Ile
385                 390                 395                 400

ggc gcc tgg ccc ttc gtg gac ggc atc acc tgg gcg tcg ggc ttc gac      1248
Gly Ala Trp Pro Phe Val Asp Gly Ile Thr Trp Ala Ser Gly Phe Asp
                405                 410                 415

ctc aag acc ggc aaa ccg atc gag aag gac aac cgt ccg ccg cag ccg      1296
Leu Lys Thr Gly Lys Pro Ile Glu Lys Asp Asn Arg Pro Pro Gln Pro
            420                 425                 430

aag gaa ggc gcg gac aag ggt gag tcg atc ttc gtc tcg ccg ccc ttc      1344
Lys Glu Gly Ala Asp Lys Gly Glu Ser Ile Phe Val Ser Pro Pro Phe
        435                 440                 445

ctc ggc ggc acc aac tgg cac ccg atg tcg tac agc ccg gac acc ggc      1392
Leu Gly Gly Thr Asn Trp His Pro Met Ser Tyr Ser Pro Asp Thr Gly
    450                 455                 460

ctg ttc tac atc ccg gcc aac cac tgg gcg atg gac tac tgg acc gag      1440
Leu Phe Tyr Ile Pro Ala Asn His Trp Ala Met Asp Tyr Trp Thr Glu
465                 470                 475                 480

aac gtc acc tac aag gcc ggc tcc gcc tat ctg ggc cag ggc ttc cgc      1488
Asn Val Thr Tyr Lys Ala Gly Ser Ala Tyr Leu Gly Gln Gly Phe Arg
                485                 490                 495

atc aag aac ttg ttc gac gac cac gtc ggc atc ctg cgc gcg atc gac      1536
Ile Lys Asn Leu Phe Asp Asp His Val Gly Ile Leu Arg Ala Ile Asp
            500                 505                 510

ccg tca ccg gca aga tcg ctg gga gca caa gga aga gtt ccc gct gtg      1584
Pro Ser Pro Ala Arg Ser Leu Gly Ala Gln Gly Arg Val Pro Ala Val
        515                 520                 525

gcc ggc acc ttg acc acc gcc ggc ggc tgg gtc tty acc ggc act tcg      1632
Ala Gly Thr Leu Thr Thr Ala Gly Gly Trp Val Phe Thr Gly Thr Ser
    530                 535                 540

gac ggc tac ytg aag gcc ttc gac gcc aag aac ggc aag gaa ctg tgg      1680
Asp Gly Tyr Xaa Lys Ala Phe Asp Ala Lys Asn Gly Lys Glu Leu Trp
545                 550                 555                 560

aag ttc cag acc ggc tcg ggc gtg gtc tcg gta ccg gtg acc tgg gaa      1728
Lys Phe Gln Thr Gly Ser Gly Val Val Ser Val Pro Val Thr Trp Glu
                565                 570                 575

atg gac ggc gag cag tac gtc gcc atc cag tcg ggc tac ggc ggc gcg      1776
Met Asp Gly Glu Gln Tyr Val Ala Ile Gln Ser Gly Tyr Gly Gly Ala
            580                 585                 590

gta ccg ctg tgg ggc ggc gac atg gcc gag ctg acc aag cag gtc acc      1824
Val Pro Leu Trp Gly Gly Asp Met Ala Glu Leu Thr Lys Gln Val Thr
        595                 600                 605

cag ggc ggt tcg atg tgg gtg ttc aag ctg ccg aaa gcc agc cgc tga      1872
Gln Gly Gly Ser Met Trp Val Phe Lys Leu Pro Lys Ala Ser Arg
    610                 615                 620

<210>  30
<211>  623
<212>  PRT

<213>  Thauera butanivorans

<220>
<221>  misc_feature
<222>  (548)..(548)
<223>  The 'Xaa' at location 548 stands for Leu.

<400>  30

Met Lys Lys Ser His Ala Lys Pro Phe Ala Leu Arg Ala Ile Val Val
1               5                   10                  15

Ala Thr Ala Ala Ala Leu Ser Leu Pro Ala Ala Ala Val Thr Asp Val
            20                  25                  30

Thr Trp Glu Asp Ile Ala Asn Asp His Lys Thr Thr Gly Asp Val Leu
        35                  40                  45

Thr Tyr Gly Leu Gly Leu Lys Ala Gln Arg His Ser Pro Leu Lys Ala
    50                  55                  60

Ile Asn Thr Asp Asn Val Ala Asn Leu Val Pro Ala Trp Ser Phe Ser
65                  70                  75                  80

Phe Gly Gly Glu Lys Gln Arg Gly Gln Glu Ala Gln Val Leu Val His
                85                  90                  95

Asp Gly Val Ile Tyr Ala Thr Ala Ser Tyr Ser Arg Ile Phe Ala Ile
            100                 105                 110

Asp Ala Arg Ser Gly Lys Arg Leu Trp Glu Tyr Asn Ala Arg Leu Pro
        115                 120                 125

Asp Asp Ile Arg Pro Cys Cys Asp Val Val Asn Arg Gly Ala Ala Ile
    130                 135                 140

Tyr Gly Asp Lys Val Phe Phe Gly Thr Leu Asp Ala Ala Met Val Ala
145                 150                 155                 160

Leu Asp Arg Lys Thr Gly Lys Val Val Trp Arg Lys Lys Phe Gly Asp
                165                 170                 175

His Lys Val Gly Tyr Thr Met Thr Gly Ala Pro Phe Val Ile Lys Asp
            180                 185                 190

Gln Lys Ser Gly Arg Thr Leu Leu Val His Gly Ser Ser Gly Asp Glu
        195                 200                 205

Phe Gly Val Val Gly Trp Leu Phe Ala Arg Asp Pro Asp Thr Gly Glu
    210                 215                 220

Glu Val Trp Ala Arg Pro Met Val Glu Gly His Met Gly Arg Leu Asn
225                 230                 235                 240

Gly Lys Asp Ser Thr Pro Thr Gly Asp Pro Lys Ala Pro Ser Trp Pro
                245                 250                 255

Asp Asp Pro Asn Ser Pro Thr Gly Lys Val Glu Ala Trp Ser Gln Gly
            260                 265                 270

Gly Gly Ala Pro Trp Gln Thr Ala Ser Phe Asp Val Glu Asn Asn Met
        275                 280                 285

Val Val Ile Gly Ala Gly Asn Pro Ala Pro Trp Asn Thr Trp Lys Arg
    290                 295                 300

Thr Ala Pro Gly Asp Asp Pro Arg Asn Trp Asp Ser Leu Phe Thr Ser
305                 310                 315                 320

Gly Gln Ala Tyr Val Asp Ala Ser Thr Gly Glu Leu Lys Gly Phe Tyr
                325                 330                 335

Gln His Thr Pro Asn Asp Ala Trp Asp Phe Ser Gly Asn Asn Ser Val
            340                 345                 350

Val Leu Phe Glu Tyr Lys Asp Pro Lys Thr Gly Lys Met Val Asn Ala
        355                 360                 365

Ser Ala His Ala Asp Arg Asn Gly Phe Phe Phe Val Thr Asp Arg Asp
    370                 375                 380

Met Leu Ala Lys Gly Ala Gly Tyr Pro Asn Lys Pro Thr Ser Leu Ile
385                 390                 395                 400

Gly Ala Trp Pro Phe Val Asp Gly Ile Thr Trp Ala Ser Gly Phe Asp
                405                 410                 415

Leu Lys Thr Gly Lys Pro Ile Glu Lys Asp Asn Arg Pro Pro Gln Pro
            420                 425                 430

Lys Glu Gly Ala Asp Lys Gly Glu Ser Ile Phe Val Ser Pro Pro Phe
        435                 440                 445

Leu Gly Gly Thr Asn Trp His Pro Met Ser Tyr Ser Pro Asp Thr Gly
    450                 455                 460

Leu Phe Tyr Ile Pro Ala Asn His Trp Ala Met Asp Tyr Trp Thr Glu

465                 470                 475                 480

Asn Val Thr Tyr Lys Ala Gly Ser Ala Tyr Leu Gly Gln Gly Phe Arg
                485                 490                 495

Ile Lys Asn Leu Phe Asp Asp His Val Gly Ile Leu Arg Ala Ile Asp
            500                 505                 510

Pro Ser Pro Ala Arg Ser Leu Gly Ala Gln Gly Arg Val Pro Ala Val
        515                 520                 525

Ala Gly Thr Leu Thr Thr Ala Gly Gly Trp Val Phe Thr Gly Thr Ser
    530                 535                 540

Asp Gly Tyr Xaa Lys Ala Phe Asp Ala Lys Asn Gly Lys Glu Leu Trp
545                 550                 555                 560

Lys Phe Gln Thr Gly Ser Gly Val Val Ser Val Pro Val Thr Trp Glu
                565                 570                 575

Met Asp Gly Glu Gln Tyr Val Ala Ile Gln Ser Gly Tyr Gly Gly Ala
            580                 585                 590

Val Pro Leu Trp Gly Gly Asp Met Ala Glu Leu Thr Lys Gln Val Thr
        595                 600                 605

Gln Gly Gly Ser Met Trp Val Phe Lys Leu Pro Lys Ala Ser Arg
    610                 615                 620

<210>  31
<211>  2076
<212>  DNA
<213>  Thauera butanivorans

<220>
<221>  CDS
<222>  (1)..(2076)

<400>  31
atg ttg acg acg aca ttt gcg cgc aaa cgg gag gag agc gtc ccg ctg      48
Met Leu Thr Thr Thr Phe Ala Arg Lys Arg Glu Glu Ser Val Pro Leu
1               5                   10                  15

cgc aag ggc att caa cgg gcg ctg ctc ggc ctg agc tgc ctg gtt ctg      96
Arg Lys Gly Ile Gln Arg Ala Leu Leu Gly Leu Ser Cys Leu Val Leu
            20                  25                  30

agt acc acg tcc ttc gct gcc ggt ggc gaa tgg cgc acg cat ggt tat      144
Ser Thr Thr Ser Phe Ala Ala Gly Gly Glu Trp Arg Thr His Gly Tyr
        35                  40                  45

gac gac gcc ggc acg cgc tac agc ccc ctg gcc cag atc acg ccg gac      192

Asp Asp Ala Gly Thr Arg Tyr Ser Pro Leu Ala Gln Ile Thr Pro Asp
    50                  55                  60

aat gcc aag gaa ctc ggg ctg gtc tgg tcc tac gac ctg gag tcg tcg      240
Asn Ala Lys Glu Leu Gly Leu Val Trp Ser Tyr Asp Leu Glu Ser Ser
65                  70                  75                  80

cgc ggc gtc gaa gcc acg ccg atc gtg gtc gat ggc gtg atg tac gtg      288
Arg Gly Val Glu Ala Thr Pro Ile Val Val Asp Gly Val Met Tyr Val
                85                  90                  95

acc gcg ccg tgg agc gtc gtg cat gcg ctg gac gtg cgc agc ggc aag      336
Thr Ala Pro Trp Ser Val Val His Ala Leu Asp Val Arg Ser Gly Lys
            100                 105                 110

cgg ttg tgg acc tac gac ccg gaa gtt ccg cgc gag aag ggc aag aat      384
Arg Leu Trp Thr Tyr Asp Pro Glu Val Pro Arg Glu Lys Gly Lys Asn
        115                 120                 125

gct tgt tgc gac gtg gtg aac cgc ggc gtg gcc gtg cat gag ggc aag      432
Ala Cys Cys Asp Val Val Asn Arg Gly Val Ala Val His Glu Gly Lys
    130                 135                 140

gtc ttc gtc ggc tcg ctc gac ggc cgg ctg gtc gcc atc gac gcc agg      480
Val Phe Val Gly Ser Leu Asp Gly Arg Leu Val Ala Ile Asp Ala Arg
145                 150                 155                 160

acc ggc aag cgg gtg tgg gag cgg aac acg ctg atc gac gac gac aag      528
Thr Gly Lys Arg Val Trp Glu Arg Asn Thr Leu Ile Asp Asp Asp Lys
                165                 170                 175

ccc tac acc atc acc ggt gcg ccg cgc gtc atc aag ggc aag gtc gtc      576
Pro Tyr Thr Ile Thr Gly Ala Pro Arg Val Ile Lys Gly Lys Val Val
            180                 185                 190

atc ggc aac ggc ggt gcg gag ttc ggc gtg cgc ggc tac atc acc gcc      624
Ile Gly Asn Gly Gly Ala Glu Phe Gly Val Arg Gly Tyr Ile Thr Ala
        195                 200                 205

tac gac ccg aca gcg gcg agc agg cct ggc gtg gtt ccc ggg ccg ggt      672
Tyr Asp Pro Thr Ala Ala Ser Arg Pro Gly Val Val Pro Gly Pro Gly
    210                 215                 220

gat ccg tcg ctg ccg ttc gag gat gcc tcg atg gag gca gcg gcc aag      720
Asp Pro Ser Leu Pro Phe Glu Asp Ala Ser Met Glu Ala Ala Ala Lys
225                 230                 235                 240

acc tgg gac ccg gcc ggc caa gta ctg gga agc ggg cgg cgg cgg cac      768
Thr Trp Asp Pro Ala Gly Gln Val Leu Gly Ser Gly Arg Arg Arg His
                245                 250                 255

ggt gtg gaa ctc gat ggt tta tac cgc aaa gct gga ttc tgt tgt aca      816
Gly Val Glu Leu Asp Gly Leu Tyr Arg Lys Ala Gly Phe Cys Cys Thr
            260                 265                 270

tcg gca ccg gca acc ccc tcg ccg tgg tcg cac cgc aag cgt agc ccg      864
Ser Ala Pro Ala Thr Pro Ser Pro Trp Ser His Arg Lys Arg Ser Pro
        275                 280                 285

gcc ggc ggc gac aac ctg tac acc gcg tcc atc gtc gcg ctg cgc ccg      912
Ala Gly Gly Asp Asn Leu Tyr Thr Ala Ser Ile Val Ala Leu Arg Pro
    290                 295                 300

gac acc ggc gaa tac gtc tgg cac tac cag cag acg ccg gcg gac aac 960
Asp Thr Gly Glu Tyr Val Trp His Tyr Gln Gln Thr Pro Ala Asp Asn
305                 310                 315                 320

tgg gac tac acc tcg acg cag gac ctg atc ctg gcc gac atc gaa ctg 1008
Trp Asp Tyr Thr Ser Thr Gln Asp Leu Ile Leu Ala Asp Ile Glu Leu
                325                 330                 335

ggg ggc aag ccg cgc aag gtc atc ctg cat gcg ccc aag aac ggc ttc 1056
Gly Gly Lys Pro Arg Lys Val Ile Leu His Ala Pro Lys Asn Gly Phe
            340                 345                 350

ttc ttc gtc atc gac cgt acc gac ggc aag ttc atc tcg gcg cag aac 1104
Phe Phe Val Ile Asp Arg Thr Asp Gly Lys Phe Ile Ser Ala Gln Asn
        355                 360                 365

ttc gtg ccg gtg aat tgg gcg acc ggc tac gac gag aac ggc cgc ccc 1152
Phe Val Pro Val Asn Trp Ala Thr Gly Tyr Asp Glu Asn Gly Arg Pro
    370                 375                 380

atc gag aac ccg gaa ggc gcg tgg ccc gga cac ctt tcg atg cga ttc 1200
Ile Glu Asn Pro Glu Gly Ala Trp Pro Gly His Leu Ser Met Arg Phe
385                 390                 395                 400

ccg gcc cct tcg gcg cgc acs aat tgg cat tcg atg tcc tac agc ccg 1248
Pro Ala Pro Ser Ala Arg Xaa Asn Trp His Ser Met Ser Tyr Ser Pro
                405                 410                 415

cag acc ggg ctg gcc tac ttc ccg gcg cag aac atc ccg ctg gtg ctg 1296
Gln Thr Gly Leu Ala Tyr Phe Pro Ala Gln Asn Ile Pro Leu Val Leu
            420                 425                 430

cag gag gac aag aac tgg tcc tac aac cag gcg cag ccc ggc cag gcg 1344
Gln Glu Asp Lys Asn Trp Ser Tyr Asn Gln Ala Gln Pro Gly Gln Ala
        435                 440                 445

atg gcc ggc atc ggc tgg aac ctg ggc atg ctg gtc aat ccc cgt ccg 1392
Met Ala Gly Ile Gly Trp Asn Leu Gly Met Leu Val Asn Pro Arg Pro
    450                 455                 460

cca gcc agc cag cct ttc ggc cgc ctg atc gcg tgg gac ccg gtg cag 1440
Pro Ala Ser Gln Pro Phe Gly Arg Leu Ile Ala Trp Asp Pro Val Gln
465                 470                 475                 480

cag aaa gag gtc tgg cgc aag gag cat gtt tcg ccg tgg aac ggc ggc 1488
Gln Lys Glu Val Trp Arg Lys Glu His Val Ser Pro Trp Asn Gly Gly
                485                 490                 495

acg ctg gtg acg gcc ggc aac gtg gtg ttc cag ggt acg gcg gac gcg 1536
Thr Leu Val Thr Ala Gly Asn Val Val Phe Gln Gly Thr Ala Asp Ala
            500                 505                 510

cgc ctg ctg gcc ttc gac gcg cgc gac ggc aag gag ctg tgg tcg gcg 1584
Arg Leu Leu Ala Phe Asp Ala Arg Asp Gly Lys Glu Leu Trp Ser Ala
        515                 520                 525

ccg atg ggc acc ggg gtg atc gcc gcg ccg gtg act tac gag gtg gat 1632
Pro Met Gly Thr Gly Val Ile Ala Ala Pro Val Thr Tyr Glu Val Asp
    530                 535                 540

ggc aag cag tac gtg tcc atc gcg gtc ggc tgg ggc ggg gtg tac ggc 1680
Gly Lys Gln Tyr Val Ser Ile Ala Val Gly Trp Gly Gly Val Tyr Gly
545                 550                 555                 560

aac ttt acc cgg gct tcg gag cgg cgc acg ccg ggc acg gtg tac acc 1728
Asn Phe Thr Arg Ala Ser Glu Arg Arg Thr Pro Gly Thr Val Tyr Thr
                565                 570                 575

ttc gcc ttg ggt ggc aag gcg gag atg ccg gct ttc acc gag tat cag 1776
Phe Ala Leu Gly Gly Lys Ala Glu Met Pro Ala Phe Thr Glu Tyr Gln
            580                 585                 590

ctc aac aac ctg gtg tcg ggc gtc gac tac aac ccc gac gac gtg gcc 1824
Leu Asn Asn Leu Val Ser Gly Val Asp Tyr Asn Pro Asp Asp Val Ala
        595                 600                 605

gaa ggt acc ggg ctg tac gtg aca aat tgc gtc ttc tgc cac ggc gta 1872
Glu Gly Thr Gly Leu Tyr Val Thr Asn Cys Val Phe Cys His Gly Val
    610                 615                 620

ccg ggt gtg gac aaa ggc ggc ggc att ccc aac cta ggc tac tcg aca 1920
Pro Gly Val Asp Lys Gly Gly Gly Ile Pro Asn Leu Gly Tyr Ser Thr
625                 630                 635                 640

gcg gaa acc atc gcc cac ctg gat cag ttc gtg ttc aag ggg ccg ttc 1968
Ala Glu Thr Ile Ala His Leu Asp Gln Phe Val Phe Lys Gly Pro Phe
                645                 650                 655

atg ccg cgc ggc atg ccg gat ttc acc ggc aag ctg acg ccc gaa cag 2016
Met Pro Arg Gly Met Pro Asp Phe Thr Gly Lys Leu Thr Pro Glu Gln
            660                 665                 670

gtg gag aag atc aag gcc ttc att ctc ggc acg gcc gac gca gtg cgg 2064
Val Glu Lys Ile Lys Ala Phe Ile Leu Gly Thr Ala Asp Ala Val Arg
        675                 680                 685

ccg aaa aag taa 2076
Pro Lys Lys
    690

<210> 32
<211> 691
<212> PRT
<213> Thauera butanivorans

<220>
<221> misc_feature
<222> (407)..(407)
<223> The 'Xaa' at location 407 stands for Thr.

<400> 32

Met Leu Thr Thr Thr Phe Ala Arg Lys Arg Glu Glu Ser Val Pro Leu
1               5                   10                  15

Arg Lys Gly Ile Gln Arg Ala Leu Leu Gly Leu Ser Cys Leu Val Leu
            20                  25                  30

Ser Thr Thr Ser Phe Ala Ala Gly Gly Glu Trp Arg Thr His Gly Tyr
        35                  40                  45

Asp Asp Ala Gly Thr Arg Tyr Ser Pro Leu Ala Gln Ile Thr Pro Asp

50                  55                  60

Asn Ala Lys Glu Leu Gly Leu Val Trp Ser Tyr Asp Leu Glu Ser Ser
65                  70                  75                  80

Arg Gly Val Glu Ala Thr Pro Ile Val Val Asp Gly Val Met Tyr Val
                85                  90                  95

Thr Ala Pro Trp Ser Val Val His Ala Leu Asp Val Arg Ser Gly Lys
            100                 105                 110

Arg Leu Trp Thr Tyr Asp Pro Glu Val Pro Arg Glu Lys Gly Lys Asn
        115                 120                 125

Ala Cys Cys Asp Val Val Asn Arg Gly Val Ala Val His Glu Gly Lys
    130                 135                 140

Val Phe Val Gly Ser Leu Asp Gly Arg Leu Val Ala Ile Asp Ala Arg
145                 150                 155                 160

Thr Gly Lys Arg Val Trp Glu Arg Asn Thr Leu Ile Asp Asp Asp Lys
                165                 170                 175

Pro Tyr Thr Ile Thr Gly Ala Pro Arg Val Ile Lys Gly Lys Val Val
            180                 185                 190

Ile Gly Asn Gly Gly Ala Glu Phe Gly Val Arg Gly Tyr Ile Thr Ala
        195                 200                 205

Tyr Asp Pro Thr Ala Ala Ser Arg Pro Gly Val Val Pro Gly Pro Gly
    210                 215                 220

Asp Pro Ser Leu Pro Phe Glu Asp Ala Ser Met Glu Ala Ala Ala Lys
225                 230                 235                 240

Thr Trp Asp Pro Ala Gly Gln Val Leu Gly Ser Gly Arg Arg Arg His
                245                 250                 255

Gly Val Glu Leu Asp Gly Leu Tyr Arg Lys Ala Gly Phe Cys Cys Thr
            260                 265                 270

Ser Ala Pro Ala Thr Pro Ser Pro Trp Ser His Arg Lys Arg Ser Pro
        275                 280                 285

Ala Gly Gly Asp Asn Leu Tyr Thr Ala Ser Ile Val Ala Leu Arg Pro
    290                 295                 300

Asp Thr Gly Glu Tyr Val Trp His Tyr Gln Gln Thr Pro Ala Asp Asn
305                 310                 315                 320

Trp Asp Tyr Thr Ser Thr Gln Asp Leu Ile Leu Ala Asp Ile Glu Leu
                325                 330                 335

Gly Gly Lys Pro Arg Lys Val Ile Leu His Ala Pro Lys Asn Gly Phe
            340                 345                 350

Phe Phe Val Ile Asp Arg Thr Asp Gly Lys Phe Ile Ser Ala Gln Asn
        355                 360                 365

Phe Val Pro Val Asn Trp Ala Thr Gly Tyr Asp Glu Asn Gly Arg Pro
    370                 375                 380

Ile Glu Asn Pro Glu Gly Ala Trp Pro Gly His Leu Ser Met Arg Phe
385                 390                 395                 400

Pro Ala Pro Ser Ala Arg Xaa Asn Trp His Ser Met Ser Tyr Ser Pro
                405                 410                 415

Gln Thr Gly Leu Ala Tyr Phe Pro Ala Gln Asn Ile Pro Leu Val Leu
            420                 425                 430

Gln Glu Asp Lys Asn Trp Ser Tyr Asn Gln Ala Gln Pro Gly Gln Ala
        435                 440                 445

Met Ala Gly Ile Gly Trp Asn Leu Gly Met Leu Val Asn Pro Arg Pro
    450                 455                 460

Pro Ala Ser Gln Pro Phe Gly Arg Leu Ile Ala Trp Asp Pro Val Gln
465                 470                 475                 480

Gln Lys Glu Val Trp Arg Lys Glu His Val Ser Pro Trp Asn Gly Gly
                485                 490                 495

Thr Leu Val Thr Ala Gly Asn Val Val Phe Gln Gly Thr Ala Asp Ala
            500                 505                 510

Arg Leu Leu Ala Phe Asp Ala Arg Asp Gly Lys Glu Leu Trp Ser Ala
        515                 520                 525

Pro Met Gly Thr Gly Val Ile Ala Ala Pro Val Thr Tyr Glu Val Asp
    530                 535                 540

Gly Lys Gln Tyr Val Ser Ile Ala Val Gly Trp Gly Gly Val Tyr Gly
545                 550                 555                 560

Asn Phe Thr Arg Ala Ser Glu Arg Arg Thr Pro Gly Thr Val Tyr Thr
                565                 570                 575

Phe Ala Leu Gly Gly Lys Ala Glu Met Pro Ala Phe Thr Glu Tyr Gln
            580                 585                 590

Leu Asn Asn Leu Val Ser Gly Val Asp Tyr Asn Pro Asp Asp Val Ala
        595                 600                 605

Glu Gly Thr Gly Leu Tyr Val Thr Asn Cys Val Phe Cys His Gly Val
    610                 615                 620

Pro Gly Val Asp Lys Gly Gly Gly Ile Pro Asn Leu Gly Tyr Ser Thr
625                 630                 635                 640

Ala Glu Thr Ile Ala His Leu Asp Gln Phe Val Phe Lys Gly Pro Phe
                645                 650                 655

Met Pro Arg Gly Met Pro Asp Phe Thr Gly Lys Leu Thr Pro Glu Gln
            660                 665                 670

Val Glu Lys Ile Lys Ala Phe Ile Leu Gly Thr Ala Asp Ala Val Arg
        675                 680                 685

Pro Lys Lys
    690